(19) Europäisches Patentamt
European Patent Office
Office européen des brevets

(11) Veröffentlichungsnummer: **0 003 814**
**B1**

(12) # EUROPÄISCHE PATENTSCHRIFT

(45) Veröffentlichungstag der Patentschrift: **16.12.81**

(21) Anmeldenummer: **79100468.2**

(22) Anmeldetag: **19.02.79**

(51) Int. Cl.³: **C 07 D 499/64,** A 61 K 31/43 // C07D239/42, C07D239/52, C07D239/56, C07D239/48

(54) **Neue Penicilline, ihre Salze, Verfahren zu ihrer Herstellung und diese Verbindungen enthaltende Arzneimittel.**

(30) Priorität: **25.02.78 DE 2808153**
**27.11.78 DE 2851270**
**27.11.78 DE 2851226**

(43) Veröffentlichungstag der Anmeldung: **05.09.79 Patentblatt 79/18**

(45) Bekanntmachung des Hinweises auf die Patenterteilung: **16.12.81 Patentblatt 81/50**

(84) Benannte Vertragsstaaten: **BE CH DE FR GB IT LU NL SE**

(56) Entgegenhaltungen: **DE-A-2 450 668**
**US-A-4 038 271**

(73) Patentinhaber: **Dr. Karl Thomae GmbH, Postfach 720, D-7950 Biberach (Riss) (DE)**

(72) Erfinder: **Wetzel, Bernd, Dr. Dipl.-Chem, Kapellenweg 21, D-7950 Biberach (DE)**
Erfinder: **Reuter, Wolfgang, Dr. Dipl.-Chem, Nelkenweg 10, Laupertshausen 139 (DE)**
Erfinder: **Woitun, Eberhard, Dr. Dipl.-Chem, Stecherweg 17, D-7950 Biberach 1 (DE)**
Erfinder: **Maier, Roland, Dr. Dipl.-Chem, Bodelschwinghstrasse 39, D-7950 Biberach 1 (DE)**
Erfinder: **Lechner, Uwe, Dr., Panoramastrasse 12, D-7951 Ummendorf (DE)**
Erfinder: **Goeth, Hanns, Dr., Oberer Bühl 6, D-7950 Biberach 1 (DE)**
Erfinder: **Werner, Rolf, Dr., Blumenstrasse 19, D-7950 Biberach 18 (DE)**

EP 0 003 814 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

Neue Penicilline, ihre Salze, Verfahren zu ihrer Herstellung und diese Verbindungen enthaltende Arzneimittel

Die Erfindung betrifft neue Penicilline der allgemeinen Formel I

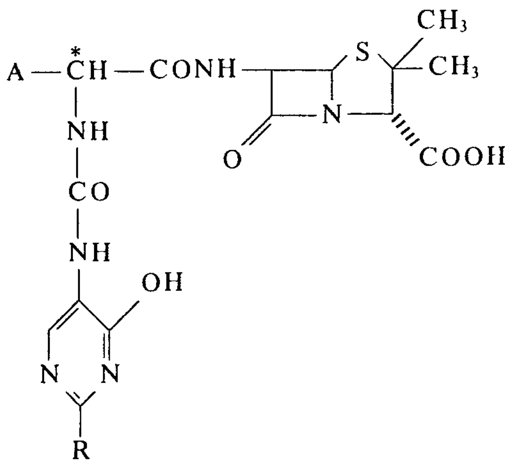

(I)

ihre physiologisch verträglichen Salze mit anorganischen oder organischen Basen, Verfahren zur Herstellung dieser Verbindungen und diese Verbindungen enthaltende Arzneimittel.

In der obigen allgemeinen Formel I bedeutet:

A die Phenyl-, 4-Hydroxyphenyl-, 2-Thienyl-, Cyclohexyl-, Cyclohexen-1-yl- oder Cyclohexa-1,4-dien-1-ylgruppe, sowie einen in 3,4-Stellung disubstituierten Phenylrest, wobei die Substituenten gleich oder verschieden und Chloratome oder Hydroxygruppen sein können,
R ein Wasserstoffatom, eine Methylgruppe, den Cyclopropylrest, der gegebenenfalls mit einer Methylgruppe substituiert sein kann, den Cyclobutylrest,
R bedeutet des weiteren,
die Hydroxygruppe, die Methoxy- oder Äthoxygruppe, die Amino- oder Dimethylaminogruppe, eine Gruppe der allgemeinen Formel $NHR_1$, wobei $R_1$ einen verzweigten oder unverzweigten Alkylrest mit 1 bis 6 Kohlenstoffatomen, einen Cycloalkylrest mit 3 bis 7 Kohlenstoffatomen, einen Cycloalkylmethylrest mit 3 bis 6 Kohlenstoffatomen im Cycloalkylteil sowie einen Allyl-, Crotyl- oder Propargylrest darstellt,
R kann weiter einen Pyrrolidino-, Piperiaino- oder Morpholinorest bedeuten,
R bedeutet weiter eine Gruppe der allgemeinen Formel

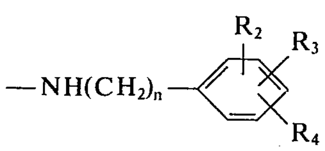

worin n die Zahlen 0 oder 1 und $R_2$, $R_3$ und $R_4$, die gleich oder voneinander verschieden sein können, Wasserstoff, Halogen, freie Aminogruppen, Alkylamino- oder Dialkylaminogruppen, wobei jeder Alkylteil 1 bis 3 Kohlenstoffatome enthält, Pyrrolidyl-, Piperidyl-, Hydroxy- oder Alkoxygruppen mit 1 bis 3 Kohlenstoffatomen, aliphatische Acylaminogruppen mit 1 bis 3 Kohlenstoffatomen, Aminocarbonylamino-, Alkylaminocarbonylamino-, Dialkylaminocarbonylaminogruppen mit 1 bis 3 Kohlenstoffatomen in jeder Alkylgruppe, Nitro-, Alkylsulfonylaminogruppen mit 1 bis 3 Kohlenstoffatomen, die Hydroxysulfonylaminogruppe, Formyl- oder Alkylcarbonylgruppen mit 1 bis 3 Kohlenstoffatomen im Alkylteil, Alkylcarbonyloxy-, Alkoxycarbonyl- und Alkoxycarbonyloxygruppen mit 1 bis 3 Kohlenstoffatomen im Alkylteil, Formyloxy-, Carboxyl-, Carbamoyl-, N-Alkyl- und N,N-Dialkylcarbamoylgruppen mit jeweils 1 bis 4 Kohlenstoffatomen in jeder Alkylgruppe, Alkoxycarbonylaminogruppen mit 1 bis 4 Kohlenstoffatomen im Alkylteil, Cyan-, Mercapto-, Alkylmercapto-, Alkylsulfinyl- und Alkylsulfonylgruppen mit 1 bis 4 Kohlenstoffatomen, Sulfamoyl-, N-Alkyl- und N,N-Dialkylsulfamoylgruppen mit 1 bis 4 Kohlenstoffatomen in jeder Alkylgruppe, Sulfo-, Alkoxysulfonyl-, Sulfamoyloxy-, Alkyl- und Dialkylsulfamoyloxygruppen mit 1 bis 4 Kohlenstoffatomen in jeder Alkylgruppe, geradkettige oder verzweigte Alkylgruppen mit 1 bis 6, vorzugsweise 1 bis 3 Kohlenstoffatomen, die mit Halogenatomen substituiert sein können, bedeuten, des weiteren kann R eine Gruppe der allgemeinen Formel

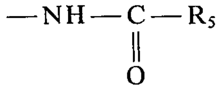

darstellen, in der $R_5$ ein Wasserstoffatom oder eine Alkylgruppe mit 1 bis 3 Kohlenstoffatomen darstellt.

Besonders bevorzugt sind diejenigen Verbindungen der allgemeinen Formel I, worin
A die Phenyl-, p-Hydroxyphenyl-, 2-Thienyl- und 1,4-Cyclohexadien-1-yl-gruppe und R die folgenden Bedeutungen besitzt:
ein Wasserstoffatom, die Cyclopropylgruppe, eine Gruppe der allgemeinen Formel

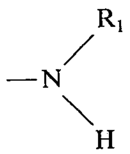

worin $R_1$ einen verzweigten oder unverzweigten Alkylrest mit 3 bis 5 Kohlenstoffatomen, einen Cycloalkylrest mit 3 bis 5 Kohlenstoffatomen, einen Cycloalkylrest mit 3 bis 6 Kohlenstoffatomen oder die Allyl- oder Propargylgruppe bedeutet,
des weiteren bedeutet R eine Gruppe der allgemeinen Formel

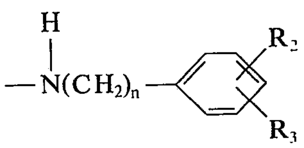

worin
n die Zahlen 0 oder 1 und einer oder beide Reste $R_2$ und $R_3$ Halogenatome, insbesondere Brom-, Chlor- oder Fluoratome, Methyl-, Äthyl-, Isopropyl-, Amino-, Methylamino-, Dimethylamino-, Hydroxy-, Methoxy-, Äthoxy-, Nitro-, Acetylamino-, Acetyl-, Methylcarbonyloxy-, Methoxycarbonyl-, Carboxyl-, Carbamoyl-, Methyl- und Dimethylcarbamoyl-, Cyan-, Methylmercapto-, Methylsulfinyl-, Äthylsulfinyl-, Propylsulfinyl- oder Isopropylsulfinyl- oder Methylsulfonyl-, Äthylsulfonyl-, Propylsulfonyl- oder Isopropylsulfonyl-, Carbamoylamino-, Sulfamoyl-, N-Methylsulfamoyl-, N-Äthylsulfamoyl-, N-Propylsulfamoyl-, N-Isopropylsulfamoyl- oder N,N-Dimethylsulfamoyl- oder Trifluormethylgruppen darstellen und der übrige Rest Wasserstoff bedeutet.

Die Penicillinverbindungen der allgemeinen Formel I können in zwei tautomeren Formen (nämlich vom Lactim- und vom Lactamtyp) vorliegen. Es hängt besonders vom jeweiligen Lösemittel und von der Art des Substituenten R ab, welche der beiden Formen I oder I′ überwiegt:

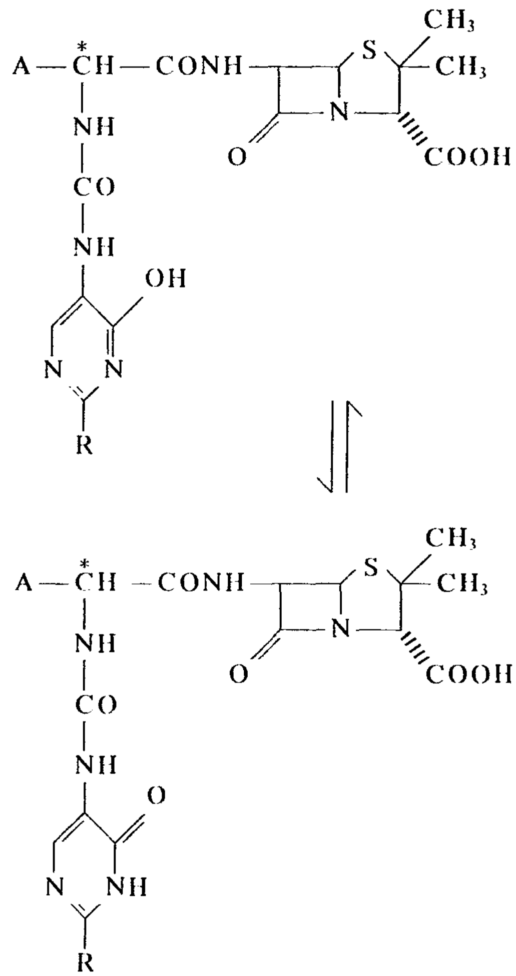

(I)

(I')

Es versteht sich von selbst, daß die eingangs angegebenen Verbindungen vom Typ I immer beide Tautomeren umfassen.

Die Verbindungen der allgemeinen Formel I können bezüglich des Chiralitätszentrums C+ in den beiden möglichen R- und S-Konfigurationen, jedoch auch als ein Gemisch dieser beiden Konfigurationen, vorliegen. Besonders bevorzugt sind solche Verbindungen, für welche die D = R-Konfiguration zutrifft.

Die neuen halbsynthetischen Penicilline der allgemeinen Formel I zeigen ein breites Wirkungsspektrum gegen grampositive und gramnegative Bakterien bei einer sehr guten Verträglichkeit beim Menschen.

Bekanntlich inhibieren Penicillin-Antibiotika das Wachstum verschiedener grampositiver und gramnegativer Bakterien. Es ist ferner bekannt, daß nur wenige Penicilline gute Wirkung gegen wichtige gramnegative Problemkeime, die vor allem im Hospitalbereich auftreten, wie Pseudomonas und Klebsiella, besitzen. Während der letzten Jahre ist jedoch die Häufigkeit des Auftretens von Infektionen, die durch diese Keime, insbesondere durch Pseudomonas-Arten, hervorgerufen wurde, ständig gestiegen. Penicillin-Derivate wie Carbenicillin (US-Patentschrift 3 142 673), Sulbenicillin (US-Patentschrift 3 660 379) sowie Ticarcillin (US-Patentschrift 3 282 926) werden zwar als antipseudomonale Antibiotika beschrieben, weisen jedoch in vitro wie in vivo nur eine mäßige Wirksamkeit auf. Eine wichtige Weiterentwicklung sind acylierte Derivate von α-Aminobenzylpenicillinen, z. B. Ampicillin und Amoxycillin. Aus diesen, in den letzten Jahren intensiv bearbeiteten Verbindungsklassen wurde vor kurzem Azlocillin = 6-{D-α-[(2-Oxo-imidazolidin-1-yl)-carbonylamino]-4-phenylacetamino}-penicillansäure-Natriumsalz (z. B. belgische Patentschrift 767 647) als weiteres Antipseudomonas-Penicillin eingeführt. Für eine erfolgreiche Behandlung muß dieses Penicillin jedoch hoch dosiert werden. Außerdem ist seine Wirkung gegen Klebsiella und E. coli-Arten nur mäßig. Es besteht daher weiter ein Bedürfnis, nach neuen Penicillinen zu suchen, die eine gesteigerte Wirksamkeit gegen Bakterien, wie Pseudomonas bzw. Klebsiella und E. coli, besitzen.

Während wie erwähnt, allgemein über Acylderivate von α-Aminobenzylpenicillinen intensiv geforscht wurde und noch wird, ist nur wenig über Derivate bekanntgeworden, bei denen ein Heterocyclus über eine Ureido-Brücke

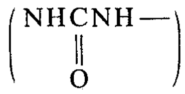

an das α-Benzylkohlenstoffatom von α-Aminobenzylpenicillinen geknüpft ist. Lediglich in der DE-OS 2 450 668 und 2 535 655 und der US-Patentschrift 4 031 230 werden Hydroxypyridylureido-benzylpenicilline der allgemeinen Formel II beschrieben:

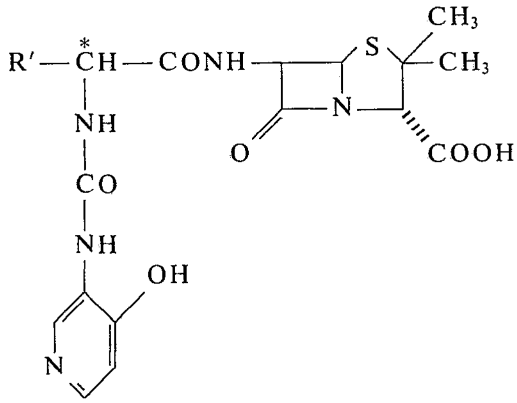

(II)

Diese Verbindungen sind den neuen erfindungsgemäßen Penicillinen strukturell am nächsten. Wie jedoch später in der Tabelle 1 gezeigt wird, zeichnen sich eine Reihe der erfindungsgemäßen neuen Penicilline durch eine erheblich stärkere antibakterielle Aktivität, insbesondere gegen Bakterien wie E. coli, Pseudomonas und Klebsiella, aus.

Die Verbindungen der allgemeinen Formel I lassen sich wie folgt darstellen:

1) Durch Umsetzung einer Verbindung der allgemeinen Formel III,

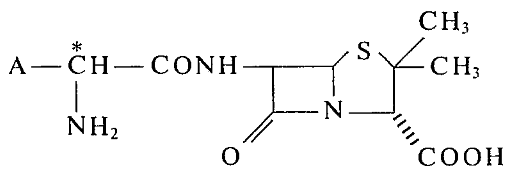

(III)

in der A wie oben definiert ist, mit einem Pyrimidinderivat der allgemeinen Formel IV,

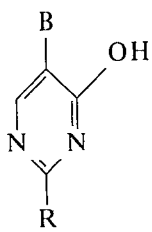

(IV)

in der R wie oben definiert ist und B die Gruppe —NCO oder ein reaktives Derivat der Gruppe NHCOOH bedeutet, wie z. B. die Gruppen

—NHCOCl, —NHCOBr oder 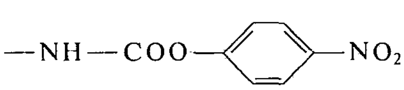

wobei die Gruppe NHCOCl besonders bevorzugt ist. Es können auch Gemische von solchen Pyrimidinderivaten der allgemeinen Formel IV verwendet werden, in der B teils die eine und teils die andere der vorstehend genannten Bedeutungen besitzt, z. B. die Gruppen

—NCO und 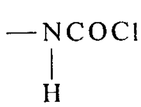

gleichzeitig nebeneinander.

Die Ausgangsprodukte der allgemeinen Formel III können in Form ihrer anorganischen oder organischen Salze, z. B. als Triäthylammoniumsalz oder Natriumsalz, eingesetzt werden. Die Reaktion

kann dann in beliebigen Mischungen von Wasser mit solchen organischen Lösungsmitteln, die mit Wasser mischbar sind, wie Ketonen, z. B. Aceton, cyclische Äther, z. B. Tetrahydrofuran oder Dioxan, Nitrilen, z. B. Acetonitril, Formamiden, z. B. Dimethylformamid, Dimethylsulfoxid oder Alkoholen, z. B. Isopropanol oder in Hexametapol durchgeführt werden. Dabei hält man den pH der Reaktionsmischung durch Zusatz von Basen oder Verwendung von Pufferlösungen in einem pH-Bereich von etwa 2,0 bis 9,0, vorzugsweise zwischen pH 6,5 und 8,0. Es ist aber auch möglich, die Reaktion in wasserfreien organischen Lösungsmitteln, z. B. halogenierten Kohlenwasserstoffen, wie Chloroform oder Methylenchlorid unter Zusatz von Basen, vorzugsweise Triäthylamin, Diäthylamin oder N-Äthylpiperidin durchzuführen. Weiterhin läßt sich die Reaktion in einem Gemenge aus Wasser und einem mit Wasser nicht mischbaren Lösungsmittel, wie Äther, z. B. Diäthyläther, halogenierten Kohlenwasserstoffen, z. B. Chloroform oder Methylenchlorid, Schwefelkohlenstoff, Ketonen, z. B. Isobutylmethylketon, Estern, z. B. Essigsäureäthylester, aromatischen Lösungsmitteln, z. B. Benzol, ausführen, wobei es zweckmäßig ist, kräftig zu rühren, und den pH-Wert durch Basenzusatz oder Verwendung von Pufferlösungen in einem Bereich von etwa pH 2,0 bis 9,0, vorzugsweise zwischen 6,5 und 8,0, zu halten. Man kann die Reaktion aber auch in Wasser allein in Gegenwart einer organischen oder anorganischen Base oder unter Zusatz von Pufferstoffen durchführen.

Verwendet man als Ausgangsprodukte für das erfindungsgemäße Verfahren die Silylderivate der Verbindungen der allgemeinen Formel III (z. B. Mono- oder Di-trimethylsilylderivate) und setzt sie mit Verbindungen der allgemeinen Formel IV um, so arbeitet man im allgemeinen zweckmäßigerweise in wasser- und hydroxyl-gruppenfreien Lösungsmitteln, beispielsweise in halogenierten Kohlenwasserstoffen, z. B. Methylenchlorid oder Chloroform, Benzol, Tetrahydrofuran, Aceton oder Dimethylformamid usw. Der Zusatz von Basen ist hierbei nicht notwendig, kann jedoch in einzelnen Fällen vorteilhaft sein, um die Ausbeute und Reinheit der Produkte zu verbessern. Als gegebenenfalls zugesetzte Basen werden zweckmäßigerweise tertiäre aliphatische oder aromatische Amine, wie Pyridin oder Triäthylamin, oder durch sterische Hinderung schweracylierbare sekundäre Amine, wie Dicyclohexylamin, benützt.

Statt der Silylester können auch alle anderen Carboxylderivate von $\alpha$-Aminobenzylpenicillinen, die auf dem Gebiet der Herstellung halbsynthetischer Penicilline bekannt sind, verwendet werden. Typische Beispiele sind die Tritylester, die p-Nitrobenzylester oder die Phenacylester. Im Anschluß an die Umsetzungen können diese Derivate nach bekannten Methoden in die erfindungsgemäßen Penicilline umgewandelt werden. Die Menge der verwendeten Basen ist z. B. durch die gewünschte Einhaltung eines bestimmten pH-Wertes festgelegt. Wo eine pH-Messung und Einstellung nicht erfolgt oder wegen des Fehlens von ausreichenden Mengen Wasser im Verdünnungsmittel nicht möglich oder nicht sinnvoll ist, werden im Falle der Verwendung der nichtsilylierten Verbindungen der allgemeinen Formel II vorzugsweise 1,0 bis 2,0 Moläquivalente Basen eingesetzt. Im Falle der Verwendung der silylierten Verbindungen verwendet man vorzugsweise bis zu einem Moläquivalent Base.

Als Basen können prinzipiell alle in der organischen Chemie üblicherweise verwendeten organischen und anorganischen Basen verwendet werden, wie Alkali- und Erdalkalihydroxide, Erdalkalioxide, Alkali- und Erdalkalicarbonate und Hydrogencarbonate, Ammoniak, primäre, sekundäre und tertiäre aliphatische und aromatische Amine sowie heterocyclische Basen. Beispielhaft seien Natrium-, Kalium- und Calziumhydroxid, Calziumoxid, Natrium- und Kaliumcarbonat, Natrium- und Kaliumhydrogencarbonat, Äthylamin, Methyl-äthylamin, Triäthylamin, Hydroxyäthylamin, Anilin, Pyridin und Piperidin genannt. Bei Verwendung der silylierten Ausgangsstoffe sollten jedoch die oben angegebenen Einschränkungen bezüglich der Art der Basen beachtet werden.

Als Puffersystem können alle üblichen Puffermischungen verwendet werden, z. B. Phosphatpuffer, Citratpuffer und Tris(hydroxymethyl)amino-methan-Puffer.

Die Reaktionstemperaturen können in einem größeren Bereich variiert werden. Im allgemeinen arbeitet man zwischen etwa $-20$ und etwa $+50°C$, vorzugsweise zwischen 0 und $+20°C$.

Die Reaktionspartner der allgemeinen Formeln (III) und (IV) können von vornherein in äquimolaren Mengen miteinander zur Reaktion gebracht werden. Es kann aber in einzelnen Fällen durchaus zweckmäßig sein, einen der beiden Reaktionspartner im Überschuß zu verwenden, um sich damit die Reinigung des Endproduktes zu erleichtern oder um die Ausbeute zu steigern.

2) Durch Umsetzung von Ureidocarbonsäuren der allgemeinen Formel V,

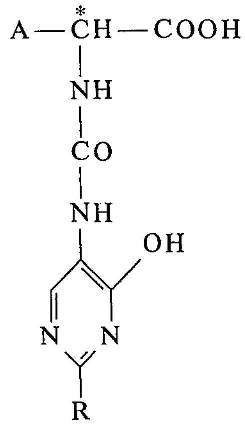

(V)

in der A und R die oben angegebenen Bedeutungen besitzen, ihren Salzen oder reaktiven Derivaten, mit der 6-Aminopenicillansäure der Formel VI,

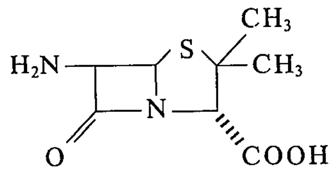

(VI)

oder ihren anorganischen oder organischen Salzen oder Derivaten, die in die 6-Aminopenicillansäure leicht überführbar sind. Das dabei entstehende Reaktionsprodukt wird gegebenenfalls anschließend zu einem Penicillin der allgemeinen Formel I hydrolysiert oder katalytisch hydrogenolisiert.

Als reaktive Derivate der Ureidocarbonsäuren der allgemeinen Formel V kommen beispielsweise deren Säureanhydride wie z. B. die, die sich von Chlorameisensäureestern, z. B. Chlorameisensäureäthyl- oder -isobutylester, ableiten, oder deren reaktive Ester, wie der p-Nitrophenylester oder der N-Hydroxysuccinimidester, oder deren reaktive Amide, wie das N-Carbonylimidazol, aber auch deren Säurehalogenide, wie das entsprechende Säurechlorid oder deren Säureazide in Frage. Prinzipiell können jedoch alle Verknüpfungsmethoden, wie sie aus der β-Lactamchemie bekannt sind, verwendet werden.

Die 6-Aminopenicillansäure wird vorteilhafterweise in Form eines ihrer Derivate eingesetzt. Als Derivate kommen hierfür z. B. in Frage: ihr Trimethylsilylester, Tritylester, p-Nitrobenzylester, Phenacylester und ihr O,N-Bis-trimethylsilylderivat. Diese Derivate werden bevorzugt in einem aprotischen Lösungsmittel, wie Methylenchlorid oder Tetrahydrofuran, umgesetzt. Man kann aber auch die 6-Aminopenicillansäure in Form ihrer Salze, beispielsweise ihres Triäthylammoniumsalzes, zum Einsatz bringen; hierbei benutzt man z. B. Methylenchlorid oder ein protisches Lösungsmittel oder ein wäßriges Medium oder ein wäßrig-organisches Lösungsmittel, wie z. B. Tetrahydrofuran-Wasser-Gemische.

Die Ureidocarbonsäure, ihre Salze oder ihre reaktiven Derivate werden mit der 6-Aminopenicillansäure oder ihren Derivaten in einem Lösungsmittel bei Temperaturen zwischen −40°C und +40°C, gegebenenfalls in Gegenwart einer Base, umgesetzt. Wird z. B. ein Anhydrid der Ureidocarbonsäure, beispielsweise das Anhydrid mit dem Äthylchloroformiat, mit einem Derivat der 6-Aminopenicillansäure umgesetzt, so wird die Reaktion unter Kühlung, beispielsweise bei −10°C bis +10°C in Gegenwart eines tertiären Amins, wie Triäthylamin oder N,N-Dimethylanilin, in einem Lösungsmittel, wie Aceton, Tetrahydrofuran, Dimethylformamid, Chloroform, Dichlormethan, Hexametapol oder in einem Gemisch dieser Lösungsmittel, durchgeführt. Setzt man beispielsweise einen N-Hydroxysuccinimidester der Ureidocarbonsäure mit der 6-Aminopenicillansäure um, so wird die Reaktion vorzugsweise bei 0 bis 20°C in Abwesenheit einer Base, wie z. B. Triäthylamin, in einem Lösungsmittel wie Dimethylformamid, Dichlormethan, Dioxan oder in einem Gemisch solcher Lösungsmittel durchgeführt.

Die Umsetzung einer Ureidocarbonsäure der allgemeinen Formel IV selbst oder ihrer Salze mit der 6-Aminopenicillansäure oder mit deren Salzen erfolgt vorteilhafterweise in Gegenwart eines Kondensationsmittels, z. B. in Gegenwart von N,N′-Dicyclohexylcarbodiimid.

Wird ein Derivat der 6-Aminopenicillansäure eingesetzt, beispielsweise einer ihrer obengenannten Ester, so wird je nach den Reaktionsbedingungen gegebenenfalls ein Reaktionsprodukt erhalten, welches z. B. noch die Esterfunktion enthält. Ein solches Reaktionsprodukt ist aber leicht in das Penicillin der allgemeinen Formel I überführbar. Liegt zum Beispiel die Carboxylgruppe der 6-Aminopenicillansäure in Form ihres Silylesters vor, so kann sie nach der Reaktion in dem erhaltenen Penicillin der allgemeinen Formel I ebenfalls in Form ihres Silylesters vorliegen. In diesem Fall wird anschließend an die eigentliche Umsetzung diese Silylestergruppe abhydrolysiert, wodurch die

Verbindung der allgemeinen Formel I entsteht. In anderen Fällen, z. B. bei Vorliegen eines p-Nitrobenzylesters, wird nach der eigentlichen Umsetzung diese p-Nitrobenzylestergruppe hydrogenolytisch gespalten, wobei dann das Penicillin der allgemeinen Formel I erhalten wird.

Die Aufarbeitung des nach beiden Verfahren erhaltenen Reaktionsgemisches nach erfolgter Umsetzung wird nach den bei β-Lactam-Antibiotika gebräuchlichen Methoden vorgenommen; dasselbe gilt für die Isolierung und Reinigung der Endprodukte, beispielsweise für die Freisetzung der Säure aus ihren Salzen und die Überführung der freien Säure in andere Salze mittels anorganischer oder organischer Basen. Für die Herstellung der Kalium- oder Natriumsalze bewährt sich besonders die Fällung dieser Salze aus einer alkoholisch-ätherischen Lösung der freien Säure durch die Zugabe von Kalium- oder Natrium-2-äthylhexanoat.

Die als Ausgangsstoffe verwendeten Verbindungen der allgemeinen Formel III sind literaturbekannt, vgl. z. B. E. H. Flynn, Cephalosporines and Penicillines, Academic Press, New York and London (1972).

Die Ausgangsstoffe der allgemeinen Formel IV können beispielsweise durch Umsetzung der entsprechenden 5-Aminopyrimidine der allgemeinen Formel VII,

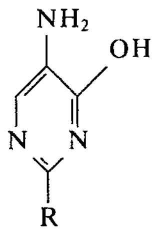

(VII)

in der R wie oben definiert ist, mit Phosgen gewonnen werden. Diese Umsetzung erfolgt vorzugsweise in einem nicht Hydroxylgruppen enthaltenden Lösungsmittel, wie Tetrahydrofuran, Methylenchlorid, Chloroform, Dimethoxyäthan oder Hexametapol bei Temperaturen zwischen −40 und +60° C, vorzugsweise zwischen −10° und +20° C. Dabei empfiehlt es sich, den entstehenden Chlorwasserstoff durch äquimolare Mengen einer inerten organischen Base, wie Triäthylamin oder Pyridin, zu binden. Als Lösungsmittel kann auch Pyridin im Überschuß verwendet werden. Sollten die betreffenden Aminopyrimidine der allgemeinen Formel VII in einem der erwähnten Lösungsmittel schwer löslich sein, kann die Phosgenierung auch in heterogener Phase durchgeführt werden. Des weiteren können die Aminopyrimidine der allgemeinen Formel VII durch Behandlung mit einem Silylierungsmittel, wie Hexamethyldisilazan oder Trimethylchlorsilan/Triäthylamin, in ein im allgemeinen in den erwähnten Lösungsmitteln sehr leicht lösliches, einfach oder, entsprechend den vorhandenen austauschbaren Wasserstoffatomen, mehrfach silyliertes Aminopyrimidin überführt werden, das mit Phosgen dann zu den entsprechenden Verbindungen der allgemeinen Formel IV reagiert. Je nach der Art des Lösungsmittels, der Höhe der Temperatur, der Menge und Art der eingesetzten Base entsteht entweder überwiegend das entsprechende Isocyanat oder Carbaminsäurehalogenid oder ein Gemisch dieser beiden Verbindungen. Je nach den Reaktionsbedingungen kann die Verbindung der allgemeinen Formel IV auch gering oder teilweise als ein, den Isocyanaten isomeres Tetrahydrooxazolo-pyrimidin der allgemeinen Formel IVa

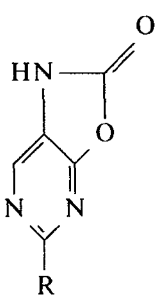

(IVa)

vorliegen.

Die durch Phosgenierung entstandenen Ausgangsprodukte der allgemeinen Formel IV bzw. deren Gemische sind in den obenerwähnten Lösungsmitteln im allgemeinen gut löslich und können, nach Entfernung des überschüssigen Phosgens, ohne weitere Reinigung mit den entsprechenden Penicillinderivaten der allgemeinen Formel III direkt umgesetzt werden.

2-Substituierte 5-Amino-4-hydroxy-pyrimidine der allgemeinen Formel VII sind in der Literatur bis jetzt kaum beschrieben, z. B. für

R = Wasserstoff: J. Chem. Soc. 1952, 4942;
= Hydroxy: J. Am. Chem. Soc. 46, 702 (1924);
= Dimethylamino: J. Chem. Soc. 1956, 3232.

Für die Darstellung der Pyrimidine der allgemeinen Formel VII wurden daher mehrere Verfahren ausgearbeitet, von denen die wichtigsten hier angegeben werden, wobei je nach Bedeutung des Restes R für die Synthese von VII das günstigste Verfahren anzuwenden ist.

a) Umsetzung von Äthyl-carbäthoxyamino-formylacetat-Natriumsalz der Formel VIII mit Verbindungen der allgemeinen Formel IX, in denen R wie oben definiert ist und anschließender alkalischer Hydrolyse gemäß dem Reaktionsschema:

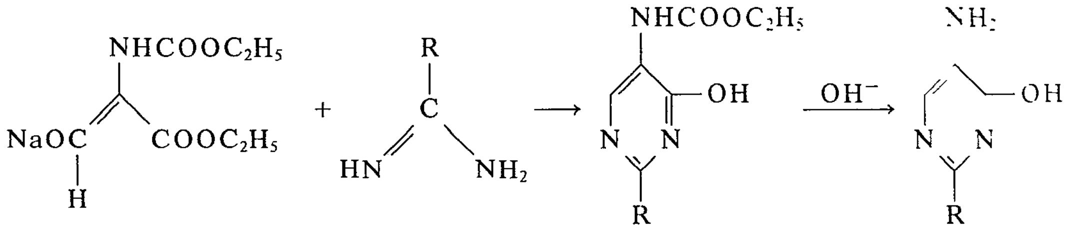


(VIII) (IX) (VII)

b) Umsetzung von Äthoxymethylen-nitroessigsäure-äthylester der Formel X mit Verbindungen der Formel IX und anschließender Reduktion der Nitrogruppe nach bekannten Methoden gemäß dem Reaktionsschema:

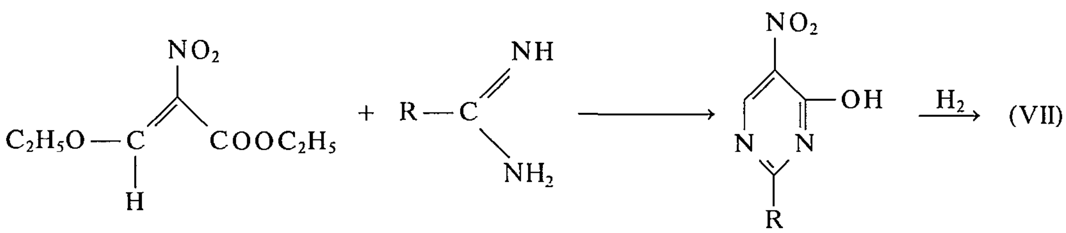


(X) (IX)

c) Umsetzung von 2-Phenyl-4-äthoxymethylen-5-oxo-2-oxazolin der Formel XI mit Verbindungen der Formel IX und anschließender saurer oder alkalischer Hydrolyse (vgl. auch Clarke, Johnson und Robinson, The Chemistry of Penicillins, Princeton University Press 1949, S. 803)

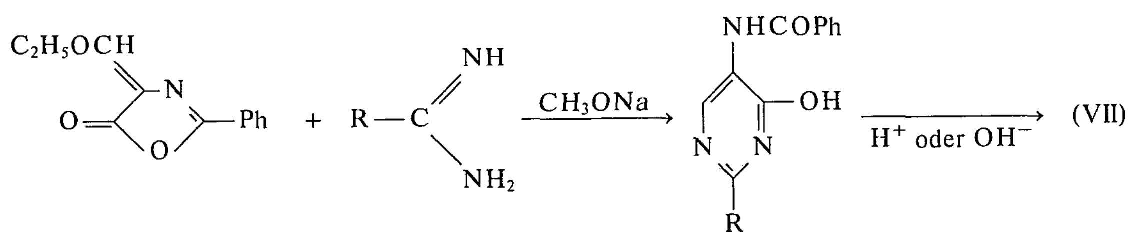


(XI) (IX)

d) Acylierung von 2-Amino-4-hydroxy-5-nitropyrimidin der Formel XII und anschließender Reduktion

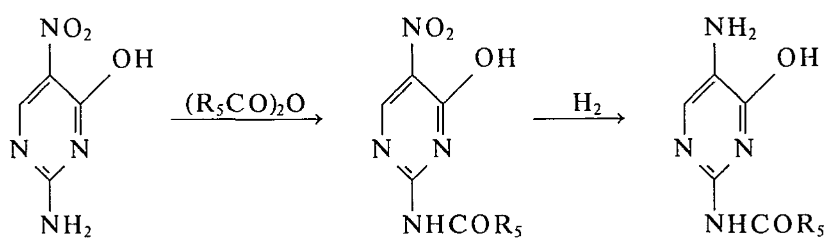


(XII)

Hierbei entstehen Verbindungen der allgemeinen Formel VII, in der R die Gruppe NHCO $R_5$ bedeutet.

e) Umsetzung von 2-Methylmercapto-4-hydroxy-5-nitropyrimidinen der allgemeinen Formel XIIIa (Lit.: Vorbrüggen u. Strehlke Chem. Ber. 106, S. 3039 [1973]) oder von 5-Benzoylamino-4-hydroxy-2-methylmercaptopyrimidin der allgemeinen Formel XIIIb (oder seiner am S oxidierten Derivate) mit

substituierten Aminen und anschließender Reduktion bzw. Hydrolyse:

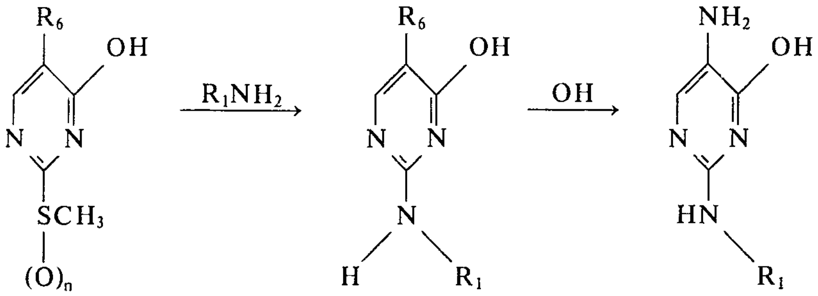


(VII)

(n = 0, 1 oder 2)
XIIIa $R_6 = NO_2$
XIIIb $R_6 = NHCOC_6H_5$

Hierbei entstehen Verbindungen der allgemeinen Formel VII, in der R die Gruppe $NHR_1$ bedeutet.

Zur Charakterisierung der so gewonnenen Ausgangsprodukte der allgemeinen Formel VII sollen hier typische Vertreter genannt werden.

5-Amino-2-methyl-4-hydroxy-pyrimidin
5-Amino-2-cyclopropyl-4-hydroxy-pyrimidin
5-Amino-2-(1'-methyl)-cyclopropyl-4-hydroxy-pyrimidin
5-Amino-2-(2'-methyl)-cyclopropyl-4-hydroxy-pyrimidin
5-Amino-2-cyclobutyl-4-hydroxy-pyrimidin
5-Amino-2,4-dihydroxy-pyrimidin
5-Amino-2-methoxy-4-hydroxy-pyrimidin
2,5-Diamino-4-hydroxy-pyrimidin
5-Amino-2-äthoxy-4-hydroxy-pyrimidin
5-Amino-2-methylamino-4-hydroxy-pyrimidin
5-Amino-2-dimethylamino-4-hydroxy-pyrimidin
5-Amino-2-äthylamino-4-hydroxy-pyrimidin
5-Amino-2-propylamino-4-hydroxy-pyrimidin
5-Amino-2-isopropylamino-4-hydroxy-pyrimidin
5-Amino-2-butylamino-4-hydroxy-pyrimidin
5-Amino-2-hexylamino-4-hydroxy-pyrimidin
5-Amino-2-allylamino-4-hydroxy-pyrimidin
5-Amino-2-propargylamino-4-hydroxy-pyrimidin
5-Amino-2-cyclopropylamino-4-hydroxy-pyrimidin
5-Amino-2-cyclobutylamino-4-hydroxy-pyrimidin
5-Amino-2-cyclohexylamino-4-hydroxy-pyrimidin
5-Amino-2-cycloheptylamino-4-hydroxy-pyrimidin
5-Amino-2-cyclopropylmethylamino-4-hydroxy-pyrimidin
5-Amino-2-pyrrolidino-4-hydroxy-pyrimidin
5-Amino-2-piperidino-4-hydroxy-pyrimidin
5-Amino-2-morpholino-4-hydroxy-pyrimidin
5-Amino-2-anilino-4-hydroxy-pyrimidin
5-Amino-2-p-chloranilino-4-hydroxy-pyrimidin
5-Amino-2-p-dimethylaminoanilino-4-hydroxy-pyrimidin
5-Amino-2-o-chloranilino-4-hydroxy-pyrimidin
5-Amino-2-m,p-dichloranilino-4-hydroxy-pyrimidin
5-Amino-2-p-hydroxyanilino-4-hydroxy-pyrimidin
5-Amino-2-p-methylanilino-4-hydroxy-pyrimidin
5-Amino-2-p-acetylamino-anilino-4-hydroxy-pyrimidin
5-Amino-2-p-fluoranilino-4-hydroxy-pyrimidin
5-Amino-2-m-chloranilino-4-hydroxy-pyrimidin
5-Amino-2-p-bromanilino-4-hydroxy-pyrimidin
5-Amino-2-(p-chlor-m-trifluormethyl)anilino-4-hydroxy-pyrimidin
5-Amino-2-p-trifluormethylanilino-4-hydroxy-pyrimidin
5-Amino-2-p-hydroxyanilino-4-hydroxy-pyrimidin
5-Amino-2-benzylamino-4-hydroxy-pyrimidin
5-Amino-2-p-chlorbenzylamino-4-hydroxy-pyrimidin
5-Amino-2-formylamino-4-hydroxy-pyrimidin
5-Amino-2-acetylamino-4-hydroxy-pyrimidin

5-Amino-2-propionylamino-4-hydroxy-pyrimidin
5-Amino-2-isobutyrylamino-4-hydroxy-pyrimidin
5-Amino-2-butyrylamino-4-hydroxy-pyrimidin

Die Ureidocarbonsäure der allgemeinen Formel V lassen sich leicht durch Umsetzung der Pyrimidinderivate der allgemeinen Formel IV mit Glycinderivaten der allgemeinen Formel XIV,

$$A-\underset{NH_2}{\underset{|}{\overset{+}{C}H}}-COOH \qquad (XIV)$$

in der A wie oben definiert ist, erhalten. Die Umsetzung erfolgt bei Temperaturen zwischen −20° und +40°C, vorzugsweise zwischen 0° und +20°, in einem Lösungsmittel. Als Lösungsmittel können hierbei z. B. Gemische von Wasser und organischen Lösungsmitteln, die mit Wasser mischbar sind, verwendet werden, beispielsweise Aceton, Tetrahydrofuran, Dioxan, Acetonitril, Dimethylformamid, Äthanol, Dimethylsulfoxid. Gegebenenfalls wird die Verwendung eines halogenwasserstoffbindenden Mittels notwendig, als solche eignen sich beispielsweise Trialkylamine, wie Triäthylamin oder anorganische Basen, wie verdünnte Natronlauge.

Es wurde gefunden, daß die Verbindungen der allgemeinen Formel I wertvolle pharmakologische Eigenschaften bei guter Verträglichkeit besitzen. Die erfindungsgemäßen Wirkstoffe können daher zur Prophylaxe und Chemotherapie von lokalen und systemischen Infektionen in der Human- und Tiermedizin verwendet werden. Als Krankheiten, die durch die erfindungsgemäßen Verbindungen verhindert bzw. geheilt werden können, seien beispielsweise solche der Atmungswege, des Rachenraumes und der Harnwege genannt; die Verbindungen wirken insbesondere gegen Pharyngitis, Pneumonie, Peritonitis, Pyelonephritis, Otitis, Cystitis, Endocarditis, Bronchitis, Arthritis und allgemeine systemische Infektionen. Weiter können diese Verbindungen als Stoffe zur Konservierung von anorganischen oder organischen Materialien verwendet werden, besonders von organischen Materialien, wie Polymeren, Schmiermittel, Farben, Fasern Leder, Papier und Holz sowie von Lebensmitteln.

Dieses wird dadurch ermöglicht, daß die Verbindungen der allgemeinen Formel I sowohl in vitro als auch in vivo gegen schädliche Mikroorganismen, insbesondere gegen grampositive und gramnegative Bakterien und bakterienähnliche Mikroorganismen sehr stark wirken, wobei sie sich besonders durch ein breites Wirkungsspektrum auszeichnen. Daneben zeigen die Verbindungen der allgemeinen Formel I nach parenteraler Gabe hohe Spiegel in Gewebe, Serum, Organen und im Urin.

Überraschenderweise zeigen eine Reihe der erfindungsgemäßen Penicilline auch nach oraler Gabe bei der Ratte hohe Serum- und Gewebsspiegel, sowie hohe Urinspiegel. Dies war auf Grund der bisherigen Erfahrungen mit Acylderivaten von α-Aminobenzylpenicillinen nicht zu erwarten.

In der folgenden Tabelle werden typische besonders gut wirksame Penicilline gemäß vorliegender Erfindung aufgezählt. Die genannten Penicilline können nach dem Verfahren 1 oder 2 hergestellt werden:

Es handelt sich um Verbindungen der allgemeinen Formel I, in der A und R wie folgt definiert sind:

| A | R |
|---|---|
| Phenyl | Wasserstoff |
| p-Hydroxyphenyl | Wasserstoff |
| Cyclohexa-1,4-dien-1-yl | Wasserstoff |
| 2-Thienyl | Wasserstoff |
| p-Hydroxy-m-chlorphenyl | Wasserstoff |
| m,p-Dihydroxyphenyl | Wasserstoff |
| Phenyl | Methyl |
| p-Hydroxyphenyl | Methyl |
| Cyclohexa-1,4-dien-1-yl | Methyl |
| 2-Thienyl | Methyl |
| p-Hydroxy-m-chlorphenyl | Methyl |

Fortsetzung

| A | R |
|---|---|
| m,p-Dihydroxyphenyl | Methyl |
| Phenyl | Cyclopropyl |
| p-Hydroxyphenyl | Cyclopropyl |
| Cyclohexa-1,4-dien-1-yl | Cyclopropyl |
| 2-Thienyl | Cyclopropyl |
| p-Hydroxy-m-chlorphenyl | Cyclopropyl |
| m,p-Dihydroxyphenyl | Cyclopropyl |
| Phenyl | 2-Methyl-cyclopropyl |
| p-Hydroxyphenyl | 2-Methyl-cyclopropyl |
| Cyclohexa-1,4-dien-1-yl | 2-Methyl-cyclopropyl |
| Phenyl | Hydroxy |
| p-Hydroxyphenyl | Hydroxy |
| Cyclohexa-1,4-dien-1-yl | Hydroxy |
| 2-Thienyl | Hydroxy |
| p-Hydroxy-m-chlorphenyl | Hydroxy |
| m,p-Dihydroxyphenyl | Hydroxy |
| Phenyl | Methoxy |
| p-Hydroxyphenyl | Methoxy |
| Cyclohexa-1,4-dien-1-yl | Methoxy |
| 2-Thienyl | Methoxy |
| Phenyl | Äthoxy |
| p-Hydroxyphenyl | Äthoxy |
| Cyclohexa-1,4-dien-1-yl | Äthoxy |
| 2-Thienyl | Äthoxy |
| Phenyl | Amino |
| p-Hydroxyphenyl | Amino |
| Cyclohexa-1,4-dien-1-yl | Amino |
| Phenyl | Methylamino |
| p-Hydroxyphenyl | Methylamino |
| Cyclohexa-1,4-dien-1-yl | Methylamino |

Fortsetzung

| A | R |
|---|---|
| 2-Thienyl | Methylamino |
| p-Hydroxy-m-chlorphenyl | Methylamino |
| m,p-Dihydroxyphenyl | Methylamino |
| Phenyl | Äthylamino |
| p-Hydroxyphenyl | Äthylamino |
| Cyclohexa-1,4-dien-1-yl | Äthylamino |
| 2-Thienyl | Äthylamino |
| p-Hydroxy-m-chlorphenyl | Äthylamino |
| m,p-Dihydroxyphenyl | Äthylamino |
| Phenyl | Isopropylamino |
| p-Hydroxyphenyl | Isopropylamino |
| Cyclohexa-1,4-dien-1-yl | Isopropylamino |
| Phenyl | Allylamino |
| p-Hydroxyphenyl | Allylamino |
| Cyclohexa-1,4-dien-1-yl | Allylamino |
| Phenyl | Propargylamino |
| p-Hydroxyphenyl | Propargylamino |
| Phenyl | Cyclopropylamino |
| p-Hydroxyphenyl | Cyclopropylamino |
| Cyclohexa-1,4-dien-1-yl | Cyclopropylamino |
| p-Hydroxyphenyl | Propylamino |
| Phenyl | Propylamino |
| p-Hydroxyphenyl | Isobutylamino |
| Phenyl | Cyclopentylamino |
| p-Hydroxyphenyl | Cyclopentylamino |
| Cyclohexa-1,4-dien-1-yl | Cyclopentylamino |
| 2-Thienyl | Cyclopentylamino |
| p-Hydroxy-m-chlorphenyl | Cyclopentylamino |
| m,p-Dihydrophenyl | Cyclopentylamino |
| Phenyl | Cyclohexylamino |

Fortsetzung

| A | R |
|---|---|
| p-Hydroxyphenyl | Cyclohexylamino |
| Cyclohexa-1,4-dien-1-yl | Cyclohexylamino |
| 2-Thienyl | Cyclohexylamino |
| p-Hydroxy-m-chlorphenyl | Cyclohexylamino |
| m,p-Dihydroxyphenyl | Cyclohexylamino |
| Phenyl | Cycloheptylamino |
| p-Hydroxyphenyl | Cycloheptylamino |
| Phenyl | Cyclopropylmethylamino |
| p-Hydroxyphenyl | Cyclopropylmethylamino |
| Phenyl | Cyclohexylmethylamino |
| p-Hydroxyphenyl | Cyclohexylmethylamino |
| Phenyl | Pyrrolidino |
| p-Hydroxyphenyl | Pyrrolidino |
| Phenyl | Piperidino |
| Phenyl | Morpholino |
| p-Hydroxyphenyl | Morpholino |
| Phenyl | p-Chlorbenzylamino |
| p-Hydroxyphenyl | p-Chlorbenzylamino |
| Cyclohexa-1,4-dien-1-yl | p-Chlorbenzylamino |
| 2-Thienyl | p-Chlorbenzylamino |
| p-Hydroxy-m-chlorphenyl | p-Chlorbenzylamino |
| m,p-Dihydroxyphenyl | p-Chlorbenzylamino |
| Phenyl | Benzylamino |
| p-Hydroxyphenyl | Benzylamino |
| p-Hydroxy-m-chlorphenyl | Benzylamino |
| m,p-Dihydroxyphenyl | Benzylamino |
| Phenyl | Anilino |
| p-Hydroxyphenyl | Anilino |
| Cyclohexa-1,4-dien-1-yl | Anilino |
| Phenyl | p-Chloranilino |

Fortsetzung

| A | R |
|---|---|
| p-Hydroxyphenyl | p-Chloranilino |
| Cyclohexa-1,4-dien-1-yl | p-Chloranilino |
| 2-Thienyl | p-Chloranilino |
| p-Hydroxy-m-chlorphenyl | p-Chloranilino |
| m,p-Dihydroxyphenyl | p-Chloranilino |
| Phenyl | m,p-Dichloranilino |
| p-Hydroxyphenyl | m,p-Dichloranilino |
| Cyclohexa-1,4-dien-1-yl | m,p-Dichloranilino |
| 2-Thienyl | m,p-Dichloranilino |
| p-Hydroxy-m-chlorphenyl | m,p-Dichloranilino |
| m,p-Dihydroxyphenyl | m,p-Dichloranilino |
| Phenyl | p-Fluoranilino |
| p-Hydroxyphenyl | p-Fluoranilino |
| Phenyl | m-Chloranilino |
| p-Hydroxyphenyl | m-Chloranilino |
| Cyclohexa-1,4-dien-1-yl | m-Chloranilino |
| Phenyl | p-Hydroxyanilino |
| p-Hydroxyphenyl | p-Hydroxyanilino |
| Cyclohexa-1,4-dien-1-yl | p-Hydroxyanilino |
| 2-Thienyl | p-Hydroxyanilino |
| Phenyl | p-Methylanilino |
| p-Hydroxyphenyl | p-Methylanilino |
| Cyclohexa-1,4-dien-1-yl | p-Methylanilino |
| 2-Thienyl | p-Methylanilino |
| p-Hydroxy-m-chlorphenyl | p-Methylanilino |
| m,p-Dihydroxyphenyl | p-Methylanilino |
| Phenyl | p-Isopropylanilino |
| p-Hydroxyphenyl | p-Isopropylanilino |
| Cyclohexa-1,4-dien-1-yl | p-Isopropylanilino |
| 2-Thienyl | p-Isopropylanilino |

Fortsetzung

| A | R |
|---|---|
| p-Hydroxy-m-chlorphenyl | p-Isopropylanilino |
| m,p-Dihydroxyphenyl | p-Isopropylanilino |
| Phenyl | p-Acetylaminoanilino |
| p-Hydroxyphenyl | p-Acetylaminoanilino |
| Cyclohexa-1,4-dien-1-yl | p-Acetylaminoanilino |
| 2-Thienyl | p-Acetylaminoanilino |
| p-Hydroxy-m-chlorphenyl | p-Acetylaminoanilino |
| m,p-Dihydroxyphenyl | p-Acetylaminoanilino |
| Phenyl | p-Dimethylanilino |
| p-Hydroxyphenyl | p-Dimethylanilino |
| Cyclohexa-1,4-dien-1-yl | p-Dimethylanilino |
| 2-Thienyl | p-Dimethylanilino |
| p-Hydroxy-m-chlorphenyl | p-Dimethylanilino |
| m,p-Dihydroxyphenyl | p-Dimethylanilino |
| Phenyl | p-Trifluormethylanilino |
| p-Hydroxyphenyl | p-Trifluormethylanilino |
| Cyclohexa-1,4-dien-1-yl | p-Trifluormethylanilino |
| 2-Thienyl | p-Trifluormethylanilino |
| p-Hydroxy-m-chlorphenyl | p-Trifluormethylanilino |
| m,p-Dihydroxyphenyl | p-Trifluormethylanilino |
| Phenyl | p-Chlor-m-trifluormethylanilino |
| p-Hydroxyphenyl | p-Chlor-m-trifluormethylanilino |
| Cyclohexa-1,4-dien-1-yl | p-Chlor-m-trifluormethylanilino |
| 2-Thienyl | p-Chlor-m-trifluormethylanilino |
| p-Hydroxy-m-chlorphenyl | p-Chlor-m-trifluormethylanilino |
| m,p-Dihydroxyphenyl | p-Chlor-m-trifluormethylanilino |
| Phenyl | m,m-Dichloranilino |
| p-Hydroxyphenyl | m,m-Dichloranilino |
| Phenyl | m,m-Dichlor-p-aminoanilino |
| p-Hydroxyphenyl | m,m-Dichlor-p-aminoanilino |

Fortsetzung

| A | R |
|---|---|
| Cyclohexa-1,4-dien-1-yl | m,m-Dichlor-p-aminoanilino |
| Phenyl | Formylamino |
| p-Hydroxyphenyl | Formylamino |
| Phenyl | Acetylamino |
| p-Hydroxyphenyl | Acetylamino |
| Cyclohexa-1,4-dien-1-yl | Acetylamino |
| 2-Thienyl | Acetylamino |
| p-Hydroxy-m-chlorphenyl | Acetylamino |
| m,p-Dihydroxyphenyl | Acetylamino |
| Phenyl | Propionylamino |
| p-Hydroxyphenyl | Propionylamino |
| Cyclohexa-1,4-dien-1-yl | Propionylamino |
| 2-Thienyl | Propionylamino |
| p-Hydroxy-m-chlorphenyl | Propionylamino |
| m,p-Dihydroxyphenyl | Propionylamino |
| Phenyl | Isobutyrylamino |
| p-Hydroxyphenyl | Isobutyrylamino |

Mit diesen Penicillinderivaten können beispielsweise lokale und/oder systematische Erkrankungen behandelt und/oder verhindert werden, die durch die folgenden Erreger oder durch Mischungen der folgenden Erreger verursacht werden:

Micrococcaceae, wie Staphylokokken;
Lactobacteriaceae, wie Streptokokken;
Neisseriaceae, wie Neisserien;
Corynebacteriaceae, wie Corynebakterien;
Enterobacteriaceae, wie Escherichiae-Bakterien der Coli-Gruppe,
Klebsiella-Bakterien, z. B. K. pneumonia;
Proteae-Bakterien der Proteus-Gruppe, z. B. Proteus vulgaris;
Salmonella-Bakterien, z. B. S. thyphimurium;
Shigella-Bakterien, z. B. Shigella dysenteriae,
Pseudomonas-Bakterien, z. B. Pseudomonas aeruginosa;
Aeromonas-Bakterien, z. B. Aeromonas lique faciens.

Spirillaceae, wie Vibrio-Bakterien, z. B. Vibrio cholerae;
Parvobacteriaceae oder Brucellaceae, wie Pasteurella-Bakterien;
Brucella-Bakterien, z. B. Brucella abortus;
Haemophilus-Bakterien, z. B. Haemophilus influenzae;
Bordetella-Bakterien, z. B. Bordetella pertussis;
Moraxella-Bakterien, z. B. Moraxella lacunata;
Bacteroidaceae, wie Bacteroides-Bakterien;
Fusiforme-Bakterien, z. B. Fusobacterium fusiforme;
Sphaerophorus-Bakterien, z. B. Sphaerophorus necrophorus;

Bacillaceae, wie aerobe Sporenbildner, z. B. Bacillus anthracis;
anaerobe Sporenbildner-Chlostridien, z. B. Chlostridium perfringens;
Spirochaetaceae, wie Borrelia-Bakterien;
Treponema-Bakterien, z. B. Treponema pallidum;
Leptospira-Bakterien, wie Leptospira interrogans.

Die obige Aufzählung von Erregern ist lediglich beispielhaft und keineswegs beschränkend aufzufassen.

Die Wirksamkeit der erfindungsgemäßen Penicilline läßt sich durch folgende Untersuchungen beibeispielhaft demonstrieren:

1. In vitro-Versuche

Für die Untersuchungen wurde die Methode des Reihenverdünnungstestes im Mikrotitersystem angewandt. Die Prüfung der Substanzen auf Bakteriostase erfolgte in flüssigem Medium. Es wurde die Bakteriostasewirkung bei folgenden Konzentrationen untersucht:

80; 40; 20; 10; 5; 2,5; 1,25; 0,6; 0,3; 0,08 und 0,02 μg/ml.

Es wurde ein Nährboden folgender Zusammensetzung benutzt: 10 g Pepton, 8 g Fleischextrakt-Oxoid, 3 g Natriumchlorid, 2 g sek. Natriumphosphat werden mit dest. Wasser auf 1000 ml aufgefüllt (pH 7,2—7,4). Lediglich bei der Testung gegen Streptokokken wurde 1% Glucose hinzugefügt. Das Alter der Primärkulturen betrug etwa 20 Stunden. Die Einstellung der Keimsuspension erfolgte am Photometer (nach »Eppendorf«) (Reagenzglas-Durchmesser 14 mm, Filter 546 nm) anhand der Trübung einer Bariumsulfat-Vergleichssuspension, die durch eine Bariumsulfat-Aufschwemmung erzeugt wurde, welche durch die Zugabe von 3,0 ml 1%ige Bariumchloridlösung in 97 ml 1%ige Schwefelsäure entstand. Nach der Einstellung wurden Streptococcus Aronson im Verhältnis 1 : 15 und die übrigen Testkeime im Verhältnis 1 : 1500 mit einer Kochsalzlösung weiter verdünnt.

16 mg der jeweiligen Substanz wurden in 10 ml-Meßkolben eingewogen und mit dem Lösungsmittel bis zur Marke aufgefüllt. Die weitere Verdünnungsreihe wurde mit destilliertem Wasser oder dem jeweiligen Lösungsmittel hergestellt.

Die Vertiefungen der Mikrotiterplatten wurden mit 0,2 ml Nährmedium, 0,01 ml der entsprechenden Substanzverdünnung und mit einem Tropfen Keimsuspension (0,01 ml) beschickt und 18 bis 20 Stunden bei 37°C bebrütet. Eine Lösungsmittelkontrolle wurde stets mitgeführt.

Die Ablesung wurde makroskopisch vorgenommen, wobei die jeweilige Grenzkonzentration (=niedrigste noch bakteriostatisch wirksame Konzentration) ermittelt wurde.

Als Testorganismen wurden benützt:

Staphylococcus aureus SG 511, Streptococcus Aronson, Streptococcus faecalis ATCC 10 541, Escherichia coli ATCC 9637, 11 775 und Escherichia coli 12 593/74 (β-Lactamase-Träger), Pseudomonas aeruginosa Hamburgensis und ATCC 10 145, Serratia marcescens ATCC 13 880, Klebsiella pneumoniae ATCC 10 031 und 272 und Proteus mirabilis Hamburgensis.

In der folgenden Tabelle 1 sind die ermittelten minimalen Hemmkonzentrationen (MIC) für typische Vertreter der erfindungsgemäßen Verbindungen aufgeführt:

Natriumsalz von Verbindungen der allgemeinen Formel I mit den Bedeutungen von A und R:

| A | R | |
|---|---|---|
| p-Hydroxyphenyl | Wasserstoff | = A |
| p-Hydroxyphenyl | Methyl | = B |
| p-Hydroxyphenyl | Cyclopropyl | = C |
| p-Hydroxyphenyl | p-Chlorbenzyl | = D |
| p-Hydroxyphenyl | Acetylamino | = E |
| p-Hydroxyphenyl | Anilino | = G |
| p-Hydroxyphenyl | p-Chloranilino | = H |
| p-Hydroxyphenyl | m,p-Dichloranilino | = J |
| p-Hydroxyphenyl | Morpholino | = K |
| p-Hydroxyphenyl | Cyclohexylamino | = L |
| p-Hydroxyphenyl | Methylamino | = N |
| p-Hydroxyphenyl | p-Chlorbenzylamino | = O |
| p-Hydroxyphenyl | Propylamino | = P |

Als Vergleichssubstanzen dienten:
Penicilline der allgemeinen Formel XV

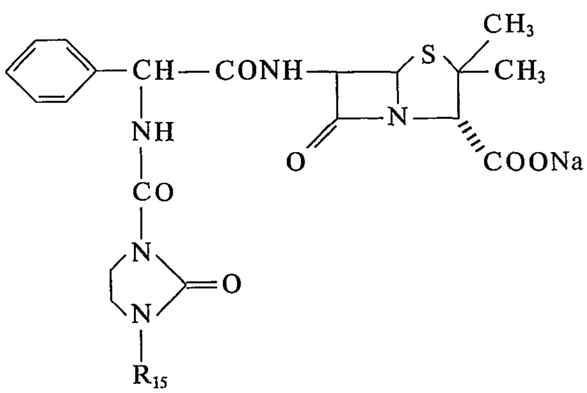

(XV)

mit

XVa $R_{15}$=H: Azlocillin = Q
XVb $R_{15}$=$SO_2CH_3$: Mezlocillin = R
und
D-α-[(4-Hydroxy-3-pyridyl)-ureido]-benzylpenicillin-Natrium = S
(vgl. DE-OS 2 450 668)

Tabelle 1
MHK-Werte verschiedener Penicilline (in µg/ml)

| Verb. | Staph. aureus SG 511 | Strept. Aronson | Strept. faecal. | E. coli ATCC 9637 | E. coli ATCC 11 775 | E. coli 12 593/74 | Pseud. aerug. Hbg. | Pseud. aerug. ATCC 10 145 | Serrat marcesc. | Klebs. pneum. ATCC 10 031 | Klebs. pneum. 272 | Prot. mirabilis |
|---|---|---|---|---|---|---|---|---|---|---|---|---|
| A | 0,3 | 0,08 | 10 | 2,5 | 2,5 | >80 | 5 | 2,5 | 5 | 80 | 80 | 0,6 |
| B | 1,25 | 0,3 | 10 | 2,5 | 1,25 | 80 | 5 | 2,5 | 5 | 80 | 80 | 0,6 |
| C | 0,3 | 0,08 | 10 | 2,5 | 1,25 | 80 | 2,5 | 1,25 | 1,25 | 40 | 80 | 1,25 |
| D | 0,08 | 0,02 | 10 | 2,5 | 1,25 | 80 | 5 | 2,5 | 1,25 | 5 | 10 | 1,25 |
| E | 1,25 | 0,3 | 10 | 5 | 2,5 | >80 | 2,5 | 2,5 | 10 | 80 | 80 | 1,25 |
| G | 0,3 | 0,08 | 5 | 0,6 | 1,25 | >80 | 5 | 2,5 | 0,3 | 10 | 5 | 2,5 |
| H | 1,25 | 0,5 | 5 | 1,25 | 0,6 | 80 | 2,5 | 1,25 | 1,25 | 5 | 2,5 | 0,6 |
| J | 0,3 | 0,02 | 2,5 | 1,25 | 0,6 | 10 | 2,5 | 2,5 | 0,6 | 2,5 | 2,5 | 0,6 |
| K | 1,25 | 0,3 | 10 | 2,5 | 2,5 | >80 | 2,5 | 1,25 | 5 | >80 | 80 | 1,25 |
| L | 0,3 | 0,08 | 5 | 0,6 | 1,25 | >80 | 2,5 | 2,5 | 1,25 | 20 | 10 | 0,6 |
| N | 0,3 | 0,08 | 10 | 2,5 | 2,5 | >80 | 2,5 | 2,5 | 5 | 80 | 40 | 0,3 |
| O | 0,3 | 0,02 | 10 | 0,3 | 0,6 | 80 | 10 | 5 | 1,25 | 20 | 10 | 0,3 |
| P | 0,3 | 0,08 | 5 | 1,25 | 1,25 | 80 | 2,5 | 1,25 | 1,25 | 10 | 10 | 0,08 |
| Vergleichsverbindungen | | | | | | | | | | | | |
| Q | | | | 20 | 10 | | 10 | 5 | | | | |
| R | | | | 10 | 5 | | 40 | 20 | | | | |
| S | | | | 10 | 2,5 | | 20 | 20 | | | | |

Wie aus der vorstehenden Tabelle ersichtlich wird, sind alle getesteten Verbindungen den Vergleichssubstanzen Q, R und S in ihrer Wirksamkeit gegenüber typischen Hospitalkeimen, wie E. coli ATCC 11 775 und E. coli 9637, Pseudomonas aerug. Hbg. und ATCC 10 145 deutlich überlegen.

Es wurde bei zwei typischen Vertretern, nämlich den Penicillinen C und H, der Einfluß der Inokulumgröße auf die MHK-Werte gegen Ps. aerug Hbg. und E. coli ATCC 11 775 im Mikrotitersystem bestimmt. Dabei wurden 3 verschiedene Inokulumgrößen ($3{,}3 \times 10^6$, $3{,}3 \times 10^4$ und $3{,}3 \times 10^2$ bei E. coli, und $4{,}7 \times 10^6$, $4{,}7 \times 10^4$ und $4{,}7 \times 10^2$ bei Pseudomonas) benützt. Bezugssystem war wiederum das Penicillin Q:

Tabelle 2

Einfluß der Inokulumgröße auf die MHK-Werte gegen Pseud. aerug. Hbg.

| | $4{,}7 \times 10^6$ Keime/ml | $4{,}7 \times 10^4$ Keime/ml | $4{,}7 \times 10^2$ Keime/ml |
|---|---|---|---|
| C | >80 | 5 | 2,5 |
| H | >80 | 2,5 | 2,5 |
| Q | >80 | 10 | 5 |

Tabelle 3

Einfluß der Inokulumgröße auf die MHK-Werte gegen E. Coli ATCC 11 775

| | $3{,}3 \times 10^6$ Keime/ml | $3{,}3 \times 10^4$ Keime/ml | $3{,}3 \times 10^2$ Keime/ml |
|---|---|---|---|
| C | 2,5 | 2,5 | 2,5 |
| H | 1,0 | 0,6 | 0,3 |
| Q | 10 | 10 | 10 |

Die akute Toxizität wurde durch perorale und subcutane Applikation der Verbindungen der Tabelle 1 an weiße Laboratoriumsmäuse in steigenden Dosen bestimmt.

Die $LD_{50}$ ist die Dosis, nach deren Applikation 50% der Tiere innerhalb von 8 Tagen sterben. Sämtliche Substanzen zeigten bei oraler Gabe eine $LD_{50}$ von über 4 g/kg, bei subcutaner Gabe eine $LD_{50}$ von über 3 g/kg, d. h. bei 3 g/kg starben keine Tiere, sie sind damit für die Praxis untoxisch.

Zur Bestimmung der Serumspiegel bei Ratten wurden typische Vertreter der erfindungsgemäßen Penicilline in einer Dosierung von 10 mg/kg subcutan oder 100 mg/kg per os an Gruppen zu je 3 weiblichen Ratten des Stammes FW 49 (Gewicht 100—130 g) appliziert. Aus dem Herzen entnommene Blutproben wurden aufgearbeitet und die gewonnenen Sera durch die Zylindertest-Agardiffusionsmethode auf Platten mit Sarcina lutea ATCC 15 957 getestet und mit Standardkurven verglichen. Die erzielten Ergebnisse finden sich in der folgenden Tabelle:

Tabelle 4

Rattenblutspiegel (µg/ml) nach Verabreichung einer Einzeldosis der jeweiligen Substanz (Mittel aus 3 Ratten)
Dosis: oral 100 mg/kg
subcutan 10 mg/kg

subcutane Gabe

| Zeit | Verb. A | B | C | D | E | G | Q |
|---|---|---|---|---|---|---|---|
| 30 min | 9,3 | — | 5,1 | — | 4,1 | — | 2,8 |
| 60 min | 8,6 | 7,3 | 6,8 | 7,1 | 5,8 | 5,6 | 3,7 |
| 120 min | 0,4 | 1,1 | 0,4 | 0,3 | 7,7 | 1,5 | 0,4 |

orale Gabe

| Zeit | A | B | C | D | E | G | Q |
|---|---|---|---|---|---|---|---|
| 30 min | 9,1 | — | 3,3 | — | 13,8 | — | 0,8 |
| 60 min | 9,6 | 18,8 | 4,8 | 4,1 | 13,9 | 0,6 | 0,6 |
| 120 min | 2,9 | 1,9 | 1,0 | 1,0 | 13,7 | 0,1 | 0,3 |

Eine Reihe der erfindungsgemäßen Verbindungen wurde in vivo bei experimentellen Infektionen an Mäusen untersucht. Man verwendete als pathogene Bakterien E. coli ATCC 11 775 und Pseudomonas aeruginosa Walter. Es wurde eine intraperitoneale Infektion mit 0,2 ml einer 5%igen Mucinsuspension der Bakterien gesetzt. Dies entspricht etwa $2 \times 10^6$ Keime E. Coli bzw. $8 \times 10^5$ Keime Pseudomonas/Maus. Weibliche Mäuse vom Stamm NMRI wurden in Gruppen von jeweils 10 Tieren aufgeteilt, zwei Gruppen blieben unbehandelt, die restlichen Gruppen wurden mit verschiedenen Dosen der jeweiligen erfindungsgemäßen Penicilline zur Bestimmung der $ED_{50}$ (Dosis bei der 50% der Tiere überleben) behandelt. Bei der E. coli-Infektion wurde am ersten Tag 3× therapiert (1,4 und 7 h post-Infektionem) und 2 Tage 2× täglich. Bei der Pseudomonas-Infektion wurde am ersten Tag 6× (1, 3, 6, 9, 12 und 15 h post-Infektionem) und 2 Tage 2× täglich behandelt. Der Beobachtungszeitraum betrug in beiden Fällen 7 Tage. Die Ergebnisse dieser Teste mit repräsentativen Vertretern der erfindungsgemäßen Penicilline sind in der Tabelle 5 dargestellt.

Tabelle 5

In vivo Aktivität bei Mäusen

a) E. coli-Infektion:

| Verbindung | $ED_{50}$ (mg/kg) |
|---|---|
| A | 6 (s. c.)<br>50 (p. o.) |
| B | 5 (s. c.) |
| C | 1,5 (s. c.)<br>30 (p. o.) |
| D | 9 (s. c.) |
| E | 15 (s. c.) |
| G | 8 (s. c.) |
| Q | 35 (s. c.)<br>320 (p. o.) |

b) Pseudomonas:

| Verbindung | $ED_{50}$ (mg/kg) |
|---|---|
| A | 50 (s. c.) |
| C | 7 (s. c.) |
| Q | 110 (s. c.) |

Die angeführten Werte zeigen, daß repräsentative Vertreter der erfindungsgemäßen Penicilline auf Grund ihres breiten antibiotischen Spektrums, ihrer hohen antibakteriellen Aktivität, ihrer niederen Toxizität und ihres hohen Serumgehalts nach subcutaner und oraler Gabe wertvolle Antibiotika sind.

Es ist eine weitere Aufgabe der vorliegenden Erfindung, pharmazeutische Mittel zu schaffen, die bei der Behandlung infektiöser Krankheiten sowohl beim Menschen als auch beim Tier wertvoll sind.

Als bevorzugte pharmazeutische Zubereitungen seien Tabletten, Dragées, Kapseln, Granulate, Suppositorien, Lösungen, Suspensionen, Emulsionen, Salben, Gele, Cremes, Puder und Sprays genannt. Vorteilhafterweise wird in der Human- oder Tiermedizin der Wirkstoff oder ein Gemisch der verschiedenen Wirkstoffe der allgemeinen Formel I in einer Dosierung zwischen 5 und 500, vorzugsweise 10–200 mg/kg Körpergewicht je 24 Stunden verabreicht, gegebenenfalls in Form mehrerer Einzelgaben. Eine Einzelgabe enthält den oder die erfindungsgemäßen Wirkstoffe, vorzugsweise in Mengen von etwa 1 bis etwa 100, insbesondere 5 bis 60 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Bei der parenteralen Verabreichung wird es bevorzugt, die erfindungsgemäße Penicillinverbindung in einem nicht-toxischen flüssigen Medium für Injektionszwecke aufzulösen und die Lösung intramuskulär, intravenös oder subkutan zu injizieren.

Es ist auch möglich, die erfindungsgemäße Penicillinverbindung in einem nicht-toxischen Medium oder in einer Salbengrundlage aufzulösen oder damit zu vermischen, und die Lösung oder das Gemisch direkt auf den geschädigten Ort aufzubringen. Die Verbindungen können auch als Suppositorien nach Vermischen mit einer Grundlage für Suppositorien oder nach dem Auflösen darin verwendet werden.

Beispiele für nicht-toxische flüssige Medien, die zur Herstellung von injizierbaren Zubereitungen, welche die Penicillinverbindung als Wirkstoff enthalten, verwendet werden können, sind sterilisiertes entionisiertes Wasser, physiologische Kochsalzlösung, Glucoselösung zur Injektion, Ringersche Lösung und Aminosäurelösung zur Injektion.

Die Penicillinverbindung kann auch in anderen injizierbaren Zubereitungen aufgelöst und parenteral verabreicht werden.

Für die orale Verabreichung werden die erfindungsgemäßen Penicillinverbindungen in Form eines pharmazeutischen Präparats verwendet, welches die Verbindung(en), gegebenenfalls in Mischung mit pharmazeutisch verträglichen Trägern, wie z. B. einem organischen oder anorganischen festen oder flüssigen Hilfsstoff, der sich für die orale Verabreichung eignet, enthält. Gewünschtenfalls können diese Präparate auch Hilfssubstanzen, Stabilisatoren und andere üblicherweise verwendete Zusätze enthalten.

Im Falle der Anwendung als Futterzusatzmittel können die neuen Verbindungen in den üblichen Konzentrationen und Zubereitungen zusammen mit dem Futter bzw. mit Futterzubereitungen oder mit dem Trinkwasser gegeben werden. Dadurch kann eine Infektion durch gramnegative oder grampositive Bakterien verhindert, gebessert und/oder geheilt werden und ebenso eine Förderung des Wachstums und eine Verbesserung der Verwertung des Futters erreicht werden.

Die erfindungsgemäßen Penicilline können zum Zweck der Erweiterung des Wirkungsspektrums und um eine Wirkungssteigerung speziell bei $\beta$-lactamasebildenden Bakterien zu erzielen, mit anderen antimikrobiellen Wirkstoffen, z. B. mit penicillinasefesten Penicillinen kombiniert werden. Hierfür eignen sich besonders Oxacillin oder Dicloxacillin. Ferner können die erfindungsgemäßen Penicilline mit $\beta$-Lactamaseinhibitoren wie z. B. Clavulansäure kombiniert werden.

Die erfindungsgemäßen Penicilline können zum Zweck der Erweiterung des Wirkungsspektrums und um eine Wirkungssteigerung zu erreichen auch mit Aminoglykosidantibiotica, wie z. B. Gentamicin, Sisomicin, Kanamicin, Amikacin oder Tobramicin kombiniert werden.

Die Erfindung betrifft somit auch ein Arzneimittel, das mindestens ein erfindungsgemäßes Penicillinderivat der allgemeinen Formel I oder ein pharmazeutisch verträgliches Salz davon und ein Aminoglykosidantibiotikum oder ein pharmazeutisch verträgliches Säureadditionssalz davon, gegebenenfalls in Mischung mit einem pharmazeutisch verträglichen Träger oder Verdünnungsmittel, enthält.

Die folgenden Beispiele sollen die Erfindung näher erläutern: (Die Angabe der Rf-Werte bezieht sich auf das System n-Butanol-Wasser-Eisessig = 60 : 25 : 15, $SiO_2$-Platte).

Mit »Ampicillin« ist dasjenige $\alpha$-Aminobenzylpenicillin, mit »Amoxycillin« dasjenige $\alpha$-Amino-p-hydroxy-benzylpenicillin und mit »Epicillin« dasjenige $\alpha$-Amino-$\alpha$-(1,4-cyclohexadien-1-yl)-methylpenicillin mit der D=R-Konfiguration in der Seitenkette gemeint.

## I. Darstellung der Ausgangsprodukte

### Beispiel A

a) 5-Amino-2-cyclopropyl-4-hydroxy-pyrimidin

120 g 2-Phenyl-4-äthoxymethylen-5-oxo-2-oxazolin (0,55 Mol), 71 g Cyclopropylformamidinhydrochlorid (0,59 Mol) und 98 g Natriumacetat werden in 3 l absolutem Äthanol 3 h am Rückfluß erhitzt. Anschließend wird im Vakuum auf 1,2 l eingeengt und in 5 l Eiswasser gegossen. Das ausgefallene Produkt wird abgesaugt und aus Eisessig umkristallisiert.

Fp. 258°C
Ausbeute: 118 g (84%)

b) 90 g (0,35 Mol) des so hergestellten Produktes werden mit 140 g Natronlauge und 560 ml Wasser 2 h zum Rückfluß erhitzt. Es wird abgekühlt und mit konz. Salzsäure unter Kühlen auf pH 1,5 angesäuert. Die ausgefallene Benzoesäure wird in Äther aufgenommen. Die wäßrige Phase wird auf pH 6,5 gebracht und zur Trockne eingeengt. Das gewünschte Produkt wird mit Tetrahydrofuran extrahiert.

Ausbeute: 44 g (83%)
Fp. 206°C

Nach dieser Methode wurden folgende Pyrimidine synthetisiert:

| R | Ausbeute | Schmelzpunkt |
|---|---|---|
| 2-Methyl-cyclopropyl | 64% | 192–195°C |
| 1-Methyl-cyclopropyl | 56% | 186°C |

Ersetzt man das Amidin durch das entsprechende Guanidin und spaltet die Benzoylgruppe durch Erhitzen zum Rückfluß mit 5 n Salzsäure ab, so erhält man folgendes Pyrimidin:

| | | |
|---|---|---|
| Pyrrolidino | 64% | Zers. ab 230°C |

Beispiel B

1) 4-Hydroxy-2-methylmercapto-5-nitro-pyrimidin

104,5 g S-Methylisothioharnstoff (0,757 Mol) und 53,3 g (1,333 Mol) Natronlauge werden 10 Min. in 350 ml Wasser zusammen gerührt. Dann gibt man langsam 114,5 g (0,605 Mol) Äthoxymethylen-nitroessigsäure-äthylester zu, wobei die Temperatur durch Kühlen auf 20°C gehalten wird. Der ausgefallene Niederschlag wird abgesaugt und mit wenig eiskaltem Wasser gewaschen. Man löst in etwa 9 l heißem Wasser und säuert mit konzentrierter Salzsäure unter Kühlen an. Das ausgefallene Produkt wird abgesaugt und mit Äther gewaschen.
Ausbeute: 65 g (58%)
F.: 220–222°C

2) 2-Anilino-4-hydroxy-5-nitro-pyrimidin

4,65 g 4-Hydroxy-2-methylmercapto-5-nitro-pyrimidin (0,025 Mol) werden unter Erhitzen in 150 ml Äthanol gelöst und mit 4,68 g Anilin (0,05 Mol) 5 Stunden lang zum Rückfluß erhitzt. Das ausgefallene Produkt wird in der Kälte abgesaugt, mit Äthanol gewaschen und getrocknet.
Ausbeute: 4,1 g (70%)
F.: >300°C

| | | | |
|---|---|---|---|
| Ber.: | C 51,73, | H 3,47, | N 24,13, |
| Gef.: | C 51,70, | H 3,39, | N 23,95. |

3) 5-Amino-2-anilino-4-hydroxy-pyrimidin

2,3 g der Nitroverbindung (0,01 Mol) des Beispiels 2 werden in 100 ml Dimethylformamid mit 500 mg Raney-Nickel bis zum Ende der Wasserstoffaufnahme hydriert. Es wird vom Katalysator abgesaugt, das Lösungsmittel im Vakuum abgedampft und etwas Äthanol zugegeben. Das sich abscheidende kristalline Produkt wird abgesaugt und getrocknet.
Ausbeute: 1,1 g (55%)
F.: 240–242°C

| | | | |
|---|---|---|---|
| Ber.: | C 59,40, | H 4,98, | N 27,71, |
| Gef.: | C 58,70, | H 5,08, | N 27,10. |

Analog wurden folgende Pyrimidine der allgemeinen Formel synthetisiert:

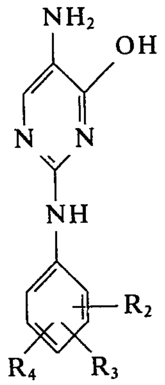

| $R_2$, $R_3$, $R_4$ | Schmelzpunkt | Ausbeute |
|---|---|---|
| p-Chlor | 240–242°C | 44% |
| p-Methoxy | 220–222°C | 61% |
| p-Hydroxy | 290–293°C | 37% |
| m,p-Dichlor | 280°C | 47% |
| p-Methyl | 212–215°C | 34% |
| p-Acetylamino | 255–256°C | 51% |
| o-Chlor | 219–220°C | 42% |
| p-Fluor | 238–240°C | 54% |
| m-Chlor | 241–242°C | 46% |
| o,p-Dichlor | 264–265°C | 40% |
| p-Dimethylamino | 264–265°C | 48% |
| p-Nitro | >300°C | 34% |
| m-Trifluormethyl-p-chlor | 283–285°C | 61% |
| o-Methyl | 220–222°C | 31% |
| p-Brom | 235–236°C | 44% |
| m,m'-Dichlor-p-amino | 275°C (Zers.) | 38% |

Durch Umsetzung von 4-Hydroxy-2-methylmercapto-5-nitropyrimidin mit aliphatischen oder araliphatischen Aminen und anschließende Reduktion der Nitrogruppe erhält man folgende Pyrimidine:

| R | Ausbeute | Schmelzpunkt |
|---|---|---|
| $CH_2=CH-CH_2NH$ | 43% | sintert ab 80°C |
| $CH_3-CH=CH-CH_2NH$ | 39% | Zers. 114°C |
| $HC\equiv C-CH_2NH$ | 48,5% | 186–187°C |
| $(CH_2=CH-CH_2)_2N$ | 53% | 139–140°C |
| Cyclopropylamino | 58% | 246°C |
| Cyclobutylamino | 41% | 261°C |
| Cyclopentylamino | 52% | 270°C |
| Cyclohexylamino | 41% | sintert ab 170°C |
| Dimethylamino | 58% | 225°C |
| Morpholino | 59% | 175–185°C |
| Methylamino | 52% | 196–198°C |
| Benzylamino | 40% | sintert ab 140°C |
| p-Chlorbenzylamino | 39% | sintert ab 90°C |

Beispiel C

5-Benzoylamino-4-hydroxy-2-methylmercapto-pyrimidin

120 g 2-Phenyl-4-äthoxymethylen-5-oxo-2-oxazolin (0,55 Mol) werden zusammen mit 115 g S-Methylisothioharnstoff-sulfat und 99 g Natriumacetat in 2 l absolutem Äthanol 6 h zum Rückfluß erhitzt. Äthanol wird im Vakuum abgezogen und der Rückstand in 3 l Eiswasser eingerührt. Es wird abgesaugt und aus 1,2 l Eisessig umkristallisiert.

Ausbeute: 87 g (60%)
Schmelzpunkt: 270°C

5-Amino-2-cyclohexylmethylamino-4-hydroxy-pyrimidin

Ein Gemisch aus 10,2 g Cyclohexylmethylamin (0,09 Mol) und 5,4 g Eisessig (0,09 Mol) wird mit 7,8 g der oberen Verbindung eine Stunde lang bei 180°C geschmolzen. Der Rückstand wird mit 50%igem Äthanol verrieben und abgesaugt.

Ausbeute: 8,6 g (88%)

Das entstandene Produkt wird in 100 ml konz. Salzsäure 1 h lang am Rückfluß erhitzt, ausgefallene Benzoesäure mit Äther entfernt und mit konz. Natronlauge unter Kühlen auf pH 6,5 gestellt. Das entstandene Produkt wird abgesaugt.

Ausbeute: 4,8 g (84%)
Schmelzpunkt: Zers. >90°C.

Im Falle von R=subst. Anilino wird die Abspaltung der Benzoylgruppe günstiger mit einem Gemisch aus konz. Schwefelsäure/Eisessig durchgeführt.
Nach dieser Methode wurden folgende Pyrimidine hergestellt:

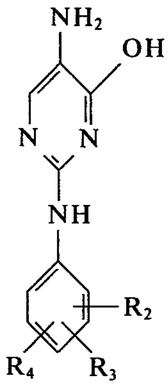

| R2, R3, R4 | Ausbeute | Schmelzpunkt |
|---|---|---|
| p-$CF_3$ | 51% | 214°C |
| p-$C_2H_5$ | 61% | 224–225°C |
| p-$CH(CH_3)_2$ | 54% | 200–202°C |
| p-$CH_3NH$ | 46% | 244–245°C |
| p-$CH_3SO_2$ | 54% | 288–290°C |
| p-$CH_3CO$ | 67% | >300°C |
| p-$H_2NCO$ | 61% | 279–280°C |
| p-CN | 44% | 246–250°C |
| m,p-$CH_3O$ | 54% | 234–235°C |
| m,m-Cl | 42% | 280–282°C |
| p-$CH_3SO$ | 51% | 259–260°C |

bzw.

| R | 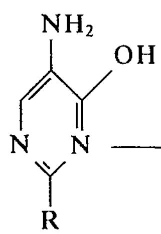 | |
|---|---|---|
| Äthylamino | 41% | Zers. >150°C |
| Propylamino | 56% | 141–144°C |
| Isopropylamino | 52% | 138–140°C |
| Butylamino | 34% | 146–148°C |
| Isobutylamino | 48% | 162–163°C |
| Hexylamino | 58% | sintert über 115°C |
| Cyclopropylmethylamino | 47% | 164–167°C |

Beispiel D

5-Amino-4-hydroxy-2-propionylamino-pyrimidin

7,8 g (0,055 Mol) 2-Amino-4-hydroxy-5-nitro-pyrimidin werden in 50 ml Propionsäureanhydrid 4 h auf 140°C erhitzt. Es wird abgekühlt und das ausgefallene Produkt abgesaugt und mit Äther gut ausgewaschen. Die Verbindung wird in 200 ml Dimethylformamid suspendiert und bei Raumtemperatur und Normaldruck mit 1 g Pd/C als Katalysator hydriert.

Ausbeute: 6,8 g (71%) Zersetzung >240°C.

Nach dieser Methode wurden folgende Pyrimidine hergestellt:

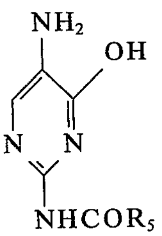

| $R_5$ | Ausbeute | Schmelzpunkt |
|---|---|---|
| Propyl | 69% | 202°C |
| Isopropyl | 74% | 214°C |

Beispiel E

D-α-[3-(2-p-Chloranilino-4-hydroxy-5-pyrimidyl)-ureido]-phenylglycin

2,36 g (0,01 Mol) 5-Amino-2-p-chloranilino-4-hydroxypyrimidin werden in der Wärme in 200 ml Tetrahydrofuran gelöst und mit 1,35 ml Triäthylamin versetzt. Die Lösung wird zu einer eisgekühlten Lösung von 1,05 g Phosgen getropft. Anschließend wird im Vakuum auf 50 ml eingeengt. Diese Lösung wird bei 0 bis 5°C zu einer Lösung von 1,73 g des Natriumsalzes des D-α-Phenylglycins in 50 ml 80%igem wäßrigem Tetrahydrofuran getropft. Während der Zugabe wird der pH-Wert der Lösung durch Zugabe einer 2n Natronlauge auf 9,5 bis 10 gehalten. Die Lösung wird 1 Stunde bei 5°C und 2 Stunden bei Raumtemperatur gehalten. Nach der Umsetzung wird das Tetrahydrofuran unter vermindertem Druck entfernt und die zurückbleibende wäßrige Phase zweimal mit Essigsäureäthylester bei pH 7,5 ausgeschüttelt. Dann wird unter Eiskühlung auf pH 1,5 gestellt (verdünnte Salzsäure). Die wäßrige Phase wird zweimal mit je 100 ml Essigsäureäthylester ausgeschüttelt. Die organischen Phasen werden mit Wasser gewaschen und über wasserfreiem Natriumsulfat getrocknet. Zu der organischen Phase gibt man dann die berechnete Menge des Natriumsalzes der 2-Äthylhexansäure, wobei wäßrige Kristalle abgeschieden werden, die abgesaugt und getrocknet werden.

Ausbeute: 2,57 g (56%).

Analog können hergestellt werden:

D-α-[3-(2-m,p-Dichloranilino-4-hydroxy-5-pyrimidyl)-ureido]-phenylglycin-Natrium
D-α-[3-(4-Hydroxy-2-o-methylanilino-5-pyrimidyl)-ureido]-phenylglycin
D-α-[3-(2-(p-Chlor-m-trifluormethylanilino)-4-hydroxy-5-pyrimidyl)-ureido]-phenylglycin
D-α-[3-(2-p-Bromanilino-4-hydroxy-5-pyrimidyl)-ureido]-phenylglycin.

II. Darstellung der Endprodukte

Beispiel 1

D-α-[3-(4-Hydroxy-5-pyrimidyl)-ureido]-p-hydroxybenzylpenicillin-Natrium

1,1 g (0,01 Mol) 4-Hydroxy-5-amino-pyrimidin werden unter Erhitzen in 800 ml absolutem Tetrahydrofuran gelöst. Die Lösung wird mit 1,35 ml Triäthylamin (0,01 Mol) versetzt. Das Gemisch tropft man bei 0°C zu einer Lösung von 1,05 g (0,01 Mol) Phosgen in 50 ml absolutem Tetrahydrofuran. Das entstehende Gemisch wird anschließend im Vakuum auf etwa 100 ml eingeengt.

4,2 g (0,01 Mol) Amoxycillin-trihydrat werden in 60 ml 80%igem wäßrigem Tetrahydrofuran aufgeschlämmt und bei 0°C langsam mit Triäthylamin versetzt, bis alles gelöst ist. Dann wird unter

Eiskühlung die oben hergestellte Lösung zugetropft, wobei der pH-Wert durch Zugabe von Triäthylamin auf 7,5 gehalten wird. Nach der Zugabe wird eine Stunde lang bei 5°C und eine Stunde bei Raumtemperatur nachgerührt und anschließend das Tetrahydrofuran im Vakuum abgezogen. Es wird mit Wasser auf 50 ml verdünnt und zweimal bei pH 7,0 mit je 50 ml Essigsäureäthylester ausgeschüttelt. Die wäßrige Phase wird dann mit 300 ml Essigsäureäthylester überschichtet und unter Eiskühlung und Rühren mit 2 n Salzsäure auf pH 2,0 gestellt. Die organische Phase wird abgetrennt, die wäßrige Phase noch einmal mit 50 ml Essigsäureäthylester ausgeschüttelt, die organischen Phasen vereinigt, mit Natriumsulfat getrocknet und im Vakuum zur Trockne eingeengt.

Das zurückbleibende Produkt wird mit einer Lösung von 1,28 g (0,007 Mol) Natriumhexanoat in 25 ml trockenem Methanol versetzt, wobei Lösung eintritt. Unter Rühren fügt man 300 ml trockenen Diäthyläther zu. Das ausgefallene Festprodukt wird abgesaugt und im Vakuum getrocknet.

Ausbeute: 3,26 g (62%);
Rf: 0,54;
IR-Spektrum: 1770, 1650, 1600, 1490 $cm^{-1}$;
NMR-Spektrum (DMSO+$CD_3OD$) Signale bei ppm: 1,55 (d, 6H), 4,05 (1H), 5,40 (q, 2H), 5,5 (1H), 6,8 (d, 2H), 7,35 (d, 2H), 7,9 (1H), 8,6 (1H).

Beispiel 2

D-α-[3-(4-Hydroxy-5-pyrimidyl)-ureido]-benzylpenicillin-Natrium

1,1 g (0,01 Mol) 4-Hydroxy-5-amino-pyrimidin werden mit 20 ml Hexamethyldisilazan und einigen Körnchen Ammonsulfat 3 Stunden zum Rückfluß erhitzt. Anschließend wird überschüssiges Hexamethyldisilazan mit Stickstoff abgeblasen. Der Rückstand wird in 30 ml absolutem Tetrahydrofuran gelöst, mit 1,38 ml Triäthylamin versetzt und unter Eiskühlung zu einer Lösung von 1 g Phosgen in 50 ml absolutem Tetrahydrofuran getropft. Dann wird etwa 1 Stunde bei 0°C gerührt. Triäthylaminhydrochlorid wird unter Stickstoff abfiltriert und die Lösung im Vakuum zur Trockne eingeengt.

4 g Ampicillintrihydrat (0,01 Mol) werden mit 2,5 ml Triäthylamin in 80 ml Methylenchlorid gelöst. Die Lösung wird über Magnesiumsulfat getrocknet, filtriert und auf 0°C abgekühlt. Dann wird eine Lösung des oben erhaltenen Festprodukts in 80 ml absolutem Methylenchlorid zugetropft. Es wird 1 Stunde bei 0°C und 2 Stunden bei Raumtemperatur nachgerührt. Dann wird im Vakuum zur Trockne eingeengt. Das erhaltene Festprodukt wird mit 50 ml Essigsäureäthylester und 50 ml Wasser versetzt und der pH-Wert auf 7,5 eingestellt. Die wäßrige Phase wird noch 2mal mit wenig Essigsäureäthylester ausgeschüttelt. Dann wird die wäßrige Phase mit 500 ml Essigester überschichtet und der pH-Wert unter Eiskühlung mit verdünnter Salzsäure langsam auf 2,0 gestellt. Es wird von etwas schwerlöslichem Produkt abfiltriert und die wäßrige Phase noch einmal mit 100 ml Essigsäureäthylester ausgeschüttelt. Nach Trocknung und Abdampfen des Lösungsmittels wird das Natriumsalz, wie in Beispiel 1 beschrieben, hergestellt.

Ausbeute: 2,45 g (48%);
Rf: 0,57;
IR-Spektrum: 1770, 1660, 1610, 1525 $cm^{-1}$;
NMR ($D_2O$) Signale bei ppm: 1,4 (3H), 1,5 (3H), 4,25 (1H), 5,3—5,6 (3H), 7,5 (5H), 8,3 (1H), 8,6 (1H).

Beispiel 3

D-α-[3-(4-Hydroxy-5-pyrimidyl)-ureido]-cyclohexa-1,4-dien-1-yl-methylpenicillin-Natrium

Dieses Penicillin wurde analog Beispiel 1 synthetisiert, indem man von dem Umsetzungsprodukt von 550 mg (0,005 Mol) 5-Amino-4-hydroxy-pyrimidin mit 0,68 ml (0,005 Mol) Triäthylamin und 0,50 g Phosgen (0,005 Mol) sowie 1,87 g (0,005 Mol) Epicillin-Natrium ausging.

Ausbeute: 3,12 g Natriumsalz (61%);
IR-Spektrum: 1770, 1655, 1605, 1535 $cm^{-1}$;
NMR-Spektrum (DMSO+$CD_3OD$) Signale bei ppm: 1,50 (6H), 2,50 (4H), 4,05 (1H), 4,90 (1H), 5,30 (2H), 5,65 (3H), 7,9 (1H), 8,55 (1H).

Beispiel 4

D-α-[3-(4-Hydroxy-2-methyl-5-pyrimidyl)-ureido]-p-hydroxybenzylpenicillin-Natrium

Analog Beispiel 1, ausgehend von 2,5 g 5-Amino-4-hydroxy-2-methyl-pyrimidin (0,02 Mol), das zunächst mit 2,1 g Phosgen und 2,7 ml Triäthylamin und dann mit 8,5 g Amoxycillintrihydrat (0,002 Mol) umgesetzt wurde.

Ausbeute: 6,45 g Natriumsalz (60%);
Rf: 0,55;
IR-Spektrum: 1770, 1660, 1610, 1545 $cm^{-1}$;
NMR-Spektrum ($D_2O$) Signale bei ppm: 1,45 (6H), 2,4 (3H), 4,2 (1H), 5,3 (1H), 5,45 (2H), 6,9 (2H), 7,35 (2H), 8,3 (1H).

Beispiel 5

D-α-[3-(4-Hydroxy-2-methyl-5-pyrimidyl)-ureido]-benzylpenicillin-Natrium

Analog Beispiel 1, ausgehend von 750 mg des Pyrimidins des Beispiels 4 (0,006 Mol), 0,8 ml Triäthylamin, 600 mg Phosgen sowie 2,25 g (0,006 Mol) Ampicillin-Natriumsalz.
Ausbeute: 1,76 g Natriumsalz (56%);
IR-Spektrum: 1770, 1660, 1610, 1530 $cm^{-1}$;
NMR-Spektrum ($D_2O$) Signale bei ppm: 1,50 (6H), 2,45 (3H), 4,15 (1H), 5,3–5,6 (3H), 7,5 (5H), 8,3 (1H).

Beispiel 6

D-α-[3-(2-Cyclopropyl-4-hydroxy-5-pyrimidyl)-ureido]-p-hydroxybenzylpenicillin-Natrium

18,2 g 5-Amino-2-cyclopropyl-4-hydroxy-pyrimidin (0,12 Mol) werden unter Erwärmen in 800 ml absolutem Tetrahydrofuran gelöst und mit 16,5 ml Triäthylamin versetzt. Diese Lösung wird bei 0°C zu einer Lösung von 12 g Phosgen in 250 ml absolutem Tetrahydrofuran getropft. Man rührt unter Eiskühlung etwa 30 Minuten nach. Anschließend wird Stickstoff durch die Lösung geblasen, um nicht umgesetztes Phosgen zu entfernen.

Man suspendiert 50,4 g Amoxicillin-trihydrat in 1,6 l wäßrigem 80%igem Tetrahydrofuran und kühlt auf 0°C ab. Dann wird soviel Triäthylamin zugegeben, daß eine Lösung entsteht (16,5 ml) innerhalb von 60 Minuten wird die oben hergestellte Suspension zugetropft, wobei der pH-Wert mit Triäthylamin auf 7,5 gehalten wird. Man setzt weitere 100 ml Wasser und hält das Reaktionsgemisch eine Stunde bei 0–2°C. Die Kühlung wird entfernt und es wird eine Stunde bei Raumtemperatur nachgerührt.

Man setzt dann 500 ml Wasser zu und entfernt das Tetrahydrofuran im Vakuum. Die zurückbleibende Wasserphase wird zweimal mit 280 ml Essigsäureäthylester gewaschen. Man verdünnt mit Eiswasser auf 2,5 l und überschichtet mit 6 l Essigsäureäthylester. Unter stetem Rühren wird langsam 2 n Salzsäure zugetropft bis pH 2,0, wobei die Temperatur unter 5°C gehalten wird. Man filtriert von etwas unlöslichem Produkt ab und trennt die Schichten. Die Wasserphase wird nochmals mit 1 l Essigsäureäthylester ausgewaschen. Die organische Phase wird über Natriumsulfat getrocknet und das Lösemittel im Vakuum entfernt. Das Produkt wird in wenig Wasser aufgeschlämmt und unter Eiskühlung und Rühren tropfenweise mit n/10 Natronlauge bis zu einem pH-Wert von 6,8 versetzt. Die entstandene Lösung wird gefriergetrocknet.
Ausbeute: 60,2 g Natriumsalz (89%);
Rf.: 0,71;
IR-Spektrum: 1775, 1660, 1615, 1555 $cm^{-1}$;
NMR-Spektrum (DMSO+$CD_3OD$) Signale bei ppm: 0,95 (4H), 1,55 (6H), 1,9 (1H), 4,05 (1H), 5,35 (2H), 6,8 (2H), 7,3 (2H), 8,48 (1H).

Beispiel 7

D-α-[3-(2-Cyclopropyl-4-hydroxy-5-pyrimidyl)-ureido]-benzylpenicillin-Natrium

Dieses Penicillin wurde analog Beispiel 6 hergestellt. Man geht aus von 4,0 g Ampicillin-trihydrat, sowie dem Umsetzungsprodukt von 1,51 g (0,01 Mol) des Pyrimidins des Beispiels 6 mit 1,0 g Phosgen und 1,35 ml Triäthylamin.
Ausbeute: 4,2 g Natriumsalz (78%);
Rf.: 0,74;
IR-Spektrum: 1770, 1655, 1615, 1540 $cm^{-1}$;
NMR-Spektrum (DMSO+$CD_3OD$) Signale bei ppm: 0,95 (4H), 1,55 (6H), 1,9 (1H), 4,0 (1H), 5,45 (3H), 7,45 (5H), 8,40 (1H).

## Beispiel 8

### D-α-[3-(2-Cyclopropyl-4-hydroxy-5-pyrimidyl)-ureido]-cyclohexa-1,4-dien-1-yl-methylpenicillin-Natrium

Dieses Penicillin wurde analog Beispiel 6 hergestellt. Man geht aus von 1,87 g Epicillin-Natrium (0,005 Mol) sowie dem Umsetzungsprodukt von 0,75 g (0,005 Mol) des Pyrimidins des Beispiels 6 mit 500 mg Phosgen und 0,68 ml Triäthylamin.

Ausbeute: 2,03 g Natriumsalz (74%);
IR-Spektrum: 1770, 1660, 1605, 1550 $cm^{-1}$;
NMR-Spektrum (DMSO+$CD_3OD$) Signale bei ppm: 0,9 (4H), 1,5 (6H), 1,95 (1H), 2,50 (4H), 4,05 (1H), 4,95 (1H), 5,30 (2H), 5,65 (3H), 8,40 (1H).

Analog wurde synthetisiert:
D-α-[3-(2-Cyclopropyl-4-hydroxy-5-pyrimidinyl)-ureido]-m,p-dihydroxybenzylpenicillin-Natrium;
Ausbeute: 56,5%;
IR-Spektrum: 1765, 1660, 1610, 1550 $cm^{-1}$;
NMR-Spektrum (DMSO+$CD_3OD$) Signale bei ppm: 0,95 (m, 4H), 1,55 (d, 6H), 1,95 (m, 1H), 4,05 (s, 1H), 5,50 (m, 3H), 6,8 (d, 2H), 7,1 (m, 1H), 8,45 (s, 1H).

## Beispiel 9

### D-α-[3-(2-Cyclobutyl-4-hydroxy-5-pyrimidyl)-ureido]-p-hydroxy-benzylpenicillin-Natrium

Dieses Penicillin wurde analog Beispiel 6 hergestellt. Man geht aus von 4,2 g Amoxycillin-trihydrat (0,01 Mol) sowie dem Umsetzungsprodukt von 1,65 g (0,01 Mol) 5-Amino-2-cyclobutyl-4-hydroxy-pyrimidin mit 1,0 g Phosgen und 1,38 ml Triäthylamin.

Ausbeute: 4,85 g Natriumsalz (84%);
Rf.: 0,73;
IR-Spektrum: 1770, 1660, 1605, 1545 $cm^{-1}$;
NMR-Spektrum (DMSO+$CD_3OD$) Signale bei ppm: 1,55 (6H), 1,8–2,4 (m, 6H), 3,4 (1H), 5,4 (q, 2H), 5,5 (1H), 6,8 (2H), 7,3 (2H), 8,6 (1H).

## Beispiel 10

### D-α-[3-(4-Hydroxy-2-{1′-methyl-cyclopropyl}-5-pyrimidyl)-ureido]-p-hydroxy-benzylpenicillin-Natrium

Dieses Penicillin wurde analog Beispiel 6 hergestellt. Man geht aus von 2,1 g Amoxycillin-trihydrat (0,005 Mol) sowie dem Umsetzungsprodukt von 0,83 g (0,005 Mol) 5-Amino-4-hydroxy-2-(1′-methyl-cyclopropyl)-pyrimidin mit 500 mg Phosgen und 0,67 ml Triäthylamin.

Ausbeute: 1,54 g Natriumsalz (53%);
Rf.: 0,70;
IR-Spektrum: 1765, 1655, 1610, 1550 $cm^{-1}$;
NMR-Spektrum (DMSO+$CD_3OD$) Signale bei ppm: 0,9–1,4 (7H), 1,55 (6H), 4,0 (1H), 5,35 (2H), 5,45 (1H), 6,8 (2H), 7,3 (2H), 8,50 (1H).

## Beispiel 11

### D-α-[3-(2,4-Dihydroxy-5-pyrimidyl-)ureido]-benzylpenicillin-Natrium

Wie im Beispiel 2 werden 1,3 g (0,01 Mol) 5-Amino-2,4-dihydroxypyrimidin mit Hexamethyldisilazan und Phosgen behandelt. Das entstehende Produkt wird in 25 ml absolutem Methylenchlorid gelöst und bei 0°C zu einer Lösung von 4,3 g Ampicillin-Triäthylammoniumsalz in 50 ml absolutem Methylenchlorid getropft. Man läßt 2 Stunden bei Raumtemperatur nachrühren. Dann wird im Vakuum eingeengt und das erhaltene Festprodukt in einem Gemisch aus 50 ml Essigsäureäthylester und 50 ml Wasser gelöst, wobei der pH-Wert mit Triäthylamin auf 7,5 gestellt wird. Die wäßrige Phase wird abgetrennt, mit 50 ml Essigsäureäthylester überschichtet und mit verdünnter Salzsäure auf einen pH-Wert von 2,0 gebracht.

Aus der getrockneten Essigsäureäthylesterlösung wird mit Kaliumhexanoat das Kaliumsalz gefällt.

Ausbeute: 1,5 g (29% der Theorie);
IR-Spektrum: 1770, 1660, 1625, 1555 $cm^{-1}$;
NMR-Spektrum ($CD_3OD$) Signale bei ppm: 1,4–1,5 (d, 6H), 4,2 (1H), 5,4–5,6 (3H), 7,5 (5H), 8,4 (1H).

Beispiel 12

D-α-[3-(2,4-Dihydroxy-5-pyrimidyl)-ureido]-p-hydroxybenzylpenicillin-Natrium

Dieses Penicillin wurde analog Beispiel 11 hergestellt. Man geht aus von 4,7 g Amoxycillin-triäthylammoniumsalz sowie dem Umsetzungsprodukt von 1,3 g (0,01) des Pyrimidins des Beispiels 11 mit 1,05 g Phosgen und 1,35 ml Triäthylamin.
Ausbeute: 1,97 g Natriumsalz (34%);
IR-Spektrum: 1770, 1650, 1610, 1560 $cm^{-1}$;
NMR-Spektrum ($CD_3OD$) Signale bei ppm: 1,5 (6H), 4,15 (1H), 5,35–5,6 (3H), 6,8 (2H), 7,3 (2H), 8,45 (1H).

Beispiel 13

D-α-[3-(4-Hydroxy-2-methoxy-5-pyrimidyl)-ureido]-p-hydroxy-benzylpenicillin-Natrium

Dieses Penicillin wurde analog Beispiel 1 hergestellt. Man geht aus von 8,4 g Amoxicillin-trihydrat (0,02 Mol) sowie dem Umsetzungsprodukt von 2,82 5-Amino-4-hydroxy-2-methoxy-pyrimidin (0,02 Mol) mit 2,1 g Phosgen und 2,75 ml Triäthylamin.
Ausbeute: 7,5 g Natriumsalz (63%);
Rf.: 0,61;
IR-Spektrum: 1770, 1655, 1610, 1550 $cm^{-1}$;
NMR-Spektrum ($D_2O$) Signale bei ppm: 1,55 (6H), 3,85 (3H), 4,15 (1H), 5,3–5,7 (3H), 6,9 (2H), 7,4 (2H), 8,1 (1H).

Beispiel 14

D-α-[3-(4-Hydroxy-2-methoxy-5-pyrimidyl)-ureido]-benzylpenicillin-Natrium

Dieses Penicillin wurde analog Beispiel 1 hergestellt. Man geht aus von 4 g Ampicillin-trihydrat (0,01 Mol) sowie dem Umsetzungsprodukt von 1,41 g (0,01 Mol) 5-Amino-4-hydroxy-2-methoxy-pyrimidin mit 1,0 g Phosgen und 1,37 ml Triäthylamin.
Ausbeute: 3,41 g Natriumsalz (59%);
IR-Spektrum: 1770, 1660, 1600 $cm^{-1}$;
NMR-Spektrum ($D_2O$) Signale bei ppm: 1,5 (6H), 3,9 (3H), 4,2 (1H), 5,3–5,7 (3H), 7,5 (5H), 8,05 (1H).

Beispiel 15

D-α-[3-(2-Äthoxy-4-hydroxy-5-pyrimidyl-)-ureido]-p-hydroxybenzylpenicillin-Natrium

1,0 g (0,006 Mol) 5-Amino-2-äthoxy-4-hydroxy-pyrimidin werden in 50 ml absolutem Tetrahydrofuran mit 0,6 g Triäthylamin und 0,6 g Phosgen behandelt. Das nach Abfiltrieren und nach Abdestillieren des Lösungsmittels erhaltene Festprodukt wird in wenig Tetrahydrofuran gelöst und bei 0°C zu einer Lösung von 2,4 g Amoxycillintrihydrat in 80%igem, wäßrigem Tetrahydrofuran und Triäthylamin (pH 8,0) getropft.
Die Aufarbeitung erfolgte in der üblichen Weise.
Ausbeute: 2,2 g (63% der Theorie);
Rf.: 0,66;
IR-Spektrum: 1770, 1670, 1620, 1560 $cm^{-1}$;
NMR-Spektrum ($D_2O$) Signale bei ppm: 1,4 (3H), 1,5 (3H), 1,6 (3H), 4,2 (1H), 4,4 (2H), 5,3 (1H), 5,5 (2H), 6,8 (2H), 7,3 (2H), 8,1 (1H).

Beispiel 16

D-α-[3-(4-Hydroxy-2-methylamino-5-pyrimidyl)-ureido]-p-hydroxy-benzylpenicillin-Natrium

Dieses Penicillin wurde analog Beispiel 6 hergestellt. Man geht aus von 1,68 g (0,004 Mol) Amoxicillin-trihydrat sowie dem Umsetzungsprodukt von 560 mg (0,004 Mol) 5-Amino-4-hydroxy-2-methylamino-pyrimidin mit 400 mg Phosgen und 0,54 ml Triäthylamin.

Ausbeute: 940 mg Natriumsalz (43,5%);
Rf.: 0,45;
IR-Spektrum: 1770, 1650, 1600, 1530 $cm^{-1}$;
NMR-Spektrum ($DMSO + CD_3OD$) Signale bei ppm: 1,55 (6H), 2,75 (3H), 4,0 (1H), 5,4 (3H), 6,75 (2H), 7,25 (2H), 8,1 (1H).

## Beispiel 17

### D-α-[3-(4-Hydroxy-2-methylamino-5-pyrimidyl)-ureido]-benzylpenicillin-Natrium

Dieses Penicillin wurde analog Beispiel 16 hergestellt. Man geht aus von 375 mg Ampicillin-Natriumsalz (0,001 Mol) sowie dem Umsetzungsprodukt von 140 mg des Pyrimidins des Beispiels 73 mit 100 mg Phosgen und 0,14 ml Triäthylamin.

Ausbeute: 230 mg Natriumsalz (43%);
Rf.: 0,41;
IR-Spektrum: 1770, 1630, 1540 $cm^{-1}$;
NMR-Spektrum ($DMSO + CD_3OD$) Signale bei ppm: 1,55 (6H), 2,70 (3H), 4,05 (1H), 5,45 (3H), 7,35 (5H), 8,15 (1H).

## Beispiel 18

### D-α-[3-(2-Amino-4-hydroxy-5-pyrimidyl)-ureido]-p-hydroxybenzylpenicillin-Natrium

1,26 g (0,01 Mol) 2,5-Diamino-4-hydroxy-pyrimidin werden mit 20 ml Hexamethyldisilazan 3 h zum Rückfluß erhitzt, wobei langsam Stickstoff eingeleitet wird. Es entsteht eine gelbe Lösung. Hexamethyldisilazan wird dann mit Stickstoff abgeblasen. Das zurückbleibende ölige Produkt wird in 40 ml absolutem Tetrahydrofuran gelöst und unter Eiskühlung zu einer Lösung von 1,0 g Phosgen in 20 ml absolutem Tetrahydrofuran getropft. Nach der Zugabe wird das Eisbad entfernt und Stickstoff durch die Lösung geblasen, um überschüssiges Phosgen zu entfernen.

4,2 g Amoxycillin-trihydrat werden mit 2,5 ml Triäthylamin in 70 ml absolutem Dimethylformamid gelöst. Man gibt 2 g Magnesiumsulfat zu und rührt 1 Stunde bei Raumtemperatur. Die filtrierte Lösung wird unter Eisbadkühlung zu der oben hergestellten Lösung getropft. Man rührt 2 h bei Raumtemperatur und dampft dann im Vakuum zur Trockne ein. Dann gibt man 50 ml Wasser und 50 ml Essigsäureäthylester zu und stellt den pH-Wert auf 7,0. Die wäßrige Phase wird noch einmal mit Essigsäureäthylester ausgeschüttelt. Dann wird die wäßrige Phase mit 2 n Salzsäure unter Kühlen auf pH 2,8 gestellt. Das ausgefallene Produkt wird abgesaugt, mit wenig eiskaltem Wasser gewaschen und getrocknet. Mit Natriumhexanoat in Methanol/Äther wird das Natriumsalz hergestellt.

Ausbeute: 1,45 g Natriumsalz (29%);
Rf.: 0,34;
IR-Spektrum: 1770, 1660, 1605, 1535 $cm^{-1}$;
NMR-Spektrum ($DMSO + CD_3OD$) Signale bei ppm: 1,55 (6H), 4,05 (1H), 5,45 (3H), 6,8 (2H), 7,3 (2H), 8,1 (1H).

## Beispiel 19

### D-α-[3-(2-Äthylamino-4-hydroxy-5-pyrimidyl)-ureido]-p-hydroxy-benzylpenicillin-Natrium

Dieses Penicillin wurde analog Beispiel 6 hergestellt. Man geht aus von 6,3 g (0,015 Mol) Amoxicillin-trihydrat sowie dem Umsetzungsprodukt von 2,31 g (0,015 Mol) 5-Amino-2-äthylamino-4-hydroxy-pyrimidin mit 1,5 g Phosgen und 2,05 ml Triäthylamin.

Ausbeute: 4,9 g Natriumsalz (57,5%);
Rf.: 0,46;
IR-Spektrum: 1770, 1650, 1530 $cm^{-1}$;
NMR-Spektrum ($DMSO + CD_3OD$) Signale bei ppm: 1,5 (9H), 3,1 (2H), 4,0 (1H), 5,4 (q, 2H), 5,5 (1H), 6,75 (2H), 7,25 (2H), 8,1 (1H).

## Beispiel 20

D-α-[3-(2-Äthylamino-4-hydroxy-5-pyrimidyl)-ureido]-benzylpenicillin-Natrium

Dieses Penicillin wurde analog Beispiel 19 hergestellt. Man geht aus von 3,75 g Ampicillin-Natrium (0,01 Mol) sowie dem Umsetzungsprodukt von 1,53 g des Pyrimidins des Beispiels 76 (0,01 Mol) mit 1,0 g Phosgen und 1,35 ml Triäthylamin.

Ausbeute: 2,48 g Natriumsalz (51%);
Rf.: 0,49;
IR-Spektrum: 1770, 1660, 1610, 1555 $cm^{-1}$;
NMR-Spektrum ($DMSO+CD_3OD$) Signale bei ppm: 1,50 (3H), 1,55 (6H), 3,1 (2H), 4,05 (1H), 5,45 (3H), 7,4 (5H), 8,15 (1H).

## Beispiel 21

D-α-[3-(2-Äthylamino-4-hydroxy-5-pyrimidyl)-ureido]-cyclohexa-1,4-dien-1-ylmethylpenicillin-Natriumsalz

Dieses Penicillin wurde analog Beispiel 19 hergestellt. Man geht aus von 3,75 g Epicillin-Natrium (0,01 Mol) sowie dem Umsetzungsprodukt von 1,53 g des Pyrimidins des Beispiels 76 (0,01 Mol) mit 1,0 g Phosgen und 1,35 ml Triäthylamin.

Ausbeute: 2,66 g Natriumsalz (48%);
IR-Spektrum: 1770, 1650, 1610, 1535 $cm^{-1}$;
NMR-Spektrum ($DMSO+CD_3OD$) Signale bei ppm: 1,55 (9H), 2,50 (4H), 3,1 (2H), 4,0 (1H), 4,90 (1H), 5,35 (2H), 5,65 (3H), 8,15 (1H).

## Beispiel 22

D-α-[3-(4-Hydroxy-2-propylamino-5-pyrimidyl)-ureido]-p-hydroxy-benzylpenicillin-Natrium

Dieses Penicillin wurde analog Beispiel 6 hergestellt. Man geht aus von 2,1 g Amoxicillin-trihydrat (0,005 Mol) sowie dem Umsetzungsprodukt von 0,84 g 5-Amino-4-hydroxy-2-propylamino-pyrimidin (0,005 Mol) mit 0,5 g Phosgen und 0,68 ml Triäthylamin.

Ausbeute: 1,69 g Natriumsalz (58%);
IR-Spektrum: 1770, 1650, 1610, 1535 $cm^{-1}$;
NMR-Spektrum ($DMSO+CD_3OD$) Signale bei ppm: 1,2 (3H), 1,55 (6H), 1,9 (2H), 3,2 (2H), 4,05 (1H), 5,4 (3H), 6,8 (2H), 7,3 (2H), 8,15 (1H).

## Beispiel 23

D-α-[3-(4-Hydroxy-2-propylamino-5-pyrimidyl)-ureido]-benzylpenicillin-Natrium

Dieses Penicillin wurde analog Beispiel 6 hergestellt. Man geht aus von 2,0 g Ampicillin-trihydrat (0,005 Mol) sowie dem Umsetzungsprodukt von 0,84 g des Pyrimidins des Beispiels 22 (0,005 Mol) mit 0,5 g Phosgen und 0,68 ml Triäthylamin.

Ausbeute: 1,51 g Natriumsalz (54%);
IR-Spektrum: 1765, 1650, 1605, 1545 $cm^{-1}$;
NMR-Spektrum ($DMSO+CD_3OD$) Signale bei ppm: 1,25 (3H), 1,5 (6H), 1,9 (2H), 3,25 (2H), 4,0 (1H), 5,35 (q, 2H), 5,45 (1H), 7,5 (5H), 8,1 (1H).

## Beispiel 24

D-α-[3-(4-Hydroxy-2-isopropylamino-5-pyrimidyl)-ureido]-p-hydroxy-benzylpenicillin-Natrium

Dieses Penicillin wurde analog Beispiel 6 hergestellt. Man geht aus von 2,52 g Amoxicillin-trihydrat (0,006 Mol) sowie dem Umsetzungsprodukt von 1,02 g 5-Amino-4-hydroxy-2-isopropylaminopyrimidin (0,006 Mol) mit 600 mg Phosgen und 0,82 ml Triäthylamin.

Ausbeute: 2,17 g Natriumsalz (62%);
IR-Spektrum: 1765, 1650, 1610, 1535 $cm^{-1}$;
NMR-Spektrum ($DMSO+CD_3OD$) Signale bei ppm: 1,2 (6H), 1,55 (6H), 3,6 (1H), 4,05 (1H), 5,35 (2H), 5,45 (1H), 6,85 (2H), 7,35 (2H), 8,20 (1H).

Beispiel 25

D-α-[3-(4-Hydroxy-2-isopropylamino-5-pyrimidyl)-ureidc]-benzylpenicillin-Natrium

Dieses Penicillin wurde analog Beispiel 6 hergestellt. Man geht aus von 4,0 g Ampicillin-trihydrat (0,01 Mol) sowie dem Umsetzungsprodukt von 1,68 g des Pyrimidins des Beispiels 24 mit 1,0 g Phosgen und 1,35 ml Triäthylamin.

Ausbeute: 3,62 g Natriumsalz (64%);
IR-Spektrum: 1765, 1655, 1610, 1540 $cm^{-1}$;
NMR-Spektrum (DMSO + $CD_3OD$) Signale bei ppm: 1,2 (6H), 1,5 (6H), 3,65 (1H), 4,0 (1H), 5,35 (2H), 5,45 (1H), 7,45 (5H), 8,15 (1H).

Beispiel 26

D-α-[3-(4-Hydroxy-2-isobutylamino-5-pyrimidyl)-ureido]-p-hydroxy-benzylpenicillin-Natrium

Dieses Penicillin wurde analog Beispiel 6 hergestellt. Man geht aus von 1,68 g Amoxicillin-trihydrat (0,004 Mol) sowie dem Umsetzungsprodukt von 650 mg (0,004 Mol) 5-Amino-4-hydroxy-2-isobutylamino-pyrimidin mit 400 mg Phosgen und 0,54 ml Triäthylamin.

Ausbeute: 1,55 g Natriumsalz (65%);
IR-Spektrum: 1770, 1650, 1610, 1540 $cm^{-1}$;
NMR-Spektrum (DMSO + $CD_3OD$) Signale bei ppm: 1,0 (6H), 1,55 (6H), 1,8 (1H), 3,0 (2H), 4,05 (1H), 5,4 (3H), 6,8 (2H), 7,25 (2H), 8,2 (1H).

Beispiel 27

D-α-[3-(4-Hydroxy-2-isobutylamino-5-pyrimidyl)-ureido]-benzylpenicillin-Natrium

Dieses Penicillin wurde analog Beispiel 6 hergestellt. Man geht aus von 2,0 g Ampicillin-trihydrat (0,005 Mol) sowie dem Umsetzungsprodukt von 0,91 g des Pyrimidins des Beispiels 26 (0,005 Mol) mit 0,5 g Phosgen und 1,35 ml Triäthylamin.

Ausbeute: 1,66 g Natriumsalz (57%);
IR-Spektrum: 1765, 1655, 1610, 1545 $cm^{-1}$;
NMR-Spektrum (DMSO + $CD_3OD$) Signale bei ppm: 1,0 (6H), 1,5 (6H), 1,85 (1H), 3,1 (2H), 4,0 (1H), 5,45 (3H), 7,45 (5H), 8,15 (1H).

Beispiel 28

D-α-[3-(2-Butylamino-4-hydroxy-5-pyrimidyl)-ureido]-p-hydroxy-benzylpenicillin-Natrium

Dieses Penicillin wurde analog Beispiel 6 hergestellt. Man geht aus von 4,2 g (0,01 Mol) Amoxicillin-trihydrat sowie dem Umsetzungsprodukt von 1,82 g 5-Amino-2-butylamino-4-hydroxy-pyrimidin (0,01 Mol) mit 1,0 g Phosgen und 1,35 ml Triäthylamin.

Ausbeute: 2,85 g Natriumsalz (49%);
IR-Spektrum: 1765, 1655, 1610, 1540 $cm^{-1}$;
NMR-Spektrum (DMSO + $CD_3OD$) Signale bei ppm: 0,9 (3H), 1,4 (6H), 1,5 (6H), 3,0 (2H), 4,05 (1H), 5,45 (3H), 6,8 (2H), 7,35 (2H), 8,2 (1H).

Beispiel 29

D-α-[3-(2-Butylamino-4-hydroxy-5-pyrimidyl)-ureido]-benzylpenicillin-Natrium

Dieses Penicillin wurde analog Beispiel 28 hergestellt. Man geht aus von 4,0 g Ampicillin-trihydrat sowie dem Umsetzungsprodukt von 1,82 g (0,01 Mol) des Pyrimidins des Beispiels 28 mit 1,0 g Phosgen und 1,35 ml Triäthylamin.

Ausbeute: 3,02 g Natriumsalz (52%);
Ausbeute: 3,02 g Natriumsalz (52%);
IR-Spektrum: 1765, 1650, 1610, 1535 $cm^{-1}$;
NMR-Spektrum (DMSO + $CD_3OD$) Signale bei ppm: 0,9 (3H), 1,5 (10H), 2,9 (2H), 4,0 (1H), 5,4 (3H), 7,45 (5H), 8,15 (1H).

## Beispiel 30

### D-α-[3-(2-Hexylamino-4-hydroxy-5-pyrimidyl)-ureido]-p-hydroxy-benzylpenicillin-Natrium

Dieses Penicillin wurde analog Beispiel 6 hergestellt. Man geht aus von 1,26 g Amoxicillin-trihydrat (0,003 Mol) sowie dem Umsetzungsprodukt von 625 mg (0,003 Mol) 5-Amino-2-hexylamino-4-hydroxy-pyrimidin mit 300 mg Phosgen und 0,4 ml Triäthylamin.

Ausbeute: 1,05 g Natriumsalz (54%);
IR-Spektrum: 1770, 1665, 1610, 1520 $cm^{-1}$;
NMR-Spektrum ($DMSO+CD_3OD$) Signale bei ppm: 0,9 (3H), 1,2—1,8 (14H), 3,3 (2H), 4,0 (1H), 5,35 (2H), 5,45 (1H), 6,85 (2H), 7,35 (2H), 8,15 (1H).

Analog wurde synthetisiert:
D-α-[3-(4-Hydroxy-2-isopropylamino-5-pyrimidyl)-ureido]-m,p-dihydroxybenzylpenicillin-Natrium
Ausbeute: 48,5%;
IR-Spektrum: 1765, 1655, 1600, 1540 $cm^{-1}$;
NMR-Spektrum ($DMSO+CD_3OD$) Signale bei ppm: 1,25 (d, 6H), 1,55 (d, 6H), 3,55 (m, 1H), 4,05 (s, 1H), 5,45 (m, 3H), 6,8 (m, 2H), 7,15 (m, 1H), 8,10 (s, 1H).

## Beispiel 31

### D-α-[3-(2-Allylamino-4-hydroxy-5-pyrimidyl)-ureido]-p-hydroxy-benzylpenicillin-Natrium

Dieses Penicillin wurde analog Beispiel 6 hergestellt. Man geht aus von 2,1 g Amoxycillin-trihydrat (0,005 Mol) sowie dem Umsetzungsprodukt von 840 mg (0,005 Mol) 2-Allylamino-5-amino-4-hydroxy-pyrimidin mit 500 mg Phosgen und 0,67 ml Triäthylamin.

Ausbeute: 1,92 g Natriumsalz (66%);
IR-Spektrum: 1770, 1660, 1610, 1550, 1510 $cm^{-1}$;
NMR-Spektrum ($DMSO+CD_3OD$) Signale bei ppm: 1,5 (6H), 3,9 (2H), 3,95 (1H), 5,0—5,6 (m, 5H), 5,9 (m, 1H), 6,75 (2H), 7,25 (2H), 8,1 (1H).

## Beispiel 32

### D-α-[3-(2-Allylamino-4-hydroxy-5-pyrimidyl)-ureido]-benzylpenicillin-Natrium

Dieses Penicillin wurde analog Beispiel 31 hergestellt. Man geht aus von 1,85 g Ampicillin-Natrium (0,005 Mol) sowie dem Umsetzungsprodukt von 840 mg des Pyrimidins des Beispiels 89 mit 500 mg Phosgen und 0,67 ml Triäthylamin.

Ausbeute: 1,7 g Natriumsalz (60%);
IR-Spektrum: 1770, 1660, 1610, 1550 $cm^{-1}$;
NMR-Spektrum ($DMSO+CD_3OD$) Signale bei ppm: 1,5 (6H), 3,95 (2H), 4,0 (1H), 5,0—5,5 (m, 5H), 6,0 (m, 1H), 7,4 (5H), 8,1 (1H).

## Beispiel 33

### D-α-[3-(2-Allylamino-4-hydroxy-5-pyrimidyl)-ureido]-cyclohexa-1,4-dien-1-yl-methylpenicillin-Natrium

Dieses Penicillin wurde analog Beispiel 31 hergestellt. Man geht aus von 3,75 g Epicillin-Natrium (0,01 Mol) sowie dem Umsetzungsprodukt von 1,66 g des Amins des Beispiels 31 mit 1,0 g Phosgen und 1,35 ml Triäthylamin.

Ausbeute: 3,5 g Natriumsalz (64%);
IR-Spektrum: 1765, 1655, 1605, 1550 $cm^{-1}$;
NMR-Spektrum ($DMSO+CD_3OD$) Signale bei ppm: 1,5 (6H), 2,55 (4H), 3,9 (2H), 4,05 (1H), 4,95 (1H), 5,0—5,7 (m, 7H), 6,0 (1H), 8,1 (1H).

Beispiel 34

D-α-[3-(2-Crotylamino-4-hydroxy-5-pyrimidyl)-ureido]-p-hydroxy-benzylpenicillin-Natrium

Dieses Penicillin wurde analog Beispiel 6 hergestellt. Man geht aus von 4,2 g Amoxycillin-trihydrat (0,01 Mol) sowie dem Umsetzungsprodukt von 1,8 g 5-Amino-2-crotylamino-4-hydroxy-pyrimidin mit 1,0 g Phosgen und 1,37 ml Triäthylamin.

Ausbeute: 3,27 g Natriumsalz (55%);
IR-Spektrum: 1770, 1660, 1615, 1550 $cm^{-1}$;
NMR-Spektrum (DMSO+$CD_3OD$) Signale bei ppm: 1,5 (9H), 3,8 (2H), 4,0 (1H), 5,5 (m, 5H), 6,8 (2H), 7,35 (2H), 8,2 (1H).

Beispiel 35

D-α-[3-(2-Crotylamino-4-hydroxy-5-pyrimidyl)-ureido]-benzylpenicillin-Natrium

Dieses Penicillin wurde analog Beispiel 6 hergestellt. Man geht aus von 3,7 g Ampicillin-Natrium (0,01 Mol) sowie dem Umsetzungsprodukt von 1,8 g des Amins des Beispiels 34 (0,01 Mol) mit 1,0 g Phosgen und 1,37 ml Triäthylamin.

Ausbeute: 3,55 g Natriumsalz (59%);
IR-Spektrum: 1770, 1660, 1610, 1555 $cm^{-1}$;
NMR-Spektrum (DMSO+$CD_3OD$) Signale bei ppm: 1,55 (9H), 3,75 (2H), 4,05 (1H), 5,3—5,9 (m, 5H), 7,45 (5H), 8,15 (1H).

Beispiel 36

D-α-[3-(4-Hydroxy-2-propargylamino-5-pyrimidyl)-ureido]-p-hydroxy-benzylpenicillin-Natrium

Dieses Penicillin wurde analog Beispiel 6 hergestellt. Man geht aus von 8,4 g Amoxicillin-trihydrat (0,02 Mol) sowie dem Umsetzungsprodukt von 3,28 g (0,02 Mol) 5-Amino-4-hydroxy-2-propargylamino-pyrimidin mit 2,05 g Phosgen und 2,70 ml Triäthylamin.

Ausbeute: 6,95 g Natriumsalz (60%);
IR-Spektrum: 1765, 1655, 1610, 1540 $cm^{-1}$;
NMR-Spektrum ($D_2O$) Signale bei ppm: 1,5 (6H), 2,6 (1H), 3,9 (1H), 4,15 (2H), 5,5 (3H), 6,9 (2H), 7,3 (2H), 8,05 (1H).

Beispiel 37

D-α-[3-(4-Hydroxy-2-propargylamino-5-pyrimidyl)-ureido]-benzylpenicillin-Natrium

Dieses Penicillin wurde analog Beispiel 6 hergestellt. Man geht aus von 3,7 g Ampicillin-Natrium (0,01 Mol) sowie dem Umsetzungsprodukt von 1,64 g (0,01 Mol) 5-Amino-4-hydroxy-2-propargylamino-pyrimidin mit 1,05 g Phosgen und 1,37 ml Triäthylamin.

Ausbeute: 2,85 g Natriumsalz (50,5%);
IR-Spektrum: 1765, 1650, 1605, 1545 $cm^{-1}$;
NMR-Spektrum ($D_2O$) Signale bei ppm: 1,55 (6H), 2,55 (1H), 3,95 (1H), 4,15 (2H), 5,45 (3H), 7,5 (5H), 8,1 (1H).

Beispiel 38

D-α-[3-(2-Dimethylamino-4-hydroxy-5-pyrimidinyl)-ureido]-p-hydroxy-benzylpenicillin-Natrium

1,74 g D-α-[3-(2-Dimethylamino-4-hydroxy-5-pyrimidinyl)-ureido]-p-hydroxy-phenylglycin werden zusammen mit 500 mg N-Methylmorpholin in 40 ml absolutem Tetrahydrofuran und 10 ml absolutem Dimethylformamid gelöst. Man kühlt auf −15°C ab und tropft eine Lösung von 550 mg (0,0055 Mol) Chlorameisensäureäthylester in 5 ml abs. Tetrahydrofuran zu. Die klare Lösung wird 30 Min. bei −10°C gerührt. Dann tropft man eine Lösung von 1,58 g 6-Aminopenicillansäure-triäthylammoniumsalz (0,005 Mol) in 20 ml Methylenchlorid so zu, daß die Temperatur −10°C nicht übersteigt. Man läßt eine Stunde bei −5°C, eine Stunde bei 5°C und eine Stunde bei Raumtemperatur nachreagieren. Anschließend engt man im Vakuum zur Trockne ein, gibt 50 ml Wasser zu und stellt den pH-Wert auf 7,0. Man schüttelt die wäßrige Phase zweimal mit Essigester aus, überschichtet dann mit 200 ml Essigester und gibt unter Kühlen und Rühren verdünnte Salzsäure zu, bis der pH-Wert 2,0 erreicht hat. Die Essigesterphase wird abgetrennt, die wäßrige Phase noch einmal mit 50 ml Essigester ausgeschüttelt, die beiden Essigesterphasen vereinigt, über Natriumsulfat getrocknet und zur Trockne eingeengt. Die freie Säure wird dann in Methanol/Äther mit Natriumhexanoat in das Natriumsalz überführt.

Ausbeute: 1,62 Natriumsalz (57%);
Rf: 0,46;
IR-Spektrum: 1765, 1660, 1610, 1550, 1530 $cm^{-1}$;
NMR-Spektrum ($DMSO + CD_3OD$) Signale bei ppm: 1,55 (6H), 3,1 (6H), 4,05 (1H), 5,4 (q, 2H), 5,5 (1H), 6,85 (2H), 7,35 (2H), 8,2 (1H).

## Beispiel 39

D-α-[3-(2-Dimethylamino-4-hydroxy-5-pyrimidyl)-ureido]-benzylpenicillin-Natrium

Dieses Penicillin wurde analog Beispiel 38 hergestellt. Man geht aus von 3,16 g 6-Aminopenicillansäure-triäthylammoniumsalz (0,01 Mol) sowie dem Umsetzungsprodukt von 3,32 g (0,01 Mol) D-α-[3-(2-Dimethylamino-4-hydroxy-5-pyrimidyl)-ureido]-phenylglycin (0,01 Mol) mit 1,0 g N-Methylmorpholin und 1,1 g Chlorameisensäureäthylester (0,01 Mol).

Ausbeute: 2,52 g Natriumsalz (46%);
Rf: 0,44;
IR-Spektrum: 1770, 1660, 1610, 1555 $cm^{-1}$;
NMR-Spektrum ($DMSO + CD_3OD$) Signale bei ppm: 1,55 (6H), 3,05 (2H), 4,05 (1H), 5,35 (2H), 5,45 (1H), 7,4 (5H), 8,25 (1H).

## Beispiel 40

D-α-[3-(2-Cyclopropylamino-4-hydroxy-5-pyrimidyl)-ureido]-p-hydroxy-benzylpenicillin-Natrium

Dieses Penicillin wurde analog Beispiel 6 hergestellt. Man geht aus von 4,2 g Amoxicillin-trihydrat (0,01 Mol) sowie dem Umsetzungsprodukt von 1,66 g (0,01 Mol) 5-Amino-2-cyclopropylamino-4-hydroxy-pyrimidin mit 1,0 g Phosgen und 1,37 ml Triäthylamin.

Ausbeute: 3,48 g Natriumsalz (60%);
Rf: 0,56;
IR-Spektrum: 1770, 1665, 1615, 1540 $cm^{-1}$;
NMR-Spektrum ($DMSO + CD_3OD$) Signale bei ppm: 0,5 (4H), 1,55 (6H), 3,7 (1H), 4,0 (1H), 5,5 (3H), 6,9 (2H), 7,35 (2H), 8,25 (1H).

## Beispiel 41

D-α-[3-(2-Cyclopropylamino-4-hydroxy-5-pyrimidyl)-ureido]-benzylpenicillin-Natrium

Dieses Penicillin wurde analog Beispiel 6 hergestellt. Man geht aus von 3,75 g Ampicillin-Natrium sowie dem Umsetzungsprodukt von 1,66 g des Amins des Beispiels 100 (0,01 Mol) mit 1,0 g Phosgen und 1,37 ml Triäthylamin.

Ausbeute: 2,93 g Natriumsalz (52%);
Rf: 0,59;
IR-Spektrum: 1765, 1660, 1610, 1525 $cm^{-1}$;
NMR-Spektrum ($DMSO + CD_3OD$) Signale bei ppm: 0,6 (4H), 1,55 (6H), 3,75 (1H), 4,05 (1H), 5,35 (2H), 5,45 (1H), 6,85 (2H), 7,35 (2H), 8,20 (1H).

## Beispiel 42

D-α-[3-(2-Cyclobutylamino-4-hydroxy-5-pyrimidyl)-ureido]-p-hydroxy-benzylpenicillin-Natrium

Dieses Penicillin wurde analog Beispiel 6 hergestellt. Man geht aus von 420 mg Amoxicillin-trihydrat (0,001 Mol) sowie dem Umsetzungsprodukt von 180 mg 5-Amino-2-cyclobutylamino-4-hydroxy-pyrimidin (0,001 Mol) mit 100 mg Phosgen und 0,14 ml Triäthylamin.

Ausbeute: 290 mg Natriumsalz (59%);
Rf: 0,59.

## Beispiel 43

D-α-[3-(2-Cyclopentylamino-4-hydroxy-pyrimidyl)-ureido]-p-hydroxy-benzylpenicillin-Natrium

Dieses Penicillin wurde analog Beispiel 6 hergestellt. Man geht aus von 840 mg Amoxicillin-trihydrat (0,002 Mol) sowie dem Umsetzungsprodukt von 390 mg (0,002 Mol) 5-Amino-2-cyclopentylamino-4-hydroxy-pyrimidin mit 200 mg Phosgen und 0,27 ml Triäthylamin.

Ausbeute: 610 mg Natriumsalz (50%);
Rf: 0,61;
IR-Spektrum: 1770, 1660, 1615, 1540 $cm^{-1}$;
NMR-Spektrum ($DMSO+CD_3OD$) Signale bei ppm: 1,2—2,2 (m, 14H), 3,8 (1H), 4,1 (1H), 5,5 (3H), 6,9 (2H), 7,4 (2H), 8,3 (1H).

## Beispiel 44

D-α-[3-(2-Cyclopentylamino-4-hydroxy-5-pyrimidyl)-ureido]-benzylpenicillin-Natrium

Dieses Penicillin wurde analog Beispiel 6 hergestellt. Man geht aus von 750 mg Ampicillin-Natrium (0,002 Mol) sowie dem Umsetzungsprodukt von 380 mg (0,002 Mol) des Amins des Beispiels 43 mit 200 mg Phosgen und 0,27 ml Triäthylamin.

Ausbeute: 685 mg Natriumsalz (56%);
Rf: 0,61;
IR-Spektrum: 1770, 1660, 1615, 1545 $cm^{-1}$;
NMR-Spektrum ($DMSO+CD_3OD$) Signale bei ppm: 1,0—2,3 (m, 14H), 3,75 (1H), 4,1 (1H), 5,35 (2H), 5,45 (1H), 7,4 (5H), 8,25 (1H).

## Beispiel 45

D-α-[3-(2-Cyclohexylamino-4-hydroxy-5-pyrimidyl)-ureido]-p-hydroxy-benzylpenicillin-Natrium

Dieses Penicillin wurde analog Beispiel 6 hergestellt. Man geht aus von 4,2 g Amoxicillin-trihydrat (0,01 Mol) sowie dem Umsetzungsprodukt von 2,1 g (0,01 Mol) 5-Amino-2-cyclohexylamino-4-hydroxy-pyrimidin mit 1,0 g Phosgen und 1,35 ml Triäthylamin.

Ausbeute: 4,12 g Natriumsalz (65,5%);
Rf: 0,63;
IR-Spektrum: 1770, 1665, 1620, 1525 $cm^{-1}$;
NMR-Spektrum ($DMSO+CD_3OD$) Signale bei ppm: 1,0—2,2 (m, 16H), 3,75 (1H), 4,05 (1H), 5,35 (q, 2H), 5,45 (1H), 6,85 (2H), 7,35 (2H), 8,30 (1H).

## Beispiel 46

D-α-[3-(2-Cyclohexylamino-4-hydroxy-5-pyrimidyl)-ureido]-benzylpenicillin-Natrium

Dieses Penicillin wurde analog Beispiel 6 hergestellt. Man geht aus von 1,85 g (0,005 Mol) Ampicillin-trihydrat sowie dem Umsetzungsprodukt von 1,07 g (0,005 Mol) des Amins des Beispiels 105 mit 500 mg Phosgen und 0,67 ml Triäthylamin.

Ausbeute: 1,78 g Natriumsalz (57,5%);
Rf: 0,65;
IR-Spektrum: 1765, 1650, 1600, 1545, 1510 $cm^{-1}$;
NMR-Spektrum ($DMSO+CD_3OD$) Signale bei ppm: 1,1—2,2 (m, 16H), 3,7 (1H), 4,05 (1H), 5,35 (2H), 5,45 (1H), 7,4 (5H), 8,25 (1H).

## Beispiel 47

D-α-[3-(2-Cyclohexylamino-4-hydroxy-5-pyrimidyl)-ureido]-
cyclohexa-1,4-dien-1-yl-methyl-penicillin-Natrium

Dieses Penicillin wurde analog Beispiel 6 hergestellt. Man geht aus von 3,75 g Epicillin-Natrium (0,01 Mol) sowie dem Umsetzungsprodukt von 2,08 g (0,01 Mol) 5-Amino-2-cyclohexylamino-4-hydroxy-5-pyrimidin mit 1,0 g Phosgen und 1,37 ml Triäthylamin.

Ausbeute: 3,15 g Natriumsalz (51%);
Rf: 0,64;
IR-Spektrum: 1765, 1655, 1610, 1540 $cm^{-1}$;
NMR-Spektrum (DMSO+$CD_3OD$) Signale bei ppm: 1,1—2,3 (m, 16H), 2,5 (4H), 3,75 (1H), 4,05 (1H), 4,85 (1H), 5,35 (2H), 5,60 (3H), 8,25 (1H).

## Beispiel 48

D-α-[3-(2-Cycloheptylamino-4-hydroxy-5-pyrimidyl)-ureido]-p-hydroxy-benzylpenicillin-Natrium

Dieses Penicillin wurde analog Beispiel 6 hergestellt. Man geht aus von 2,52 g (0,006 Mol) Amoxicillin-trihydrat sowie dem Umsetzungsprodukt von 1,33 g 5-Amino-2-cycloheptylamino-4-hydroxy-pyrimidin (0,006 Mol) mit 600 mg Phosgen und 0;82 ml Triäthylamin.
Ausbeute: 2,55 g Natriumsalz (66%);
Rf: 0,69;
IR-Spektrum: 1770, 1650, 1610, 1545 $cm^{-1}$;
NMR-Spektrum (DMSO+$CD_3OD$) Signale bei ppm: 1,0—2,3 (m, 18H), 3,65 (1H), 4,05 (1H), 5,35 (2H), 5,45 (1H), 6,8 (2H), 7,3 (2H), 8,25 (1H).

## Beispiel 49

D-α-[3-(2-Cyclohexylmethylamino-4-hydroxy-5-pyrimidyl)-ureido]-p-hydroxy-benzylpenicillin-Natrium

Dieses Penicillin wurde analog Beispiel 6 hergestellt. Man geht aus von 3,36 g (0,008 Mol) Amoxicillin-trihydrat sowie dem Umsetzungsprodukt von 1,78 g (0,008 Mol) 5-Amino-2-cyclohexylmethylamino-4-hydroxy-pyrimidin mit 800 mg Phosgen und 1,1 ml Triäthylamin.
Ausbeute: 2,78 g Natriumsalz (55%);
Rf: 0,69;
IR-Spektrum: 1770, 1665, 1620, 1545, 1510 $cm^{-1}$;
NMR-Spektrum (DMSO+$CD_3OD$) Signale bei ppm: 1,0—2,3 (m, 17H), 3,4 (2H), 4,05 (1H), 5,45 (3H), 6,85 (2H), 7,35 (2H), 8,15 (1H).

## Beispiel 50

D-α-[3-(2-Cyclopropylmethylamino-4-hydroxy-5-pyrimidyl)-ureido]-p-hydroxy-benzylpenicillin-Natrium

Dieses Penicillin wurde analog Beispiel 6 hergestellt. Man geht aus von 700 mg Amoxicillin-trihydrat (0,00167 Mol) sowie dem Umsetzungsprodukt von 300 mg (0,0017 Mol) 5-Amino-2-cyclopropylmethylamino-4-hydroxy-pyrimidin mit 170 mg Phosgen und 0,23 ml Triäthylamin.
Ausbeute: 520 g Natriumsalz (52,5%);
Rf: 0,62;
IR-Spektrum: 1765, 1660, 1610, 1520 $cm^{-1}$;
NMR-Spektrum (DMSO+$CD_3OD$) Signale bei ppm: 0,4 (4H), 1,3 (1H), 1,55 (6H), 3,3 (2H), 4,1 (1H), 5,45 (3H), 6,85 (2H), 7,35 (2H), 8,15 (1H).

## Beispiel 51

D-α-[3-(2-Cyclopropylmethylamino-4-hydroxy-5-pyrimidyl)-ureido]-benzylpenicillin-Natrium

Dieses Penicillin wurde analog Beispiel 6 hergestellt. Man geht aus von 750 mg (0,002 Mol) Ampicillin-Natrium sowie dem Umsetzungsprodukt von 360 mg (0,002 Mol) des Pyrimidins des Beispiels 114 mit 200 mg Phosgen und 0,27 ml Triäthylamin.
Ausbeute: 690 mg Natriumsalz (69%);
Rf: 0,60;
IR-Spektrum: 1770, 1660, 1610, 1545, 1510 $cm^{-1}$;
NMR-Spektrum (DMSO+$CD_3OD$) Signale bei ppm: 0,5 (4H), 1,35 (1H), 1,55 (6H), 3,35 (2H), 4,05 (1H), 5,35 (2H), 5,45 (1H), 7,5 (5H), 8,15 (1H).

Beispiel 52

D-α-[3-(4-Hydroxy-2-pyrrolidino-5-pyrimidyl)-ureido]-p-hydroxy-benzylpenicillin-Natrium

Dieses Penicillin wird analog Beispiel 6 synthetisiert. Man geht aus von 4,2 g Amoxicillin-trihydrat (0,01 Mol) sowie dem Umsetzungsprodukt von 1,8 g 5-Amino-4-hydroxy-2-pyrrolidino-pyrimidin. Die Aufarbeitung wird wie folgt abgeändert: Die wäßrige Phase wird einmal bei pH 7,0 mit Essigester ausgeschüttelt und anschließend unter Kühlung mit verdünnter Salzsäure bis pH 2,5 versetzt. Das ausgefallene Produkt wird schnell abgesaugt und mit wenig Wasser gewaschen. Zur Herstellung des Natriumsalzes wird das Produkt in wenig Wasser aufgeschlämmt, mit 1/10 Natronlauge unter Kühlung tropfenweise bis pH 6,8 versetzt und gefriergetrocknet.

Ausbeute: 3,33 g Natriumsalz (55%);
Rf: 0,62;
IR-Spektrum: 1770, 1665, 1620, 1550 $cm^{-1}$;
NMR-Spektrum ($DMSO+CD_3OD$) Signale bei ppm: 1,5 (10H), 3,55 (4H), 4,0 (1H), 5,4 (q, 2H), 5,5 (1H), 6,8 (2H), 7,35 (2H), 8,1 (1H).

Beispiel 53

D-α-[3-(4-Hydroxy-2-pyrrolidino-5-pyrimidyl)-ureido]-benzylpenicillin-Natrium

Dieses Penicillin wurde analog Beispiel 52 hergestellt. Man geht aus von 750 mg (0,002 Mol) Ampicillin-Natrium sowie dem Umsetzungsprodukt von 360 mg 5-Amino-4-hydroxy-2-pyrrolidino-pyrimidin (0,002 Mol) mit 200 mg Phosgen und 0,27 ml Triäthylamin.

Ausbeute: 710 mg Natriumsalz (61%);
Rf: 0,63;
IR-Spektrum: 1770, 1665, 1620, 1500 $cm^{-1}$;
NMR-Spektrum ($DMSO+CD_3OD$) Signale bei ppm: 1,55 (10H), 3,5 (4H), 4,05 (1H), 5,45 (3H), 7,5 (5H), 8,15 (1H).

Beispiel 54

D-α-[3-(4-Hydroxy-2-piperidino-5-pyrimidyl)-ureido]-p-hydroxy-benzylpenicillin-Natrium

Dieses Penicillin wurde analog Beispiel 6 hergestellt. Man geht aus von 840 mg Amoxicillin-trihydrat (0,002 Mol) sowie dem Umsetzungsprodukt von 385 mg 5-Amino-4-hydroxy-2-piperidino-pyrimidin mit 200 mg Phosgen und 0,27 ml Triäthylamin.

Ausbeute: 770 mg Natriumsalz (63%);
Rf: 0,67;
IR-Spektrum: 1770, 1670, 1620, 1550 $cm^{-1}$;
NMR-Spektrum ($DMSO+CD_3OD$) Signale bei ppm: 1,55 (12H), 3,55 (4H), 4,05 (1H), 5,4 (3H), 6,8 (2H), 7,35 (2H), 8,1 (1H).

Beispiel 55

D-α-[3-(4-Hydroxy-2-morpholino-5-pyrimidyl)-ureido]-p-hydroxy-benzylpenicillin-Natrium

Dieses Penicillin wurde analog Beispiel 6 hergestellt. Man geht aus von 4,2 g Amoxycillin-trihydrat (0,01 Mol) sowie dem Umsetzungsprodukt von 1,96 g (0,01 Mol) 5-Amino-4-hydroxy-2-morpholino-pyrimidin mit 1,0 g Phosgen und 1,37 ml Triäthylamin.

Ausbeute: 3,72 g Natriumsalz (61%);
IR-Spektrum: 1770, 1650, 1600, 1530 $cm^{-1}$;
NMR-Spektrum ($DMSO+CD_3OD$) Signale bei ppm: 1,50 (6H), 3,5 (4H), 3,7 (4H), 4,0 (1H), 5,4 (q, 2H), 5,5 (1H), 6,8 (2H), 7,3 (2H), 8,2 (1H).

Beispiel 56

D-α-[3-(4-Hydroxy-2-morpholino-5-pyrimidyl)-ureido]-benzylpenicillin-Natrium

Dieses Penicillin wurde analog Beispiel 6 hergestellt. Man geht aus von 2,0 g (0,0054 Mol) Ampicillin-Natrium sowie dem Umsetzungsprodukt von 1,06 g (0,0054 Mol) 5-Amino-4-hydroxy-2-morpholino-pyrimidin mit 550 mg Phosgen und 0,75 ml Triäthylamin.

Ausbeute: 1,62 g Natriumsalz (49%);
IR-Spektrum: 1770, 1655, 1605, 1530 $cm^{-1}$;
NMR-Spektrum ($DMSO+CD_3OD$) Signale bei ppm: 1,55 (6H), 3,45 (4H), 3,70 (4H), 4,05 (1H), 5,35 (q, 2H), 5,45 (1H), 7,45 (5H), 8,15 (1H).

## Beispiel 57

D-α-[3-(2-Anilino-4-hydroxy-5-pyrimidyl)-ureido]-p-hydroxybenzylpenicillin-Natrium

600 mg (0,003 Mol) 5-Amino-2-anilino-4-hydroxy-pyrimidin werden in der Hitze in absolutem Tetrahydrofuran zusammen mit 300 mg Triäthylamin gelöst. Dieses Gemisch wird unter Eiskühlung zu einer Lösung von 305 mg Phosgen in 50 ml absolutem Tetrahydrofuran getropft. Nach dem Zusammengeben wird eine halbe Stunde nachgerührt und anschließend im Vakuum auf etwa 50 ml eingeengt. Die Mischung wird zu einer in der Kälte mit Triäthylamin bereiteten Lösung von 1,26 g Amoxycillin-trihydrat (0,003 Mol) in 60 ml 80%igem wäßrigem Tetrahydrofuran unter Eisbadkühlung zugetropft. Dabei wird der pH-Wert mit Triäthylamin bei etwa 7,5 gehalten. Nach Zugabe wird eine Stunde bei 5°C und eine Stunde bei Raumtemperatur nachgerührt. Tetrahydrofuran wird dann im Vakuum abgezogen, mit Wasser auf etwa 50 ml aufgefüllt und bei pH 7,0 zweimal mit Essigsäureäthylester extrahiert. Anschließend wird mit Eis gekühlt, mit 200 ml Essigsäureäthylester überschichtet und mit verdünnter Salzsäure und heftigem Rühren auf pH 2,0 gestellt. Die organische Phase wird abgetrennt, die wäßrige Phase noch einmal mit 50 ml Essigsäureäthylester extrahiert, die organischen Phasen vereinigt, mit Natriumsulfat getrocknet und das Lösemittel im Vakuum entfernt.

Das zurückbleibende Festprodukt wird in wenig Äthanol gelöst, mit Natriumhexanoat versetzt und durch Zugabe von absolutem Äther das Natriumsalz ausgefällt.
Ausbeute: 1,46 g (79%);
IR-Spektrum: 1775, 1660, 1610, 1540 $cm^{-1}$;
NMR-Spektrum ($DMSO+CD_3OD$) Signale bei ppm: 1,55 (6H), 4,1 (1H), 5,5 (3H), 6,8 (d, 2H), 7,35 (m, 7H), 8,35 (1H).

## Beispiel 58

D-α-[3-(2-Anilino-4-hydroxy-5-pyrimidyl)-ureido]-benzylpenicillin-Natrium

Analog Beispiel 57, ausgehend von 2,0 g (0,005 Mol) Ampicillin-trihydrat und dem Umsetzungsprodukt von 1,0 g (0,005 Mol) 5-Amino-2-anilino-4-hydroxy-pyrimidin mit 500 mg Phosgen.
Ausbeute: 2,25 g (75%) Natriumsalz;
IR-Spektrum: 1775, 1660, 1610, 1540 $cm^{-1}$;
NMR-Spektrum: 1,55 (6H), 4,05 (1H), 5,4 (q, 2H), 5,6 (1H), 7,3 (m, 10H), 8,40 (1H).

## Beispiel 59

D-α-[3-(2-Anilino-4-hydroxy-5-pyrimidyl)-ureido]-1,4-cyclohexadien-1-yl-methylpenicillin-Natrium

Analog Beispiel 57, ausgehend von 3,73 g Epicillin-Natrium (0,01 Mol) und dem Umsetzungsprodukt von 2,0 g (0,01 Mol) 5-Amino-2-anilino-4-hydroxy-pyrimidin mit 1,0 g Phosgen.
Ausbeute: 4,56 g Natriumsalz (76%);
IR-Spektrum: 1770, 1655, 1610, 1545 $cm^{-1}$;
NMR-Spektrum: 1,50 (6H), 2,50 (4H), 4,05 (1H), 4,90 (1H), 5,30 (2H), 5,65 (3H), 7,45 (5H), 8,35 (1H).

## Beispiel 60

D-α-[3-(2-p-Chloranilino-4-hydroxy-5-pyrimidyl)-ureido]-p-hydroxybenzylpenicillin-Natrium

Analog Beispiel 57, ausgehend von 1,68 g (0,004 Mol) Amoxycillin-trihydrat und dem Umsetzungsprodukt von 940 mg (0,004 Mol) 5-Amino-2-p-chloranilino-4-hydroxypyrimidin mit 410 mg Phosgen und 400 mg Triäthylamin.
Ausbeute: 2,1 g (81%);
IR-Spektrum: 1770, 1660, 1610, 1540, 1515 $cm^{-1}$;
NMR-Spektrum ($DMSO+CD_3OD$) Signale bei ppm: 1,55 (6H), 4,1 (1H), 5,5 (q, 2H), 5,6 (1H), 6,85 (d, 2H), 7,4 (m, 4H), 7,9 (d, 2H), 8,45 (1H).

## Beispiel 61

D-α-[3-(2-p-Chloranilino-4-hydroxy-5-pyrimidyl)-ureido]-cyclohexa-1,4-dien-1-yl-methylpenicillin-Natrium

Analog Beispiel 57, ausgehend von 1,87 g Epicillin-Natrium (0,005 Mol) und 1,18 g des Pyrimidins des Beispiels 60 (0,005 Mol) sowie 510 mg Phosgen und 500 mg Triäthylamin.

Ausbeute: 2,29 g (74%) Natriumsalz;

IR-Spektrum: 1765, 1655, 1610, 1540, 1505 $cm^{-1}$;

NMR-Spektrum (DMSO + $CD_3OD$): Signale bei ppm: 1,55 (6H), 2,55 (4H), 4,05 (1H), 4,95 (1H), 5,35 (2H), 5,70 (3H), 7,35 (d, 2H), 7,85 (d, 2H), 8,40 (1H).

## Beispiel 62

D-α-[3-(2-p-Chloranilino-4-hydroxy-5-pyrimidyl) ureido]-benzylpenicillin-Natrium

Dieses Penicillin wurde analog Beispiel 57 hergestellt. Man geht aus von 3,75 g Ampicillin-Natrium (0,01 Mol) sowie dem Umsetzungsprodukt von 2,36 g des Pyrimidins des Beispiels 60 (0,01 Mol) mit 1,0 g Phosgen und 1,35 ml Triäthylamin.

Ausbeute: 4,5 g Natriumsalz (69%);

IR-Spektrum: 1770, 1650, 1610, 1540 $cm^{-1}$;

NMR-Spektrum (DMSO + $CD_3OD$) Signale bei ppm: 1,55 (6H), 4,05 (1H), 5,5 (3H), 7,5 (7H), 7,9 (2H), 8,4 (1H).

## Beispiel 63

D-α-[3-(2-p-Fluoranilino-4-hydroxy-5-pyrimidyl)-ureido]-p-hydroxybenzylpenicillin-Natrium

2,19 g (0,01 Mol) 5-Amino-2-p-fluoranilino-4-hydroxy-pyrimidin werden mit 1,05 g Phosgen und 1,0 g Triäthylamin wie in Beispiel 57 angegeben umgesetzt.

4,2 g (0,01 Mol) Amoxycillin werden unter Eiskühlung in 80 ml 80%igem wäßrigem Tetrahydrofuran vorsichtig mit 10 ml 1 n Natronlauge in Lösung gebracht. Zu dem entstandenen Amoxycillin-Natrium-Salz wird unter Eiskühlung die oben hergestellte Lösung zugetropft. Der pH-Wert wird durch tropfenweise Zugabe von n/10 Natronlauge auf 7 gehalten.

Die Aufarbeitung erfolgt analog Beispiel 57.

Ausbeute: 5,45 g Natriumsalz (86%);

IR-Spektrum: 1770, 1660, 1610, 1545, 1510 $cm^{-1}$;

NMR-Spektrum (DMSO + $CD_3OD$) Signale bei ppm: 1,55 (6H), 4,05 (1H), 5,50 (q, 2H), 5,55 (1H), 6,85 (d, 2H), 7,4 (m, 4H), 7,85 (d, 2H), 8,40 (1H).

## Beispiel 64

D-α-[3-(2-p-Fluoranilino-4-hydroxy-5-pyrimidyl)-ureido]-benzylpenicillin-Natrium

Analog Beispiel 57, ausgehend von 3,71 g (0,01 Mol) Ampicillin-Natrium und dem Umsetzungsprodukt von 2,20 g (0,01 Mol) 5-Amino-2-p-fluoranilino-4-hydroxy-pyrimidin mit 1,05 g Phosgen und 1,35 ml Triäthylamin.

Ausbeute: 5,24 g (85%) Natriumsalz;

IR-Spektrum: 1765, 1660, 1610, 1550, 1510 $cm^{-1}$;

NMR-Spektrum (DMSO + $CD_3OD$): Signale bei ppm: 1,55 (6H), 4,05 (1H), 5,45 (q, 2H), 5,50 (1H), 6,90 (d, 2H), 7,55 (m, 7H), 8,35 (1H).

## Beispiel 65

D-α-[3-(2-m,p-Dichloranilino-4-hydroxy-5-pyrimidyl)-ureido]-p-hydroxybenzylpenicillin-Natrium

Dieses Penicillin wird analog Beispiel 57 hergestellt, indem man von 405 mg 5-Amino-2-m,p-dichloranilino-4-hydroxy-pyrimidin (0,0015 Mol), 155 mg Phosgen und 0,2 ml Triäthylamin ausgeht und mit 630 mg Amoxycillin-trihydrat umsetzt.

Ausbeute: 710 mg Natriumsalz (69%);

IR-Spektrum: 1770, 1655, 1610, 1545, 1510 $cm^{-1}$;

NMR-Spektrum ($DMSO+CD_3OD$): Signale bei ppm: 1,55 (6H), 4,05 (1H), 5,50 (q, 2H), 5,55 (1H), 6,85 (d, 2H), 7,5 (m, 5H), 8,5 (1H).

## Beispiel 66

D-α-[3-(2-o-Chloranilino-4-hydroxy-5-pyrimidyl)-ureido]-p-hydroxybenzylpenicillin-Natrium

Analog Beispiel 57, ausgehend von 710 mg 5-Amino-2-o-chloranilino-4-hydroxy-pyrimidin (0,003 Mol), 310 mg Phosgen, 300 mg Triäthylamin und 1,26 g Amoxycillin-trihydrat (0,003 Mol).
Ausbeute: 1,79 g Natriumsalz (91%);
IR-Spektrum: 1770, 1660, 1610, 1540, 1515 $cm^{-1}$;
NMR-Spektrum ($DMSO+CD_3OD$): Signale bei ppm: 1,55 (6H), 4,10 (1H), 5,45 (q, 2H), 5,55 (1H), 6,85 (d, 2H), 7,4 (m, 6H), 8,35 (1H).

## Beispiel 67

D-α-[3-(2-o-Chloranilino-4-hydroxy-5-pyrimidyl)-ureido]-benzylpenicillin-Natrium

Analog Beispiel 57, jedoch werden statt des Amoxycillins 4,03 g (0,01 Mol) Ampicillin-trihydrat mit 2,36 g (0,01 Mol) des dort genannten Pyrimidins umgesetzt.
Ausbeute: 5,35 g Natriumsalz (82%);
IR-Spektrum: 1770, 1655, 1610, 1535, 1510 $cm^{-1}$;
NMR-Spektrum ($DMSO+CD_3OD$): Signale bei ppm: 1,55 (6H), 4,05 (1H), 5,45 (q, 2H), 5,55 (1H), 7,45 (m, 9H), 8,35 (1H).

## Beispiel 68

D-α-[3-(2-m-Chloranilino-4-hydroxy-5-pyrimidyl)-ureido]-p-hydroxybenzylpenicillin-Natrium

Dieses Penicillin wird erhalten, wenn man in der in Beispiel 57 beschriebenen Weise 8,40 g Amoxycillin-trihydrat (0,02 Mol) und das Reaktionsprodukt von 4,75 g 5-Amino-2-m-chloranilino-4-hydroxy-pyrimidin (0,02 Mol) mit 2,05 g Phosgen miteinander reagieren läßt.
Ausbeute: 9,8 g Natriumsalz (75%);
IR-Spektrum: 1770, 1660, 1615, 1545, 1515 $cm^{-1}$;
NMR-Spektrum ($DMSO+CD_3OD$): Signale bei ppm: 1,55 (6H), 4,0 (1H), 5,45 (q, 2H), 5,55 (1H), 6,85 (d, 2H), 7,50 (m, 6H), 8,40 (1H).

## Beispiel 69

D-α-[3-(2-m-Chloranilino-4-hydroxy-5-pyrimidyl)-ureido]-benzylpenicillin-Natrium

Analog Beispiel 57, ausgehend von 2,82 g Ampicillin-trihydrat (0,007 Mol), 1,65 g (0,007 Mol) 5-Amino-2-m-chloranilino-4-hydroxy-pyrimidin und 700 mg Phosgen.
Ausbeute: 3,19 g Natriumsalz (72%);
IR-Spektrum: 1765, 1655, 1610, 1540, 1510 $cm^{-1}$;
NMR-Spektrum ($DMSO+CD_3OD$): Signale bei ppm: 1,55 (6H), 4,05 (1H), 5,50 (q, 2H), 5,55 (1H), 7,50 (m, 9H), 8,35 (1H).

## Beispiel 70

D-α-[3-(4-Hydroxy-2-p-trifluormethylanilino-5-pyrimidyl)-ureido]-p-hydroxybenzylpenicillin-Natrium

2,70 g (0,01 Mol) 5-Amino-4-hydroxy-2-p-trifluormethylanilinopyrimidin läßt man mit 1,05 g Phosgen und 1,35 ml Triäthylamin reagieren. Analog Beispiel 57 wird dann mit 4,20 g (0,01 Mol) Amoxycillin umgesetzt.
Ausbeute: 6,0 g Natriumsalz (88%);
IR-Spektrum: 1770, 1660, 1550, 1515 $cm^{-1}$;
NMR-Spektrum ($DMSO+CD_3OD$): Signale bei ppm: 1,55 (6H), 4,05 (1H), 5,45 (q, 2H), 5,50 (1H), 6,85 (d, 2H), 7,35 (m, 4H), 7,55 (d, 2H), 8,40 (1H).

Beispiel 71

D-α-[3-(4-Hydroxy-2-p-trifluormethylanilino-5-pyrimidyl)-ureido]-benzylpenicillin-Natrium

Analog Beispiel 57, ausgehend von 3,71 g Ampicillin-Natrium (0,01 Mol) und dem Umsetzungsprodukt von 2,70 g des Pyrimidins des Beispiels 70 (0,01 Mol) mit 1,05 g Phosgen.

Ausbeute: 4,77 g Natriumsalz (71%);

IR-Spektrum: 1765, 1655, 1610, 1550, 1515 $cm^{-1}$;

NMR-Spektrum (DMSO+$CD_3OD$): Signale bei ppm: 1,55 (6H), 4,10 (1H), 5,50 (q, 2H), 5,55 (1H), 7,55 (m, 9H), 8,35 (1H).

Beispiel 72

D-α-[3-(4-Hydroxy-2-p-methoxyanilino-5-pyrimidyl)-ureido]-p-hydroxybenzylpenicillin-Natrium

Dieses Penicillin wird analog Beispiel 57 synthetisiert, wobei man zunächst 1,16 g 5-Amino-4-hydroxy-2-p-methoxyanilino-pyrimidin (0,005 Mol) mit 500 mg Phosgen und 0,67 ml Triäthylamin reagieren läßt. Das entstandene Produkt wird analog Beispiel 57 mit 2,1 g Amoxycillin-trihydrat (0,005 Mol) umgesetzt. Die Aufarbeitung erfolgt so, daß man die entstandene Penicillinsäure bei pH 2,0 aus Wasser ausfällt, absaugt, mit Äther auswäscht und trocknet. Anschließend wird sie auf übliche Weise in das Natriumsalz überführt.

Ausbeute: 2,73 g (83%);

IR-Spektrum: 1770, 1660, 1610, 1550, 1510 $cm^{-1}$;

NMR-Spektrum (DMSO+$CD_3OD$): Signale bei ppm: 1,55 (6H), 3,8 (3H), 4,1 (1H), 5,45 (q, 2H), 5,55 (1H), 6,9 (m, 4H), 7,4 (d, 2H), 7,6 (d, 2H), 8,3 (1H).

Beispiel 73

D-α-[3-(4-Hydroxy-2-p-methoxyanilino-5-pyrimidyl)-ureido]-benzylpenicillin-Natrium

Dieses Penicillin wurde analog Beispiel 57 hergestellt. Man geht aus von 2,0 g Ampicillin-trihydrat (0,005 Mol) sowie dem Umsetzungsprodukt von 1,16 g des Pyrimidins des Beispiels 72 (0,005 Mol) mit 0,5 g Phosgen und 0,68 ml Triäthylamin.

Ausbeute: 2,37 g Natriumsalz (77%);

IR-Spektrum: 1770, 1660, 1605, 1540 $cm^{-1}$;

NMR-Spektrum (DMSO+$CD_3OD$) Signale bei ppm: 1,50 (6H), 3,75 (3H), 4,05 (1H), 5,40 (q, 2H), 5,5 (1H), 7,0 (2H), 7,5 (7H), 8,25 (1H).

Beispiel 74

D-α-[3-(2-p-Acetylaminoanilino-4-hydroxy-5-pyrimidyl)-ureido]-p-hydroxybenzylpenicillin-Natrium

Man geht aus von 780 mg (0,003 Mol) 2-p-Acetylamino-5-amino-4-hydroxy-pyrimidin, sowie 300 mg Phosgen und 0,41 ml Triäthylamin in absolutem Tetrahydrofuran. Das entstandene Produkt wird mit 1,26 g (0,003 Mol) Amoxycillin-trihydrat analog Beispiel 57 umgesetzt. Nach erfolgter Umsetzung wird Tetrahydrofuran im Vakuum abgezogen, die wäßrige Phase auf 50 ml verdünnt und zweimal bei pH 7,0 mit Essigester extrahiert. Dann wird die wäßrige Phase unter Eiskühlung mit verdünnter Salzsäure auf pH 2,0 gestellt. Das ausgefallene Produkt wird abgesaugt, mit Äther ausgewaschen und getrocknet. Auf übliche Weise wird das Natriumsalz hergestellt.

Ausbeute: 1,08 g Natriumsalz (54%);

IR-Spektrum: 1770, 1655, 1610, 1545, 1510 $cm^{-1}$;

NMR-Spektrum (DMSO+$CD_3OD$) Signale bei ppm: 1,55 (6H), 2,05 (3H), 4,0 (1H), 5,4 (q, 2H), 5,5 (1H), 6,8 (d, 2H), 7,3 (m, 4H), 7,6 (2H), 8,3 (1H).

Beispiel 75

D-α-[3-(2-p-Acetylaminoanilino-4-hydroxy-5-pyrimidyl)-ureido]-benzylpenicillin-Natrium

Dieses Penicillin wurde analog Beispiel 74 hergestellt. Man geht aus von 1,2 g Ampicillin-trihydrat (0,003 Mol) sowie dem Umsetzungsprodukt von 780 mg (0,003 Mol) des Pyrimidins des Beispiels 74 mit 300 mg Phosgen und 0,41 ml Triäthylamin.

Ausbeute: 950 mg Natriumsalz (51%);
IR-Spektrum: 1765, 1650, 1605, 1540, 1510 $cm^{-1}$;
NMR-Spektrum (DMSO + $CD_3OD$) Signale bei ppm: 1,5 (6H), 2,05 (3H), 4,05 (1H), 5,45 (3H), 7,5 (9H), 8,3 (1H).

Beispiel 76

D-α-[3-(2-o,p-Dichloranilino-4-hydroxy-5-pyrimidyl)-ureido]-p-hydroxybenzylpenicillin-Natrium

Analog Beispiel 57, ausgehend von dem Reaktionsprodukt von 810 mg (0,003 Mol) 5-Amino-2-o,p-dichloranilino-4-hydroxy-pyrimidin mit 300 mg Phosgen und 0,4 ml Triäthylamin sowie 1,26 g Amoxycillin (0,003 Mol).
Ausbeute: 1,40 g (68%) Natriumsalz;
IR-Spektrum: 1770, 1660, 1615, 1555, 1520 $cm^{-1}$;
NMR-Spektrum (DMSO + $CD_3OD$): Signale bei ppm: 1,55 (6H), 4,05 (1H), 5,45 (m, 3H), 6,85 (d, 2H), 7,50 (m, 5H), 8,40 (1H).

Beispiel 77

D-α-[3-(2-o,p-Dichloranilino-4-hydroxy-5-pyrimidyl)-ureido]-benzylpenicillin-Natrium

Analog Beispiel 57, ausgehend von 4,03 g Ampicillin-trihydrat (0,01 Mol) und dem Umsetzungsprodukt von 2,71 g (0,01 Mol) des Pyrimidins des Beispiels 76 mit 1,05 g Phosgen.
Ausbeute: 5,15 g Natriumsalz (77%);
IR-Spektrum: 1770, 1660, 1615, 1555, 1320 $cm^{-1}$;
NMR-Spektrum (DMSO + $CD_3OD$): Signale bei ppm: 1,55 (6H), 4,05 (1H), 5,45 (q, 2H), 5,55 (1H), 7,55 (m, 8H), 8,35 (1H).

Beispiel 78

D-α-[3-(2-p-Bromanilino-4-hydroxy-5-pyrimidyl)-ureido]-p-hydroxybenzylpenicillin-Natrium

Dieses Penicillin wurde analog Beispiel 57 hergestellt. Man geht aus von 2,1 g Amoxicillin-trihydrat (0,005 Mol) sowie dem Umsetzungsprodukt von 1,4 g 5-Amino-2-p-bromanilino-4-hydroxy-pyrimidin (0,005 Mol) mit 500 mg Phosgen und 0,67 ml Triäthylamin.
Ausbeute: 4,4 g Natriumsalz (63%);
IR-Spektrum: 1770, 1660, 1610, 1550, 1520 $cm^{-1}$;
NMR-Spektrum (DMSO + $CD_3OD$) Signale bei ppm: 1,5 (6H), 4,0 (1H), 5,45 (3H), 6,8 (2H), 7,35 (4H), 7,8 (2H), 8,35 (1H).

Beispiel 79

D-α-[3-(2-p-Äthylanilino-4-hydroxy-5-pyrimidyl)-ureido]-p-hydroxy-benzylpenicillin-Natrium

Dieses Penicillin wurde analog Beispiel 57 hergestellt. Man geht aus von 6,3 g Amoxicillin-trihydrat (0,015 Mol) sowie dem Umsetzungsprodukt von 3,45 g 5-Amino-2-p-äthylanilino-4-hydroxy-pyrimidin mit 1,5 g Phosgen und 2,05 ml Triäthylamin.
Ausbeute: 7,15 g Natriumsalz (94%);
IR-Spektrum: 1770, 1660, 1610, 1550 $cm^{-1}$;
NMR-Spektrum (DMSO + $CD_3OD$) Signale bei ppm: 1,2 (3H), 1,55 (6H), 2,5 (2H), 4,05 (1H), 5,45 (3H), 6,75 (2H), 7,5 (6H), 8,2 (1H).

Beispiel 80

D-α-[3-(4-Hydroxy-2-p-isopropylanilino-5-pyrimidyl)-ureido]-p-hydroxybenzylpenicillin-Natrium

Analog Beispiel 57, ausgehend von 1,22 g 5-Amino-4-hydroxy-2-p-isopropylanilino-pyrimidin (0,005 Mol), das zunächst mit 500 mg Phosgen und 0,67 ml Triäthylamin und dann mit 2,1 g (0,005 Mol) Amoxycillin umgesetzt wurde.
Ausbeute: 2,75 g Natriumsalz (83,5%);
IR-Spektrum: 1765, 1665, 1615, 1550, 1520 $cm^{-1}$;

NMR-Spektrum ($D_2O$): Signale bei ppm: 1,25 (d, 6H), 1,5 (d, 6H), 2,8 (m, 1H), 4,15 (1H), 5,45 (q, 2H), 5,55 (1H), 6,9 (d, 2H), 7,3 (m, 4H), 7,7 (d, 2H), 8,3 (1H).

## Beispiel 81

D-α-[(2-p-Dimethylaminoanilino-4-hydroxy-5-pyrimidyl)-ureido]-p-hydroxybenzylpenicillin-Natrium

Analog Beispiel 57, ausgehend von 6,3 g Amoxycillin-trihydrat (0,015 Mol) und dem Umsetzungsprodukt von 3,65 g 5-Amino-2-p-dimethylaminoanilino-4-hydroxy-pyrimidin (0,015 Mol) mit 1,55 g Phosgen und 2,05 ml Triäthylamin.

Ausbeute: 6,3 g Natriumsalz (64%);

IR-Spektrum: 1770, 1660, 1615, 1540, 1510 $cm^{-1}$;

NMR-Spektrum (DMSO + $CD_3OD$) Signale bei ppm: 1,55 (6H), 3,05 (6H), 4,05 (1H), 5,45 (q, 2H), 5,50 (1H), 6,9 (d, 2H), 7,25 (m, 4H), 7,60 (d, 2H), 8,35 (1H).

## Beispiel 82

D-α-[3-(2-p-Dimethylaminoanilino-4-hydroxy-5-pyrimidyl)-ureido]-benzylpenicillin-Natrium

Dieses Penicillin wurde analog Beispiel 57 hergestellt. Man geht aus von 1,92 g (0,00475 Mol) Ampicillin-trihydrat sowie dem Umsetzungsprodukt von 1,16 g (0,00475 Mol) des Pyrimidins des Beispiels 81 mit 500 mg Phosgen und 0,63 ml Triäthylamin.

Ausbeute: 2,05 g Natriumsalz (68%);

IR-Spektrum: 1770, 1660, 1615, 1540, 1520 $cm^{-1}$;

NMR-Spektrum (DMSO + $CD_3OD$): Signale bei ppm: 1,55 (6H), 3,1 (6H), 4,05 (1H), 5,35 (q, 2H), 5,45 (1H), 7,45 (6H), 7,6 (2H), 8,3 (1H).

## Beispiel 83

D-α-[3-(2-p-Aminoanilino-4-hydroxy-5-pyrimidyl)-ureido]-p-hydroxy-benzylpenicillin-Natrium

Dieses Penicillin wurde analog Beispiel 57 hergestellt. Man geht aus von 1,0 g (0,00237 Mol) Amoxicillin-trihydrat sowie dem Umsetzungsprodukt von 515 mg (0,00237 Mol) 5-Amino-2-p-aminoanilino-4-hydroxy-pyrimidin mit 240 mg Phosgen und 0,31 ml Triäthylamin.

Ausbeute: 670 mg Natriumsalz (43,5%);

IR-Spektrum: 1770, 1650, 1600, 1550 $cm^{-1}$;

NMR-Spektrum (DMSO + $CD_3OD$) Signale bei ppm: 1,55 (6H), 4,05 (1H), 5,35 (2H), 5,45 (1H), 6,85 (2H), 2,75 (4H), 7,60 (2H), 8,35 (1H).

## Beispiel 84

D-α-[3-(4-Hydroxy-2-p-methylanilino-5-pyrimidyl)-ureido]-p-hydroxybenzylpenicillin-Natrium

Dieses Penicillin wird hergestellt, indem man von 1,68 g (0,004 Mol) Amoxycillin-trihydrat und dem Umsetzungsprodukt von 860 mg (0,004 Mol) 5-Amino-4-hydroxy-2-p-methylanilino-pyrimidin mit 405 mg Phosgen und 0,54 ml Triäthylamin ausgeht. Die Aufarbeitung erfolgt in der Weise, daß man zunächst bei pH 7,0 die wäßrige Phase zweimal mit Essigsäureäthylester ausschüttelt, dann mit Essigsäureäthylester (100 ml) überschichtet und unter Kühlen mit verdünnter Salzsäure auf pH 2,0 stellt. Das ausgefallene Produkt wird abgesaugt, mit Äther gewaschen und getrocknet. Die organische Phase wird abgetrennt, die wäßrige Phase wird zweimal mit Essigsäureäthylester ausgeschüttelt, die organischen Phasen vereinigt, getrocknet und das Lösemittel im Vakuum abgezogen. Das erhaltene Produkt ist laut DC (n-Butanol—$CH_3COOH$—$H_2O$ = 60 : 15 : 25) mit dem zunächst ausgefallenen Produkt identisch. Beide Fraktionen werden zusammen in das Natriumsalz überführt.

Ausbeute: 1,89 g (74,5%);

IR-Spektrum: 1770, 1655, 1605, 1550, 1510 $cm^{-1}$;

NMR-Spektrum (DMSO + $CD_3OD$): Signale bei ppm: 1,55 (6H), 4,05 (1H), 5,5 (m, 3H), 6,85 (d, 2H), 7,3 (m, 4H), 7,7 (d, 2H), 8,3 (1H).

## Beispiel 85

### D-α-[3-(4-Hydroxy-2-p-methylanilino-5-pyrimidyl)-ureido]-benzylpenicillin-Natrium

Herstellung analog Beispiel 57, indem man von 3,71 g (0,01 Mol) Ampicillin-Natrium sowie dem Umsetzungsprodukt von 2,15 g (0,01 Mol) des Pyrimidins des Beispiels 84 mit 1,05 g Phosgen ausgeht. Bei der Aufarbeitung wird das entstandene Penicillin bei pH 2,0 aus Wasser ausgefällt, abgesaugt, mit Wasser und Äther gewaschen und getrocknet.

Ausbeute: 4,3 g Natriumsalz (69%);
IR-Spektrum: 1765, 1655, 1610, 1550, 1510 $cm^{-1}$;
NMR-Spektrum (DMSO+$CD_3OD$): Signale bei ppm: 1,55 (6H), 2,3 (3H), 4,05 (1H), 5,45 (q, 2H), 5,55 (1H), 7,35 (m, 7H), 7,7 (d, 2H), 8,35 (1H).

## Beispiel 86

### D-α-[3-(2-{p-Chlor-m-trifluormethylanilino}-4-hydroxy-5-pyrimidyl)-ureido]-p-hydroxybenzylpenicillin-Natrium

Herstellung analog Beispiel 57. Man geht aus von 2,1 g Amoxycillin-trihydrat (0,005 Mol) und dem Umsetzungsprodukt von 1,52 g (0,005 Mol) 5-Amino-2-(p-chlor-m-trifluormethyl-anilino)-4-hydroxypyrimidin mit 505 mg Phosgen und 0,67 ml Triäthylamin.

Ausbeute: 2,74 g Natriumsalz (76%);
IR-Spektrum: 1765, 1650, 1610, 1545, 1505 $cm^{-1}$;
NMR-Spektrum (DMSO+$CD_3OD$): Signale bei ppm: 1,55 (6H), 4,05 (1H), 5,45 (q, 2H), 5,55 (1H), 6,85 (d, 2H), 7,5 (m, 5H), 8,40 (1H).

## Beispiel 87

### D-α-[3-(4-Hydroxy-2-p-nitroanilino-5-pyrimidyl)-ureido]-p-hydroxybenzylpenicillin-Natrium

Dieses Penicillin wird hergestellt, indem man von 630 mg Amoxycillin-trihydrat (0,0015 Mol) und dem Umsetzungsprodukt von 365 mg 5-Amino-4-hydroxy-2-nitroanilino-pyrimidin (0,0015 Mol) mit 150 mg Phosgen und 0,20 ml Triäthylamin (in 1 l absolutem Tetrahydrofuran) ausgeht.

Ausbeute: 440 mg Natriumsalz (45%);
IR-Spektrum: 1770, 1660, 1615, 1550, 1510 $cm^{-1}$;
NMR-Spektrum (DMSO+$CD_3OD$): Signale bei ppm: 1,55 (6H), 4,10 (1H), 5,50 (q, 2H), 5,60 (1H), 6,90 (d, 2H), 7,3 (d, 2H), 7,6 (d, 2H), 7,90 (d, 2H), 8,45 (1H).

## Beispiel 88

### D-α-[3-(2-{p-Amino-m,m-dichlor-anilino}-4-hydroxy-5-pyrimidyl)-ureido]-p-hydroxybenzylpenicillin-Natrium

Herstellung analog Beispiel 57. Man geht aus von 1,26 g Amoxycillin-trihydrat (0,003 Mol) und dem Umsetzungsprodukt von 860 mg (0,003 Mol) 5-Amino-2-(p-amino-m,m-dichlor-anilino)-4-hydroxy-pyrimidin mit 300 mg Phosgen und 0,41 ml Triäthylamin.

Ausbeute: 1,22 g Natriumsalz (58%);
IR-Spektrum: 1765, 1660, 1610, 1550, 1510 $cm^{-1}$;
NMR-Spektrum (DMSO+$CD_3OD$): Signale bei ppm: 1,55 (6H), 4,0 (1H), 5,4 (q, 2H), 5,5 (1H), 6,85 (d, 2H), 7,4 (d, 2H), 7,8 (2H), 8,35 (1H).

## Beispiel 89

### D-α-[3-(2-p-Hydroxyanilino-4-hydroxy-5-pyrimidyl)-ureido]-p-hydroxybenzylpenicillin-Natrium

Dieses Penicillin wird analog Beispiel 57 synthetisiert, indem man von 840 mg (0,002 Mol) Amoxycillin-trihydrat sowie dem Reaktionsprodukt von 440 mg 5-Amino-2-p-hydroxyanilino-4-hydroxypyrimidin (0,002 Mol) mit 205 mg Phosgen und 0,27 ml Triäthylamin ausgeht.

Ausbeute: 755 mg (60%);
IR-Spektrum: 1765, 1660, 1610, 1550, 1510 $cm^{-1}$;

NMR-Spektrum ($DMSO+CD_3OD$) Signale bei ppm: 1,55 (6H), 4,0 (1H), 5,4 (g, 2H), 5,5 (1H), 6,85 (m, 4H), 7,4 (m, 4H), 8,25 (1H).

Beispiel 90

D-α-[3-(2-p-Hydroxyanilino-4-hydroxy-5-pyrimidyl)-ureido]-benzylpenicillin-Natrium

Herstellung analog Beispiel 57, wenn man von 2,0 g (0,005 Mol) Ampicillin-trihydrat und dem Umsetzungsprodukt von 1,08 g (0,005 Mol) des Pyrimidins des Beispiels 89 mit 505 mg Phosgen und 0,67 ml Triäthylamin ausgeht.
Ausbeute: 1,48 g Natriumsalz (48%);
IR-Spektrum: 1765, 1660, 1610, 1550, 1510 $cm^{-1}$;
NMR-Spektrum ($DMSO+CD_3OD$) Signale bei ppm: 1,55 (6H), 4,05 (1H), 5,45 (q, 2H), 5,55 (1H), 6,85 (d, 2H), 7,5 (m, 7H), 8,30 (1H).

Beispiel 91

D-α-[3-(4-Hydroxy-2-p-methylaminoanilino-5-pyrimidyl)-ureido]-p-hydroxy-benzylpenicillin-Natrium

Dieses Penicillin wurde analog Beispiel 57 hergestellt. Man geht aus von 1,5 g Amoxicillin-trihydrat (0,00357 Mol) sowie dem Umsetzungsprodukt von 830 mg 5-Amino-4-hydroxy-2-methylaminoanilino-pyrimidin mit 370 mg Phosgen und 0,5 ml Triäthylamin.
Ausbeute: 1,14 g Natriumsalz (51%);
IR-Spektrum: 1770, 1660, 1615, 1550 $cm^{-1}$;
NMR-Spektrum ($DMSO+CD_3OD$) Signale bei ppm: 1,55 (6H), 3,1 (3H), 4,05 (1H), 5,45 (3H), 6,85 (2H), 7,45 (4H), 7,6 (2H), 8,3 (1H).

Beispiel 92

D-α-[3-(4-Hydroxy-2-p-methylsulfonylanilino-pyrimidyl)-ureido]-p-hydroxy-benzylpenicillin-Natrium

Dieses Penicillin wurde analog Beispiel 57 hergestellt. Man geht aus von 3,5 g Amoxicillin-trihydrat (0,0083 Mol) sowie dem Umsetzungsprodukt von 2,35 (0,0085 Mol) 5-Amino-4-hydroxy-2-methylsulfonyl-pyrimidin mit 850 mg Phosgen und 1,16 ml Triäthylamin.
Ausbeute: 3,95 g Natriumsalz (69,5%);
IR-Spektrum: 1770, 1650, 1600, 1535 $cm^{-1}$;
NMR-Spektrum ($DMSO+CD_3OD$) Signale bei ppm: 1,55 (6H), 2,9 (3H), 4,05 (1H), 5,45 (3H), 6,75 (2H), 7,35 (4H), 7,65 (2H), 8,30 (1H).

Beispiel 93

D-α-[3-(2-p-Acetylanilino-4-hydroxy-5-pyrimidyl)-ureido]-p-hydroxy-benzylpenicillin-Natrium

Dieses Penicillin wurde analog Beispiel 57 hergestellt. Man geht aus von 4,2 g (0,01 Mol) Amoxicillin-trihydrat sowie dem Umsetzungsprodukt von 2,44 g (0,01 Mol) 2-p-Acetylanilino-5-amino-4-hydroxy-pyrimidin mit 1,0 g Phosgen und 1,37 ml Triäthylamin.
Ausbeute: 3,83 g Natriumsalz (58%);
IR-Spektrum: 1770, 1660, 1610, 1540 $cm^{-1}$;
NMR-Spektrum ($DMSO+CD_3OD$) Signale bei ppm: 1,55 (6H), 2,1 (3H), 4,05 (1H), 5,30 (q, 2H), 5,45 (1H), 6,8 (2H), 7,35 (4H), 7,6 (2H), 8,35 (1H).

Beispiel 94

D-α-[3-(2-p-Carbamoylanilino-4-hydroxy-5-pyrimidyl)-ureido]-p-hydroxy-benzylpenicillin-Natrium

Dieses Penicillin wurde analog Beispiel 6 hergestellt. Man geht aus von 2,1 g Amoxicillin-trihydrat (0,005 Mol) sowie dem Umsetzungsprodukt von 1,23 g (0,005 Mol) 5-Amino-2-p-Carbamoylanilino-4-hydroxy-pyrimidin mit 500 mg Phosgen und 0,67 ml Triäthylamin.
Ausbeute: 2,45 g Natriumsalz (74%);
IR-Spektrum: 1770, 1650, 1600, 1530 $cm^{-1}$;

NMR-Spektrum (DMSO+$CD_3OD$) Signale bei ppm: 1,55 (6H), 4,1 (1H), 5,3 (g, 2H), 5,4 (1H), 6,8 (2H), 7,3 (4H), 7,55 (2H), 8,25 (1H).

## Beispiel 95

D-α-[3-(4-Hydroxy-2-{p-3'-methylureido-anilino}-5-pyrimidyl)-ureido]-p-hydroxy-benzylpenicillin-Natrium

Dieses Penicillin wurde analog Beispiel 6 hergestellt. Man geht aus von 3,36 g (0,008 Mol) Amoxicillin-trihydrat sowie dem Umsetzungsprodukt von 2,2 g (0,008 Mol) 5-Amino-4-hydroxy-2-(p-3'-methylureido-anilino)-pyrimidin mit 800 mg Phosgen und 1,1 ml Triäthylamin.
Ausbeute: 2,55 g Natriumsalz (46%);
IR-Spektrum: 1770, 1660, 1630, 1600, 1540 $cm^{-1}$;
NMR-Spektrum (DMSO+$CD_3OD$) Signale bei ppm: 1,55 (6H), 2,8 (3H), 4,05 (1H), 5,35 (q, 2H), 5,45 (1H), 6,85 (2H), 7,35 (4H), 7,65 (2H), 8,35 (1H).

## Beispiel 96

D-α-[3-(2-p-Cyananilino-4-hydroxy-5-pyrimidyl)-ureido]-p-hydroxy-benzylpenicillin-Natrium

Dieses Penicillin wurde analog Beispiel 57 hergestellt. Man geht aus von 600 mg Amoxicillin-trihydrat (0,00153 Mol) sowie dem Umsetzungsprodukt von 350 mg (0,0016 Mol) 5-Amino-2-p-cyananilino-4-hydroxy-pyrimidin mit 160 mg Phosgen und 0,22 ml Triäthylamin.
Ausbeute: 490 mg Natriumsalz (50%);
IR-Spektrum: 2215, 1770, 1650, 1600, 1550 $cm^{-1}$;
NMR-Spektrum (DMSO+$CD_3OD$) Signale bei ppm: 1,50 (6H), 4,0 (1H), 5,35 (2H), 5,45 (1H), 6,85 (2H), 7,4 (4H), 7,75 (2H), 8,40 (1H).

## Beispiel 97

D-α-[3-(4-Hydroxy-2-m,p-dimethoxy-anilino-5-pyrimidyl)-ureido]-p-hydroxy-benzylpenicillin-Natrium

Dieses Penicillin wurde analog Beispiel 57 hergestellt. Man geht aus von 4,2 g Amoxicillin-trihydrat (0,01 Mol) sowie dem Umsetzungsprodukt von 2,62 g 5-Amino-4-hydroxy-2-m,p-dimethoxyanilino-pyrimidin mit 1,0 g Phosgen und 1,37 ml Triäthylamin.
Ausbeute: 5,45 g Natriumsalz (81%);
IR-Spektrum: 1770, 1650, 1610, 1535 $cm^{-1}$;
NMR-Spektrum (DMSO+$CD_3OD$) Signale bei ppm: 1,55 (6H), 3,8 (3H), 3,85 (3H), 4,05 (1H), 5,5 (3H), 6,8—7,7 (7H), 8,3 (1H).

## Beispiel 98

D-α-[3-(2-m,m-Dichloranilino-4-hydroxy-5-pyrimidyl)-ureido]-p-hydroxy-benzylpenicillin-Natrium

Dieses Penicillin wurde analog Beispiel 57 hergestellt. Man geht aus von 1,0 g Amoxicillin-trihydrat (0,00237 Mol) sowie dem Umsetzungsprodukt von 670 mg 5-Amino-2-m,m-dichloranilino-4-hydroxy-pyrimidin (0,0024 Mol) mit 240 mg Phosgen und 0,34 ml Triäthylamin.
Ausbeute: 1,55 g Natriumsalz (78,5%);
IR-Spektrum: 1770, 1660, 1610, 1600, 1540 $cm^{-1}$;
NMR-Spektrum (DMSO+$CD_3OD$) Signale bei ppm: 1,55 (6H), 4,1 (1H), 5,55 (3H), 6,9 (2H), 7,1 (1H), 7,4 (2H), 8,0 (2H), 8,5 (1H).

## Beispiel 99

D-α-[3-(2-m,m-Dichloranilino-4-hydroxy-5-pyrimidyl)-ureido]-benzylpenicillin-Natrium

Dieses Penicillin wurde analog Beispiel 57 hergestellt. Man geht aus von 1,34 g (0,0036 Mol) Ampicillin-Natrium sowie dem Umsetzungsprodukt von 1,02 g (0,0036 Mol) des Amins des Beispiels 98 mit 360 mg Phosgen und 0,5 ml Triäthylamin.
Ausbeute: 1,23 g Natriumsalz (59%);
IR-Spektrum: 1770, 1660, 1610, 1530 $cm^{-1}$;

NMR-Spektrum ($DMSO+CD_3OD$) Signale bei ppm: 1,55 (6H), 4,05 (1H), 5,4 (2H), 5,55 (1H), 7,1 (1H), 7,4 (5H), 7,9 (2H), 8,45 (1H).

## Beispiel 100

D-α-[3-(2-m,p-Dihydroxyanilino-4-hydroxy-5-pyrimidyl)-ureido]-p-hydroxy-benzylpenicillin-Natrium

Dieses Penicillin wurde analog Beispiel 57 hergestellt. Man geht aus von 840 mg (0,002 Mol) Amoxycillin-trihydrat sowie dem Umsetzungsprodukt von 460 mg (0,002 Mol) 5-Amino-2-m,p-dihydroxyanilino-4-hydroxy-pyrimidin mit 200 mg Phosgen und 0,27 ml Triäthylamin.
Ausbeute: 790 mg Natriumsalz (60%);
IR-Spektrum: 1765, 1660, 1610, 1545 $cm^{-1}$;
NMR-Spektrum ($DMSO+CD_3OD$) Signale bei ppm: 1,55 (6H), 4,05 (1H), 5,4 (q, 2H), 5,5 (1H), 6,85—7,5 (m, 7H), 8,25 (1H).

## Beispiel 101

D-α-[3-(2-m,p-Dihydroxyanilino-4-hydroxy-5-pyrimidyl)-ureido]-benzylpenicillin-Natrium

Dieses Penicillin wurde analog Beispiel 100 hergestellt. Man geht aus von 2,0 g Ampicillin-Natrium (0,005 Mol) sowie dem Umsetzungsprodukt von 1,15 g (0,005 Mol) des Pyrimidins des Beispiels 100 mit 0,5 g Phosgen und 0,68 ml Triäthylamin.
Ausbeute: 1,59 g Natriumsalz (52%);
IR-Spektrum: 1765, 1650, 1600, 1545 $cm^{-1}$;
NMR-Spektrum ($DMSO+CD_3OD$) Signale bei ppm: 1,5 (6H), 4,0 (1H), 5,4 (q, 2H), 5,5 (1H), 6,85—7,6 (m, 8H), 8,2 (1H).

## Beispiel 102

D-α-[3-(4-Hydroxy-2-p-methylsulfinylanilino-5-pyrimidyl)-ureido]-p-hydroxy-benzylpenicillin-Natrium

Dieses Penicillin wurde analog Beispiel 57 hergestellt. Man geht aus von 2,1 g Amoxycillin-trihydrat (0,005 Mol) sowie dem Umsetzungsprodukt von 1,32 g 5-Amino-4-hydroxy-2-p-methylsulfinylanilino-pyrimidin mit 500 mg Phosgen und 0,68 ml Triäthylamin.
Ausbeute: 2,1 g Natriumsalz (65%);
IR-Spektrum: 1770, 1655, 1610, 1545 $cm^{-1}$;
NMR-Spektrum ($DMSO+CD_3OD$) Signale bei ppm: 1,55 (6H), 2,75 (3H), 4,05 (1H), 5,5 (3H), 6,8—7,9 (m, 8H), 8,25 (1H).

## Beispiel 103

D-α-[3-(2-p-Sulfamoylanilino-4-hydroxy-5-pyrimidinyl)-ureido]-p-hydroxy-benzylpenicillin-Natrium

Dieses Penicillin wurde analog Beispiel 57 hergestellt. Man geht aus von 4,2 g Amoxycillin-trihydrat sowie dem Umsetzungsprodukt von 2,8 g (0,01 Mol) 5-Amino-3-p-sulfamoylanilino-4-hydroxy-pyrimidin mit 1,0 g Phosgen und 1,35 ml Triäthylamin.
Ausbeute: 4,25 g Natriumsalz (62%);
IR-Spektrum: 1765, 1660, 1610, 1540 $cm^{-1}$;
NMR-Spektrum ($DMSO+CD_3OD$) Signale bei ppm: 1,55 (6H), 4,05 (1H), 5,4 (q, 2H), 5,5 (1H), 6,8 (2H), 7,3 (2H), 7,6 (2H), 7,95 (2H), 8,3 (1H).

## Beispiel 104

D-α-[3-(2-Benzylamino-4-hydroxy-5-pyrimidyl)-ureido]-p-hydroxy-benzylpenicillin-Natrium

Dieses Penicillin wurde analog Beispiel 57 hergestellt. Man geht aus von 2,1 g Amoxicillin-trihydrat (0,005 Mol) sowie dem Umsetzungsprodukt von 1,08 g (0,005 Mol) 5-Amino-2-benzylamino-4-hydroxy-pyrimidin mit 500 mg Phosgen und 0,67 ml Triäthylamin.
Ausbeute: 2,03 g Natriumsalz (63,5%);
IR-Spektrum: 1770, 1670, 1600, 1530 $cm^{-1}$;

NMR-Spektrum (DMSO+$CD_3OD$) Signale bei ppm: 1,5 (6H), 4,0 (1H), 4,3 (2H), 5,5 (3H), 6,8 (2H), 7,4 (7H), 8,2 (1H).

Beispiel 105

D-α-[3-(2-p-Chlorbenzylamino-4-hydroxy-5-pyrimidyl)-ureido]-p-hydroxy-benzylpenicillin-Natrium

Dieses Penicillin wurde analog Beispiel 57 hergestellt. Man geht aus von 4,2 g Amoxicillin-trihydrat (0,01 Mol) sowie dem Umsetzungsprodukt von 2,51 g (0,01 Mol) 5-Amino-2-p-chlorbenzylamino-4-hydroxy-pyrimidin mit 1,0 g Phosgen und 1,37 ml Triäthylamin.
Ausbeute: 5,52 g Natriumsalz (82%);
IR-Spektrum: 1770, 1650, 1610, 1530 $cm^{-1}$;
NMR-Spektrum (DMSO) Signale bei ppm: 1,55 (6H), 4,0 (1H), 4,4 (2H), 5,4 (q, 2H), 5,5 (1H), 6,8 (2H), 7,4 (4H), 7,6 (2H), 8,2 (1H).
Analog wurden hergestellt:
D-α-[3-(2-Benzylamino-4-hydroxy-5-pyrimidyl)-ureido]-m,p-dihydroxy-benzylpenicillin-Natrium
Ausbeute: 58%;
IR-Spektrum: 1760, 1660, 1600 $cm^{-1}$;
NMR-Spektrum (DMSO+$CD_3OD$) Signale bei ppm: 1,55 (d, 6H), 4,05 (s, 1H), 4,45 (breites s, 2H), 5,45 (m, 3H), 6,80 (d, 2H), 7,10 (m, 1H), 7,45 (m, 5H), 8,1 (s, 1H).
D-α-[3-(4-Hydroxy-2-p-hydroxybenzylamino-5-pyrimidyl)-ureido]-m,p-dihydroxy-benzylpenicillin-Natrium
Ausbeute: 54%;
IR-Spektrum: 1760, 1655, 1610 $cm^{-1}$;
NMR-Spektrum (DMSO+$CD_3OD$) Signale bei ppm: 1,55 (d, 6H), 4,05 (s, 1H), 4,25 (s, 2H), 5,50 (m, 3H), 6,70 (m, 4H), 7,1—7,4 (m, 3H), 8,10 (s, 1H).

Beispiel 106

D-α-[3-(2-p-Chloranilino-4-hydroxy-5-pyrimidyl)-ureido]-benzylpenicillin-Natrium

Zu einer Suspension von 2,3 g (0,005 Mol) D-α-[(2-p-Chloranilino-4-hydroxy-5-pyrimidyl)-ureido]-phenylglycin-Natriumsalz in 25 ml wasserfreiem Aceton gibt man 10 mg N-Methylmorpholin. Man kühlt auf −20° bis −15°C ab, und tropft bei dieser Temperatur eine Lösung von 550 mg Chlorameisensäureäthylester (0,005 Mol) in 10 ml wasserfreiem Aceton zu. Man rührt eine Stunde bei −20°C nach. Anschließend wird bei dieser Temperatur eine Lösung von 1,6 g des Triäthylammoniumsalzes der 6-Aminopenicillansäure in 10 ml wasserfreiem Methylenchlorid zugetropft. Man rührt 1 Stunde bei −20°C, 1 Stunde bei 0°C und eine Stunde bei Raumtemperatur nach. Das organische Lösungsmittel wird dann im Vakuum abgezogen und der Rückstand in einem Gemisch von 40 ml Wasser und 60 ml Essigsäureäthylester bei pH 7,0 gelöst. Die wäßrige Phase wird abgetrennt, mit 100 ml Essigsäureäthylester überschichtet und unter Eiskühlung auf pH 2,0 (mit verdünnter Salzsäure) gestellt. Die organische Phase wird abgetrennt, getrocknet und das Lösungsmittel im Vakuum entfernt. Mit Natriumäthylhexanoat wird das Natriumsalz hergestellt.
Ausbeute: 2,05 g (61%);
IR-Spektrum: 1770, 1660, 1610, 1545, 1515 $cm^{-1}$;
NMR-Spektrum (DMSO+$CD_3OD$) Signale bei ppm: 1,55 (6H), 4,1 (1H), 5,5 (q, 2H), 5,55 (1H), 7,5 (m, 5H), 7,9 (d, 2H), 8,45 (1H).

Beispiel 107

D-α-[3-(2-p-Acetylaminoanilino-4-hydroxy-5-pyrimidyl)-ureido]-m,p-dihydroxy-benzylpenicillin-Natrium

2,16 g (0,01 Mol) wasserfreie 6-Aminopenicillansäure werden mit 2,5 ml N,O-(Bistrimethylsilyl)-acetamid bei Raumtemperatur in trockenem Dimethylformamid silyliert. Die entstandene Lösung wird bei −10°C zu einer bei −20°C bereiteten Lösung (siehe auch Beispiel 106) von 4,9 g (0,01 Mol) D-α-[3-(2-p-Acetylaminoanilino-4-hydroxy-5-pyrimidyl)-ureido]-m,p-dihydroxy-phenylglycin, 1,1 g Chlorameisensäureäthylester und 1,05 g N-Methylmorpholin getropft. Weitere Umsetzung und Aufarbeitung siehe Beispiel 106.
Ausbeute: 3,54 g Natriumsalz (49%);
IR-Spektrum: 1770, 1650, 1610, 1545, 1510 $cm^{-1}$;
NMR-Spektrum (DMSO+$CD_3OD$) Signale bei ppm: 1,55 (6H), 2,10 (3H), 4,05 (1H), 5,45 (q, 2H), 5,55 (1H), 7,3 (m, 5H), 7,6 (d, 2H), 8,30 (1H).

Beispiel 108

D-α-[3-(2-Acetylamino-4-hydroxy-5-pyrimidyl)-ureido]-benzylpenicillin-Natrium

2,0 g (0,00125 Mol) 5-Amino-4-hydroxy-2-acetylamino-pyrimidin (erhalten durch katalytische Hydrierung der entsprechenden Nitroverbindung wie in JACS 1964, S. 5668, beschrieben) werden in absolutem Tetrahydrofuran gelöst. Bei 0°C werden 1,2 g Triäthylamin zugegeben und dieses Gemisch anschließend zu 1,3 g Phosgen, gelöst in Tetrahydrofuran, zugetropft. Es wird 30 Min. bei 0°C nachgerührt. Überschüssiges Phosgen wird mit Stickstoff abgeblasen und das Gemisch auf 50 ml konzentriert. Dieses Gemisch wird ohne Abfiltrieren des Triäthylaminhydrochlorids eingesetzt. 2,4 g Ampicillin-trihydrat (6 mMol) werden in 50 ml 80%igem, wäßrigem Tetrahydrofuran suspendiert. Dazu gibt man unter Eiskühlung und Rühren soviel Triäthylamin, bis das Ampicillin bei etwa pH 8 eben gelöst ist. Zu dieser Lösung wird das obige Gemisch zugetropft, wobei der pH der Mischung durch Zugabe von Triäthylamin bei etwa 7,5 gehalten wird. Man rührt so lange nach, bis zur Aufrechterhaltung dieses pH-Wertes keine Base mehr zugegeben werden muß. Dann wird mit 50 ml Wasser verdünnt, der pH-Wert auf 7,0 eingestellt und das Tetrahydrofuran am Rotationsverdampfer entfernt. Die verbleibende wäßrige Lösung wird bei pH 7,0 einmal mit Essigester gewaschen, dann mit ca. 200 ml Essigester überschichtet und unter Rühren und Kühlung vorsichtig mit 2 n Salzsäure auf pH 2,0 gebracht. Die organische Phase wird abgetrennt, etwas unlösliches Produkt abfiltriert und die wäßrige Phase nochmals mit Essigester ausgeschüttelt. Die vereinigten Essigesterextrakte werden mit gesättigter Kochsalzlösung gewaschen und dann über Natriumsulfat getrocknet.

Nach Entfernen des Lösungsmittels verbleibt ein farbloser Rückstand, der in 30 ml absolutem Methanol gelöst und mit 100 ml absolutem Äther verdünnt wird. Durch Zugeben einer Lösung von Natrium-2-äthylhexanoat in Äther wird das Natrium-Salz ausgefällt.

Ausbeute: 1,2 g (34%);
Rf: 0,57;
IR-Spektrum: 1770, 1660, 1610, 1525 $cm^{-1}$;
NMR-Spektrum: ($D_2O$) Signale bei ppm: 1,4 (3H), 1,5 (3H), 2,3 (3H), 4,2 (1H), 5,3—5,6 (3H), 7,5 (5H), 8,2 (1H).

Beispiel 109

D-α-[3-(2-Acetylamino-4-hydroxy-5-pyrimidyl)-ureido]-p-hydroxy-benzylpenicillin-Natrium

3,36 g 2-Acetylamino-5-amino-4-hydroxy-pyrimidin (0,02 Mol) werden eine Stunde lang mit 10 ml Hexamethyldisilazan auf 80°C erhitzt. Das nach Entfernen des überschüssigen Silylierungsmittels zurückbleibende Festprodukt wird in 20 ml absolutem Tetrahydrofuran gelöst, mit 2,0 g Triäthylamin versetzt und unter Eiskühlung zu einer Lösung von 2,1 g Phosgen in 30 ml absolutem Tetrahydrofuran getropft. Unter Stickstoff wird vom entstandenen Triäthylaminhydrochlorid abgesaugt.

Diese Lösung wird bei 0°C zu einer Lösung, die man aus 8,4 g Amoxycillin-trihydrat (0,02 Mol) in 80% Tetrahydrofuran mit Triäthylamin bei pH 7,8 hergestellt hat, getropft. Dabei wird der pH-Wert durch Zugabe von Triäthylamin bei etwa pH 7,5 gehalten. Die weitere Aufarbeitung erfolgt wie im letzten Beispiel angegeben.

Ausbeute: 5,7 g (48%) Natriumsalz;
Rf: 0,54;
IR-Spektrum: 1760, 1650, 1600, 1510 $cm^{-1}$;
NMR-Spektrum ($D_2O$) in ppm: 1,45 (3H), 1,55 (3H), 2,3 (3H), 4,25 (1H), 5,3 (1H), 5,5 (2H), 7,2 (4H), 8,3 (1H).

Beispiel 110

D-α-[3-(4-Hydroxy-2-propionylamino-5-pyrimidyl)-ureido]-benzylpenicillin-Natrium

Aus 2,4 g Ampicillin-Trihydrat (0,006 Mol) und dem Umsetzungsprodukt von 1,1 g 5-Amino-4-hydroxy-2-propionylamino-pyrimidin (0,006 Mol), 600 mg Phosgen und 600 mg Triäthylamin in Tetrahydrofuran.

Ausbeute: 2,55 g (75%).
Rf: 0,66.
IR-Spektrum: 1770, 1660, 1610, 1520 $cm^{-1}$;
NMR-Signale ($CD_3OD$) in ppm: 1,2—1,5 (t, 3H), 1,5 (3H), 1,6 (3H), 2,5 (q, 2H), 4,2 (1H), 5,6 (m, 3H), 7,5 (5H), 8,4 (1H).

## Beispiel 111

D-α-[3-(4-Hydroxy-2-propionylamino-5-pyrimidyl)-ureido]-p-hydroxy-benzylpenicillin

2,52 g Amoxycillin-trihydrat (0,006 Mol) werden in 50 ml 80% wäßrigem Tetrahydrofuran suspendiert. Dazu fügt man unter Eiskühlung und Rühren soviel Triäthylamin bis das Amoxicillin bei etwa pH 8,5 eben gelöst ist.

1,1 g 5-Amino-4-hydroxy-2-propionylamino-pyrimidin werden (0,006 Mol) in Tetrahydrofuran unter Eiskühlung mit 0,6 g Phosgen und 0,6 g Triäthylamin behandelt. Das entstehende Gemisch wird auf 40 ml eingeengt und unter Eiskühlung zu obiger Lösung getropft. Dabei wird durch Hinzufügen von Triäthylamin ständig ein pH-Wert von 7,5 aufrechterhalten. Man rührt so lange nach, bis der pH-Wert konstant bleibt.

Die Aufarbeitung erfolgt analog Beispiel 108.

Ausbeute: 1,7 g (46%).
Rf: 0,67;
IR-Spektrum: 1765, 1650, 1610, 1510 $cm^{-1}$;
NMR-Signale ($D_2O$) in ppm: 1,1—1,3 (t, 3H), 1,4 (1H), 1,5 (1H), 2,5 (2H), 4,2 (1H), 5,2 (1H), 5,5 (2H), 7,1 (4H), 8,2 (1H).

## Beispiel 112

D-α-[3-(4-Hydroxy-2-isobutyrylamino-5-pyrimidyl)-ureido]-benzylpenicillin-Natrium

Aus 2,4 g Ampicillintrihydrat und dem Umsetzungsprodukt von 1,17 g 5-Amino-4-hydroxy-2-isobutyrylamino-pyrimidin und Phosgen.

Ausbeute: 58%;
Rf: 0,77;
IR-Spektrum: 1765, 1660, 1600, 1525 $cm^{-1}$;
NMR-Signale ($D_2O$) bei ppm: 1,2 (m, 6H), 1,4 (3H), 1,5 (3H), 2,7 (m, 1H), 4,2 (s, 1H), 5,5 m (3H), 7,5 (5H), 8,2 (1H).

## Beispiel 113

D-α-[3-(2-Formylamino-4-hydroxy-5-pyrimidyl)-ureido]-p-hydroxy-benzylpenicillin-Natrium

Dieses Penicillin wurde analog Beispiel 109 hergestellt. Man geht aus von 2,35 g Amoxicillin-trihydrat (0,0056 Mol) sowie dem Umsetzungsprodukt von 860 mg (0,0056 Mol) 5-Amino-2-formyl-amino-4-hydroxy-pyrimidin mit 560 mg Phosgen und 0,77 ml Triäthylamin.

Ausbeute: 1,88 g Natriumsalz (33%);
IR-Spektrum: 1770, 1660, 1640, 1610, 1525 $cm^{-1}$;
NMR-Spektrum (DMSO + $CD_3OD$) Signale bei ppm: 1,55 (6H), 4,1 (1H), 5,35 (2H), 5,45 (1H), 6,85 (2H), 7,45 (2H), 8,20 (1H), 8,45 (1H).

## Beispiel 114

D-α-[3-(2-Formylamino-4-hydroxy-5-pyrimidyl)-ureido]benzylpenicillin-Natrium

Dieses Penicillin wurde analog Beispiel 113 hergestellt. Man geht aus von 1,0 g Ampicillin-Natrium (0,0027 Mol) sowie dem Umsetzungsprodukt von 415 mg des Amins des Beispiels 113 (0,0027 Mol) mit 270 mg Phosgen und 0,37 ml Triäthylamin.

Ausbeute: 355 mg Natriumsalz (26%);
IR-Spektrum: 1770, 1660, 1635, 1605, 1520 $cm^{-1}$;
NMR-Spektrum (DMSO + $CD_3OD$) Signale bei ppm: 1,55 (6H), 4,0 (1H), 5,35 (2H), 5,45 (1H), 7,45 (5H), 8,15 (1H), 8,45 (1H).

## III. Darstellung pharmazeutischer Zubereitungen

### Beispiel I

Tabletten enthaltend D-α-[3-(2-Cyclopropyl-4-hydroxy-5-pyrimid-yl)-ureido]-p-hydroxybenzylpenicillin-Natrium

Ein Gemisch bestehend aus 2 kg Wirksubstanz, 5 kg Lactose, 1,8 kg Kartoffelstärke, 0,1 kg Magnesiumstearat und 0,1 kg Talk wird in üblicher Weise zu Tabletten gepreßt, derart, daß jede Tablette 200 mg Wirkstoff enthält.

### Beispiel II

Dragees enthaltend D-α-[3-(2-Cyclopropyl-4-hydroxy-5-pyrimidyl)-ureido]-p-hydroxybenzylpenicillin-Natrium

Analog Beispiel I werden Tabletten gepreßt, die anschließend in üblicher Weise mit einem Überzug, bestehend aus Zucker, Kartoffelstärke, Talk und Tragant überzogen werden.

### Beispiel III

Kapseln enthaltend D-α-[3-(2-Cyclopropyl-4-hydroxy-5-pyrimidyl)-ureido]-p-hydroxybenzylpenicillin-Natrium

5 kg Wirksubstanz werden in üblicher Weise in Hartgelatinekapseln gefüllt, derart, daß jede Kapsel 500 mg des Wirkstoffs enthält.

### Beispiel IV

Trockenampullen enthaltend D-α-[2-Cyclopropyl-4-hydroxy-5-pyrimidyl)-ureido]-p-hydroxybenzylpenicillin-Natrium

In einem aseptischen Bereich wurde 251 g Wirkstoff in 2008 ml destilliertem Wasser zur Injektion aufgelöst. Die Lösung wurde durch ein Millipore-Filter (Porengröße 0,22 μm, Produkt der Millipore Corporation, Bedford, USA) filtriert. Die Lösung wurde jeweils in einer Menge von 2,0 ml in 1000 Gläschen (Kapazität 10 ml) eingegossen und es wurde lyophilisiert. Die Gläschen wurden sodann mit einem Kautschukstöpsel und einer Aluminiumkappe verschlossen. Somit wurden Gläschen (Nr. A) jeweils mit 250 mg Wirkstoff erhalten.

Eine physiologische Kochsalzlösung zur Injektion wurde in einer Menge von jeweils 2,0 ml in Ampullen abgefüllt und die Ampullen wurden verschlossen. Auf diese Weise wurden Ampullen (Nr. B) erhalten. Die physiologische Kochsalzlösung in den Ampullen (Nr. B) wurde in die Gläschen (Nr. A) gegossen, wodurch eine injizierbare Zubereitung für die intravenöse Verarbeitung erhalten wurde.

Destilliertes Wasser zur Injektion wurde in einer Menge von 20 ml in die Gläschen (Nr. A) gegossen und die Lösung wurde in einer 5%igen Lösung von Glucose für Injektionen (250 ml) aufgelöst. Auf diese Weise wurden Lösungen für die kontinuierliche Infusion hergestellt.

Analog sind Tabletten, Dragées, Kapseln und Ampullen erhältlich, die einen oder mehrere der übrigen Wirkstoffe der Formel I oder die physiologisch unbedenklichen Salze dieser Verbindungen enthalten.

**Patentansprüche**

1. Neue Penicilline der allgemeinen Formel I,

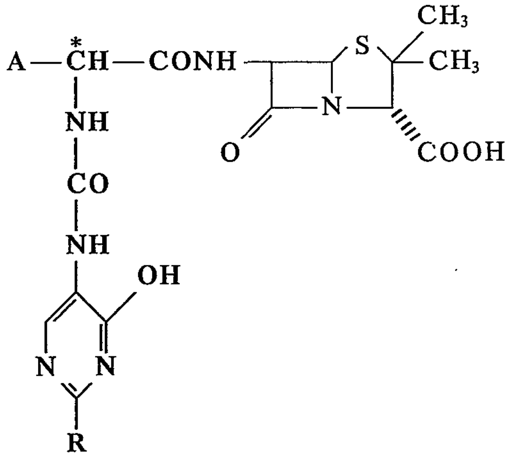

(I)

bzw. deren Tautomere der allgemeinen Formel I'

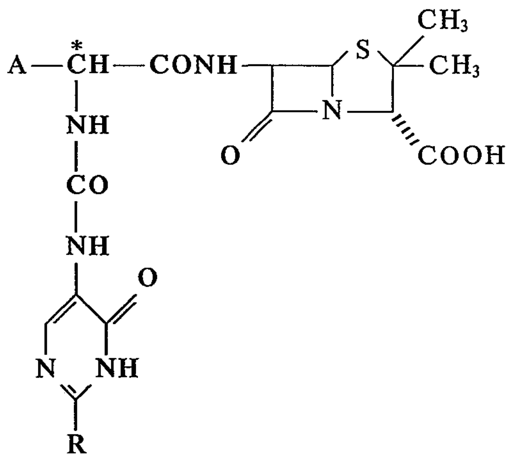

(I')

wobei
in der obigen allgemeinen Formel I:

A die Phenyl-, 4-Hydroxyphenyl-, 2-Thienyl-, Cyclohexyl-, Cyclohexen-1-yl- oder Cyclohexa-1,4-dien-1-ylgruppe, sowie einen in 3,4-Stellung disubstituierten Phenylrest, wobei die Substituenten gleich oder verschieden und Chloratome oder Hydroxygruppen sein können,
R ein Wasserstoffatom, eine Methylgruppe, den Cyclopropylrest, der gegebenenfalls mit einer Methylgruppe substituiert sein kann, den Cyclobutylrest bedeuten,
R bedeutet desweiteren,
die Hydroxygruppe, die Methoxy- oder Äthoxygruppe, die Amino- oder Dimethylaminogruppe,
eine Gruppe der allgemeinen Formel $NHR_1$, wobei $R_1$ einen verzweigten oder unverzweigten Alkylrest mit 1 bis 6 Kohlenstoffatomen, einen Cycloalkylrest mit 3 bis 7 Kohlenstoffatomen, einen Cycloalkylmethylrest mit 3 bis 6 Kohlenstoffatomen im Cycloalkylteil sowie einen Allyl-, Crotyl- oder Propargylrest darstellt,
R kann weiter einen Pyrrolidino-, Piperidino- oder Morpholinorest bedeuten, oder eine Gruppe der allgemeinen Formel

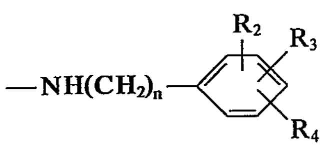

worin n die Zahlen 0 oder 1 und $R_2$, $R_3$ und $R_4$, die gleich oder voneinander verschieden sein können, Wasserstoff, Halogen, freie Aminogruppen, Alkylamino- oder Dialkylaminogruppen, wobei jeder Alkylteil 1 bis 3 Kohlenstoffatome enthält, Pyrrolidyl-, Piperidyl-, Hydroxy- oder Alkoxygruppen

mit 1 bis 3 Kohlenstoffatome, aliphatische Acylaminogruppen mit 1 bis 3 Kohlenstoffatomen, Aminocarbonylamino-, Alkylaminocarbonylamino-, Dialkylaminocarbonylaminogruppen mit 1 bis 3 Kohlenstoffatomen in jeder Alkylgruppe, Nitro-, Alkylsulfonylaminogruppen mit 1 bis 3 Kohlenstoffatomen, die Hydroxysulfonylaminogruppe, Formyl- oder Alkylcarbonylgruppen mit 1 bis 3 Kohlenstoffatomen im Alkylteil, Alkylcarbonyloxy-, Alkoxycarbonyl- und Alkoxycarbonyloxygruppen mit 1 bis 3 Kohlenstoffatomen im Alkylteil, Formyloxy-, Carboxyl-, Carbamoyl-, N-Alkyl- und N,N-Dialkylcarbamoylgruppen, mit jeweils 1 bis 4 Kohlenstoffatomen in jeder Alkylgruppe, Alkoxycarbonylaminogruppen mit 1 bis 4 Kohlenstoffatomen im Alkylteil, Cyan-, Mercapto-, Alkylmercapto-, Alkylsulfinyl- und Alkylsulfonylgruppen mit 1 bis 4 Kohlenstoffatomen, Sulfamoyl-, N-Alkyl- und N,N-Dialkylsulfamoylgruppen mit 1 bis 4 Kohlenstoffatomen in jeder Alkylgruppe, Sulfo-, Alkoxysulfonyl-, Sulfamoyloxy-, Alkyl- und Dialkylsulfamoyloxygruppen mit 1 bis 4 Kohlenstoffatomen in jeder Alkylgruppe, geradkettige oder verzweigte Alkylgruppen mit 1 bis 6, vorzugsweise 1 bis 3 Kohlenstoffatomen, die mit Halogenatomen substituiert sein können, bedeuten,
desweiteren kann R eine Gruppe der allgemeinen Formel

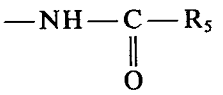

darstellen, in der $R_5$ ein Wasserstoffatom oder eine Alkylgruppe mit 1 bis 3 Kohlenstoffatomen darstellt, und deren physiologisch verträgliche Salze mit anorganischen oder organischen Basen.

2. Neue Penicilline der allgemeinen Formel I,

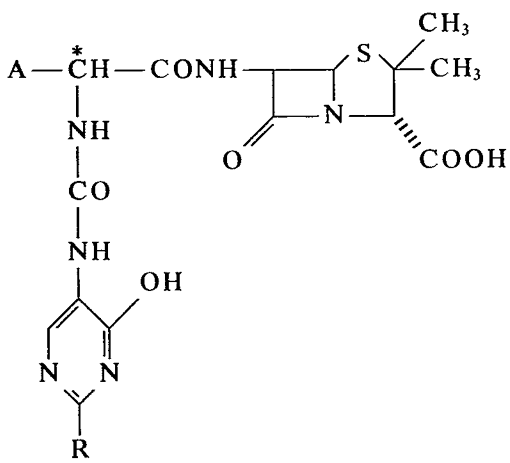

(I)

bzw. deren Tautomere der allgemeinen Formel I',

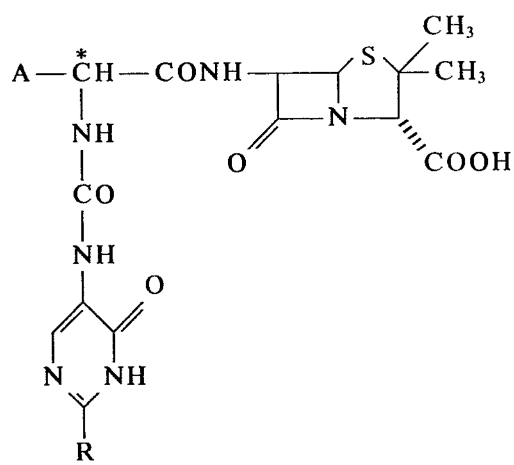

(I')

worin A die Phenyl- oder p-Hydroxyphenylgruppe bedeutet und R die folgenden Bedeutungen besitzt:
ein Wasserstoffatom, die Cyclopropylgruppe,
eine Gruppe der allgemeinen Formel

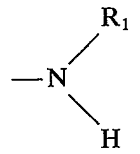

worin $R_1$ einen verzweigten oder unverzweigten Alkylrest mit 3 bis 5 Kohlenstoffatomen, einen Cycloalkylrest mit 3 bis 6 Kohlenstoffatomen oder die Allyl- oder Propargylgruppe bedeutet, des weiteren bedeutet R eine Gruppe der allgemeinen Formel

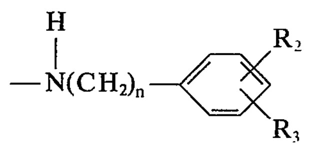

worin
n die Zahlen 0 oder 1 und einer oder beide Reste $R_2$ und $R_3$ Halogenatome, insbesondere Brom-, Chlor- oder Fluoratome, Methyl-, Äthyl-, Isopropyl-, Amino-, Methylamino-, Dimethylamino-, Hydroxy-, Methoxy-, Äthoxy-, Nitro-, Acetylamino-, Acetyl-, Methylcarbonyloxy-, Methoxycarbonyl-, Carboxyl-, Carbamoyl-, Methyl- und Dimethylcarbamoyl-, Cyan-, Methylmercapto-, Methylsulfinyl-, Äthylsulfinyl-, Propylsulfinyl- oder Isopropylsulfinyl- oder Methylsulfonyl-, Äthylsulfonyl-, Propylsulfonyl- oder Isopropylsulfonyl-, Carbamoylamino-, Sulfamoyl-, N-Methylsulfamoyl-, N-Äthylsulfamoyl-, N-Propylsulfamoyl-, N-Isopropylsulfamoyl- oder N,N-Dimethylsulfamoyl- oder Trifluormethylgruppen darstellen und der übrige Rest Wasserstoff bedeutet und deren physiologisch verträgliche Salze mit anorganischen oder organischen Basen.

3. D-α-[3-(2-p-Sulfamoylanilino-4-hydroxy-5-pyrimidinyl)-ureido]-p-hydroxy-benzylpenicillin-Natrium.

4. Neue Penicilline der allgemeinen Formel I bzw. der tautomeren Formel I' gemäß den Ansprüchen 1, 2 und 3, für welche die D = R-Konfiguration zutrifft, und deren physiologisch verträgliche Salze mit anorganischen oder organischen Basen.

5. Verfahren zur Herstellung neuer Penicilline der allgemeinen Formel I bzw. I' gemäß Anspruch 1 und von deren physiologisch verträglichen Salzen mit anorganischen oder organischen Basen, dadurch gekennzeichnet, daß

a) eine Verbindung der allgemeinen Formel III

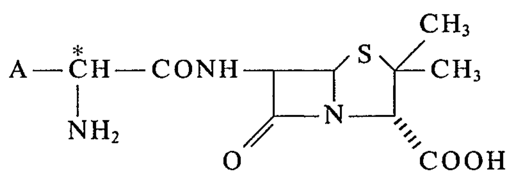

(III)

in der A wie oben definiert ist, mit einem Pyrimidinderivat der allgemeinen Formel IV

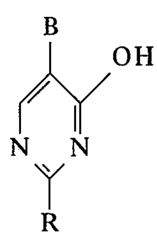

(IV)

in der R wie oben definiert ist und B die Gruppe −NCO oder ein reaktives Derivat der Gruppe −NHCOOH bedeutet, in einem Lösungsmittel bei Temperaturen zwischen −20 und +50°C umgesetzt wird oder

b) eine Ureidocarbonsäure der allgemeinen Formel V

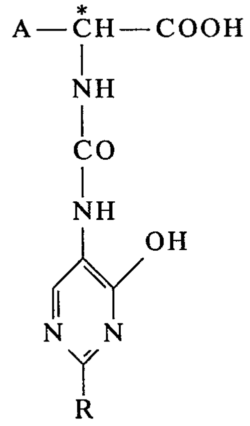

(V)

in der
A und R die oben angegebenen Bedeutungen besitzen, bzw. ihre Salze oder reaktiven Derivate mit der 6-Aminopenicillansäure der Formel VI

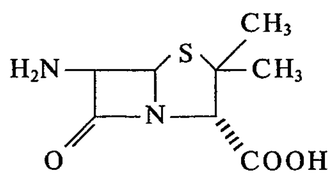

(VI)

oder ihren anorganischen oder organischen Salzen oder Derivaten, die in die 6-Aminopenicillansäure leicht überführbar sind, zur Reaktion gebracht werden und, gegebenenfalls anschließend, das so erhaltene Produkt zu einem Penicillin der allgemeinen Formel I hydrolysiert oder katalytisch hydrogenolysiert wird und/oder gewünschtenfalls anschließend das erhaltene Penicillin der allgemeinen Formel I mittels anorganischer oder organischer Basen in sein Salz übergeführt wird.

6. Verfahren gemäß Anspruch 5a, dadurch gekennzeichnet, daß ein Pyrimidinderivat der allgemeinen Formel IV gemäß Anspruch 5a, in der B die Gruppe $-NCO$ oder in der B ein reaktives Derivat der Gruppe $-NHCOOH$ bedeutet, oder, im letzteren Fall, ein Gemisch eines solchen Pyrimidinderivates mit einem anderen Pyrimidinderivat der allgemeinen Formel IV, in der B die Gruppe $-NCO$ darstellt, verwendet wird und die Umsetzung entweder, bei Vorliegen eines anorganischen oder organischen Salzes einer Verbindung der allgemeinen Formel III, in Mischungen vom Wasser mit in Wasser mischbaren organischen Lösungsmitteln bzw. in organischen Lösungsmitteln bzw. in Gemengen aus Wasser und mit Wasser nichtmischbaren organischen Lösungsmitteln in einem pH-Bereich zwischen 2,0 und 9,0, bzw. in Wasser als Lösungsmittel in Gegenwart einer Base bzw. einer Pufferlösung oder, bei Vorliegen eines Silylderivates oder Carboxylderivates, einer Verbindung der allgemeinen Formel III, in wasser- und hydroxylgruppenfreien Lösungsmitteln, gegebenenfalls unter Zusatz von Basen, durchgeführt wird.

7. Verfahren gemäß Anspruch 5b, dadurch gekennzeichnet, daß als reaktive Derivate der Ureidocarbonsäuren der allgemeinen Formel V deren Säureanhydride, deren reaktive Ester oder Amide, deren Säurehalogenide oder Säureazide verwendet werden und die 6-Aminopenicillansäure der Formel VI in Form ihrer Silylester, Tritylester, p-Nitrobenzylester, Phenacylester und O,N-Bis-trimethylsilylderivate in einem aprotischen Lösungsmittel, in Form ihrer Salze jedoch in einem protischen Lösungsmittel, in Methylenchlorid oder in einem wäßrigen Medium oder in einem wässerigorganischen Lösungsmittel bei Temperaturen zwischen $-40$ und $+40°C$, gegebenenfalls in Gegenwart einer Base und/oder eines Kondensationsmittels, zur Reaktion gebracht wird und, falls das Endprodukt noch als Ester vorliegt, die Estergruppe anschließend hydrolysiert oder hydrogenolysiert wird.

8. Arzneimittel enthaltend eine oder mehrere Verbindungen gemäß den Ansprüchen 1 bis 4 neben den üblichen Träger- und/oder Hilfsstoffen.

9. Arzneimittel enthaltend zumindest eine Verbindung gemäß den Ansprüchen 1–4 neben einem Aminoglykosidantibiotikum und/oder einer Clavulansäure oder deren pharmakologisch verträglichen Salzen und einem üblichen Träger- und/oder Hilfsstoff.

**Claims**

1. New penicillins of general formula I,

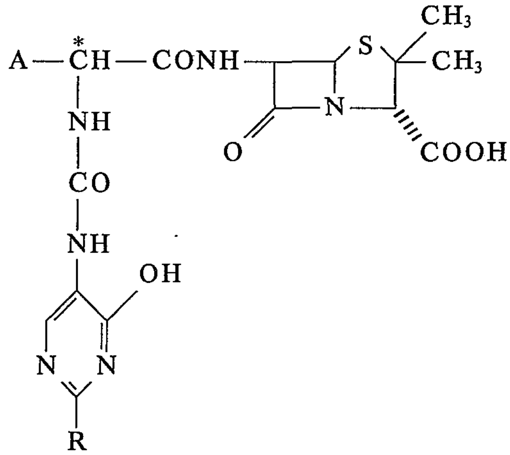

(I)

and the tautomers thereof of general formula I'

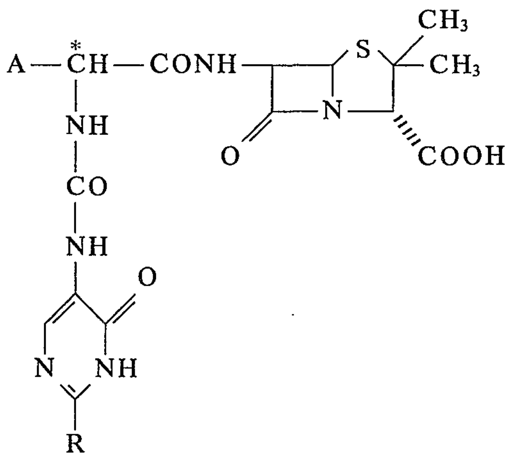

(I')

wherein,
in general formula I above,
A represents the phenyl, 4-hydroxyphenyl, 2-thienyl, cyclohexyl, cyclohexen-1-yl or cyclohexa-1,4-dien-1-yl group, and a phenyl radical disubstituted in the 3,4 position, whilst the substituents may be identical or different and may be chlorine atoms or hydroxy groups,
R represents a hydrogen atom, a methyl group, the cyclopropyl radical, which may optionally be substituted by a methyl group, and the cyclobutyl radical,
R further represents the hydroxy group, the methoxy or ethoxy group, the amino or dimethylamino group, a group of formula $NHR_1$, wherein $R_1$ represents a branched or nonbranched alkyl group with 1 to 6 carbon atoms, a cycloalkyl group with 3 to 7 carbon atoms, a cycloalkylmethyl group with 3 to 6 carbon atoms in the cycloalkyl part, and an allyl, crotyl or propargyl group,
R may also represent a pyrrolidino, piperidino or morpholino group or a group of general formula

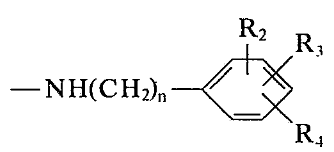

wherein n represents the numbers 0 or 1 and $R_2$, $R_3$ and $R_4$, which may be identical or different, represent hydrogen, halogen, free amino groups, alkylamino or dialkylamino groups in which each alkyl part contains 1 to 3 carbon atoms, pyrrolidyl, piperidyl, hydroxy or alkoxy groups with 1 to 3 carbon atoms, aliphatic acylamino groups with 1 to 3 carbon atoms, aminocarbonylamino, alkylaminocarbonylamino, dialkylaminocarbonylamino groups with 1 to 3 carbon atoms in each alkyl group, nitro, alkylsulphonylamino groups with 1 to 3 carbon atoms, the hydroxysulphonylamino

group, formyl or alkylcarbonyl groups with 1 to 3 carbon atoms in the alkyl part, alkylcarbonyloxy, alkoxycarbonyl and alkoxycarbonyloxy groups with 1 to 3 carbon atoms in the alkyl part, formyloxy, carboxyl, carbamoyl, N-alkyl- and N,N-dialkylcarbamoyl groups with 1 to 4 carbon atoms in each alkyl group, alkoxycarbonylamino groups with 1 to 4 carbon atoms in the alkyl part, cyano, mercapto, alkylmercapto, alkylsulphinyl and alkylsulphonyl groups with 1 to 4 carbon atoms, sulphamoyl, N-alkyl- and N,N-dialkylsulphamoyl groups with 1 to 4 carbon atoms in each alkyl group, sulpho, alkoxysulphonyl, sulphamoyloxy, alkyl- and dialkylsulfamoyloxy groups with 1 to 4 carbon atoms in each alkyl group, straight-chained or branched alkyl groups with 1 to 6, preferably 1 to 3 carbon atoms, which may be substituted by halogen atoms, and

furthermore R may represent a group of general formula

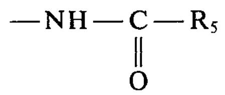

wherein $R_5$ represents a hydrogen atom or an alkyl group with 1 to 3 carbon atoms, and the physiologically acceptable salts thereof with inorganic or organic bases.

2. New penicillins of general formula I

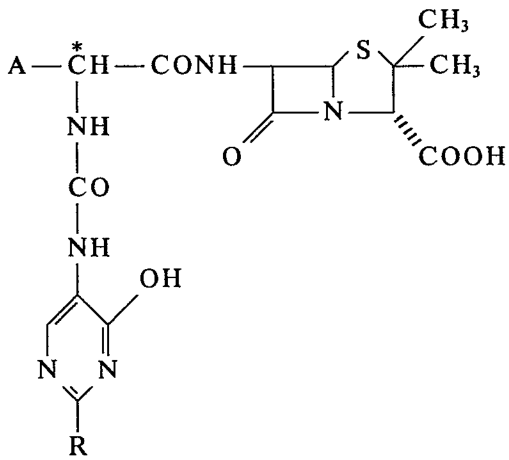

(I)

or the tautomers thereof of general formula I'

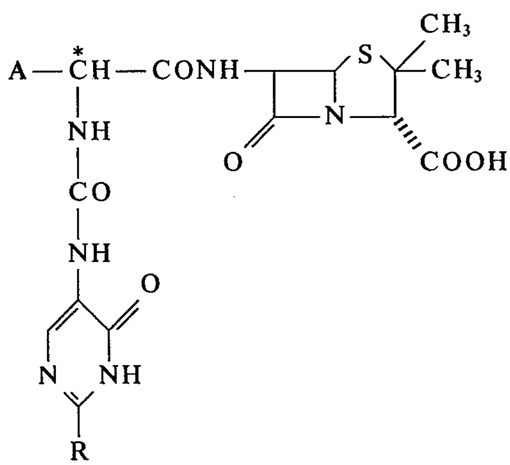

(I')

wherein A represents the phenyl or p-hydroxyphenyl group and R has the following meanings: a hydrogen atom, the cyclopropyl group, a group of general formula

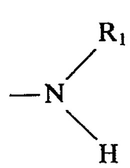

wherein $R_1$ represents a branched or non-branched alkyl group with 3 to 5 carbon atoms, a cycloalkyl

group with 3 to 6 carbon atoms or the allyl or propargyl group, and furthermore R represents a group of general formula

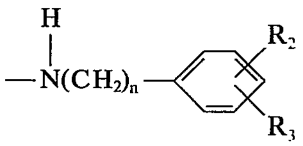

wherein
n represents the numbers 0 or 1 and one or both of the groups $R_2$ and $R_3$ represent halogen atoms, especially bromine, chlorine or fluorine atoms, mehtyl, ethyl, isopropyl, amino, methylamino, dimethylamino, hydroxy, methoxy, ethoxy, nitro, acetylamino, acetyl, methylcarbonyloxy, methoxycarbonyl, carboxyl, carbamoyl, methyl- and dimethyl- carbamoyl, cyano, methylmercapto, methylsulphinyl, ethylsulphinyl, propylsulphinyl or isopropylsulphinyl or methylsulphonyl, ethylsulphonyl, propylsulphonyl or isopropylsulphonyl, carbamoylamino, sulphamoyl, N-methylsulphamoyl, N-ethylsulphamoyl, N-propylsulphamoyl, N-isopropylsulphamoyl or N,N-dimethylsulphamoyl or trifluoromethyl groups, and the other group represents hydrogen, and the physiologically acceptable salts thereof with inorganic or organic bases.

3. D-α-[3-(2-p-Sulfamoylanilino-4-hydroxy-5-pyrimidinyl)-ureido]-p-hydroxy-benzylpenicillin sodium.

4. New penicillins of general formula I or of the tautomeric formula I' as claimed in claims 1, 2 and 3 having the D=R configuration, and the physiologically acceptable salts thereof with inorganic or organic bases.

5. Process for preparing new penicillins of general formula I or I' as claimed in claim 1 and the physiologically acceptable salts thereof with inorganic or organic bases, characterised in that

a) a compound of general formula III

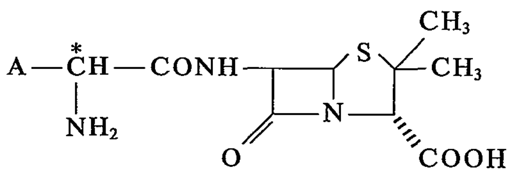

(III)

wherein A is as hereinbefore defined, is reacted with a pyrimidine derivative of general formula IV

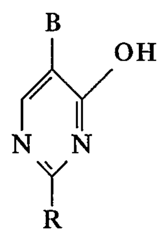

(IV)

wherein R is as hereinbefore defined and B represents the group −NCO or a reactive derivative of the group −NHCOOH, in a solvent at temperatures of between −20 and +50°C, or

b) a ureidocarboxylic acid of general formula V

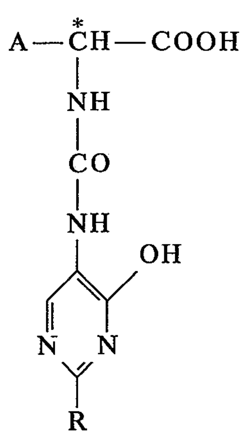

(V)

wherein
A and R are as hereinbefore defined, or the salts or reactive derivatives thereof, is reacted with 6-aminopenicillanic acid of formula VI

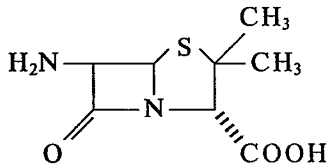

(VI)

or the inorganic or organic salts or derivatives thereof which can readily be converted into 6-aminopenicillanic acid, and subsequently, if desired, the product thus obtained is hydrolysed or catalytically hydrogenolysed to form a penicillin of general formula I, and/or subsequently, if desired, the penicillin of general formula I obtained is converted into a salt thereof with inorganic or organic bases.

6. Process according to claim 5a, characterised in that a pyrimidine derivative of general formula IV as defined in claim 5a, wherein B represents the group $-NCO$ or wherein B represents a reactive derivative of the group $-NHCOOH$ or, in the latter case, a mixture of a pyrimidine derivative of this kind and another pyrimidine derivative of general formula IV, wherein B represents the group $-NCO$, is used and the reaction is carried out either — when an inorganic or organic salt of a compound of general formula III is present — in mixtures of water with water-miscible organic solvents or in organic solvents or in mixtures of water and water-immiscible organic solvents in a pH range of between 2.0 and 9.0, or in water as the solvent in the presence of a base or a buffer solution, or — when a silyl or carboxyl derivative of a compound of general formula III is present — in anhydrous solvents free from hydroxyl groups, possibly with the addition of bases.

7. Process according to claim 5b, characterised in that the compounds used as the reactive derivatives of the ureidocarboxylic acids of general formula V are the acid anhydrides, reactive esters or amides, acid halides or acid azides thereof, and the 6-amino-penicillanic acid of formula VI, when used in the form of its silyl ester, trityl ester, p-nitrobenzyl ester, phenacyl ester and O,N-bis-trimethylsilyl derivatives, is reacted in an aprotic solvent, but, when used in the form of its salts, is reacted in a protic solvent, in methylene chlorine or in an aqueous medium or in an aqueous-organic solvent at temperatures between $-40$ and $+40°C$, optionally in the presence of a base and/or a condensing agent, and if the end product is obtained in ester form, the ester group is subsequently hydrolysed or hydrogenolysed.

8. Pharmaceutical compositions containing one or more compounds as claimed in claims 1 to 4, together with the conventional carriers and/or excipients.

9. Pharmaceutical compositions containing at least one compound as claimed in claims 1 to 4, together with an aminoglycoside antibiotic and/or a clavulanic acid or the pharmacologically acceptable salts thereof and a conventional carrier and/or excipient.

## Revendications

1. Nouvelles pénicillines de formule gpenérale I,

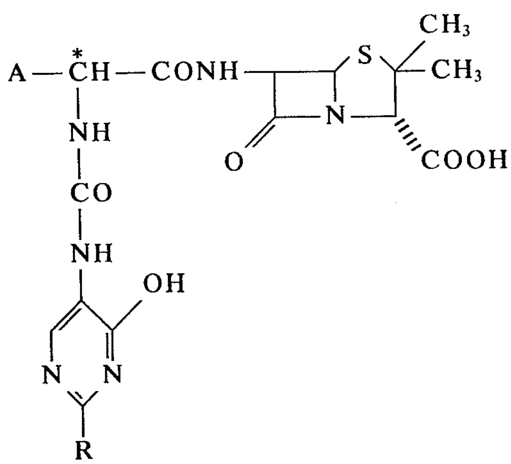

(I)

ou respectivement leurs tautomères de formule générale I'

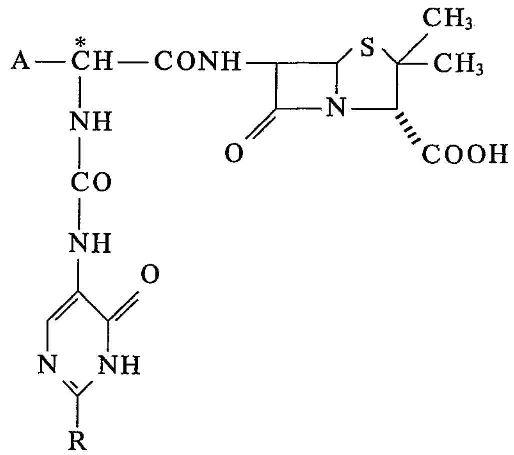

(I')

où
dans la formule générale I ci-dessus:
A représente le groupe phényle, 4-hydroxyphényle, 2-thiényle, cyclohexyle, cyclohexène-1-yle ou cyclohexa-1,4-diène-1-yle, ainsi qu'un radical phényle disubstitué en position 3, 4, les substituants pouvant être identiques ou différents et des atomes de chlore ou des groupes hydroxy,
R représente un atome d'hydrogène, un groupe méthyle, le radical cyclopropyle qui peut éventuellement être substitué par un groupe méthyle, le radical cyclobutyle,
R représente en outre, le groupe hydroxy, le groupe méthoxy ou éthoxy, le groupe amino ou diméthylamino, un groupe de formule générale $NHR_1$, où $R_1$ représente un radical alcoyle ramifié ou non ramifié avec 1 à 6 atomes de carbone, un radical cycloalcoyle avec 3 à 7 atomes de carbone, un radical cycloalcoylméthyle avec 3 à 6 atomes de carbone dans la partie cycloalcoyle ainsi qu'un radical allyle, crotyle ou propargyle,
R peut en outre représenter un radical pyrrolidino, pipéridino ou morpholino, ou un groupe de formule générale

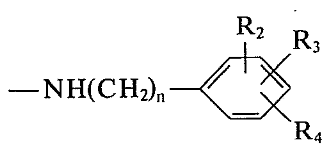

dans laquelle n représente les nombres 0 ou 1, et $R_2$, $R_3$ et $R_4$ qui peuvent être identiques ou différents les uns des autres représentent l'hydrogène, un halogène, des groupes amino libres, des groupes alcoylamino ou dialcoylamino dans lesquels chaque partie alcoyle contient 1 à 3 atomes de carbone, des groupes pyrrolidyle, pipéridyle, hydroxy ou alcoxy avec 1 à 3 atomes de carbone, des groupes acylamino aliphatiques avec 1 à 3 atomes de carbone, des groupes aminocarbonylamino, alcoylaminocarbonylamino, dialcoylaminocarbonylamino avec 1 à 3 atomes de carbone dans chaque groupe alcoyle, des groupes nitro, alcoylsulfonylamino avec 1 à 3 atomes de carbone, le groupe hydroxysulfonylamino, des groupes formyle ou alcoylcarbonyle avec 1 à 3 atomes de carbone dans la partie alcoyle, des groupes alcoylcarbonyloxy, alcoxycarbonyle et alcoxycarbonyloxy avec 1 à 3 atomes de carbone dans la partie alcoyle, des groupes formyloxy, carboxyle, carbamoyle, N-alcoyl- et N,N-dialcoylcarbamoyle avec chaque fois 1 à 4 atomes de carbone dans chaque groupe alcoyle, des groupes alcoxycarbonylamino avec 1 à 4 atomes de carbone dans la partie alcoyle, des groupes cyano, mercapto, alcoylmercapto, alcoylsulfinyle et alcoylsulfonyle avec 1 à 4 atomes de carbone, des groupes sulfamoyle, N-alcoyl- et N,N-dialcoylsulfamoyle avec 1 à 4 atomes de carbone dans chaque groupe alcoyle, des groupes sulfo, alcoxysulfonyle, sulfamoyloxy, alcoyl- et dialcoylsulfamoyloxy avec 1 à 4 atomes de carbone dans chaque groupe alcoyle, des groupes alcoyle rectilignes ou ramifiés avec 1 à 6, de préférence 1 à 3 atomes de carbone, qui peuvent être substitués par des atomes d'halogène, en outre R peut représenter un groupe de formule générale

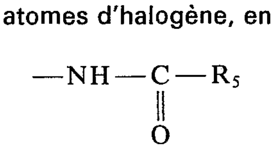

dans laquelle $R_5$ représente un atome d'hydrogène ou un groupe alcoyle avec 1 à 3 atomes de carbone, et leurs sels physiologiquement supportables avec des bases minérales ou organiques.

2. Nouvelles pénicillines de formule générale I,

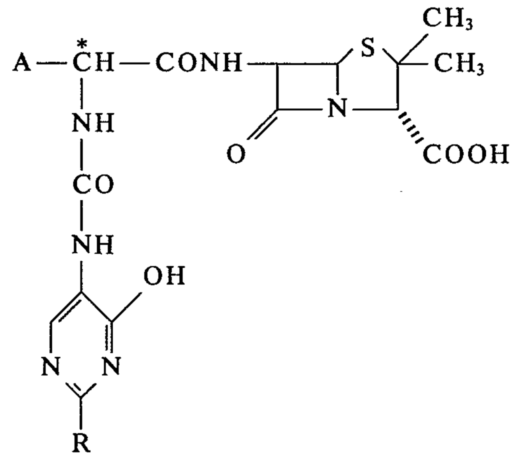

(I)

ou respectivement leurs tautomères de formule générale I'

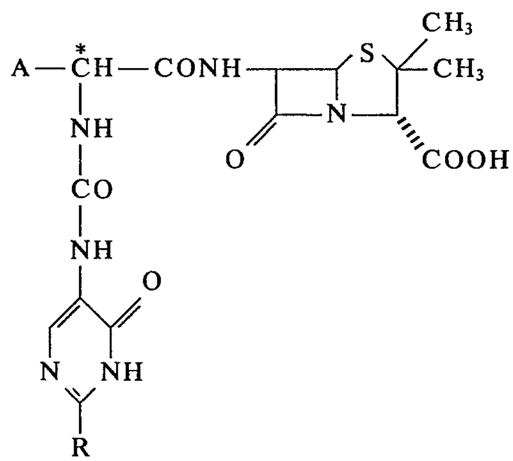

(I')

dans laquelle A représente le groupe phényle ou p-hydroxyphényle et R possède les significations suivantes:
un atome d'hydrogène, le groupe cyclopropyle, un groupe de formule générale,

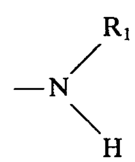

dans laquelle $R_1$ représente un radical alcoyle ramifié ou non ramifié avec 3 à 5 atomes de carbone, un radical cycloalcoyle avec 3 à 6 atomes de carbone ou le groupe allyle ou propargyle,
R représente en outre un groupe de formule générale

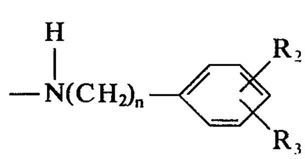

dans laquelle:
n représente les nombres 0 ou 1 et l'un ou les deux radicaux $R_2$ et $R_3$ représentent des atomes d'halogène, en particulier des atomes de brome, de chlore ou de fluor, des groupes méthyle, éthyle, isopropyle, amino, méthylamino, diméthylamino, hydroxy, méthoxy, éthoxy, nitro, acétylamino, acétyle, méthylcarbonyloxy, méthoxycarbonyle, carboxyle, carbamoyle, méthyl- et diméthylcarbamoyle, cyano, méthylmercapto, méthylsulfinyle, éthylsulfinyle, propylsulfinyle ou isopropylsulfinyle ou méthylsulfonyle, éthylsulfonyle, propylsulfonyle ou isopropylsulfonyle, carbamoylamino,

sulfamoyle, N-méthylsulfamoyle, N-éthylsulfamoyle, N-propylsulfamoyle, N-isopropylsulfamoyle ou N,N-diméthylsulfamoyle ou trifluorométhyle et l'autre radical représente l'hydrogène et leurs sels physiologiquement supportables avec des bases minérales ou organiques.

3. D-α-[3-(2-p-Sulfamoylanilino-4-hydroxy-5-pyrimidinyl)-ureido]-p-hydroxy-benzylpenicilline sodium.

4. Nouvelles pénicillines de formule générale I ou respectivement de formule tantomère I' selin les revendications 1, 2 et 3 pour lesquelles la confifuration D=R s'applique, et leurs sels physiologiquement supportables avec des bases minérales ou organiques.

5. Procédé de préparation des nouvelles pénicillines de formule générale I ou respectivement I' selon la revendication 1 et leurs sels physiologiquement supportables avec des bases minérales ou organiques, caractérisé en ce que

a) on fait réagir un composé de formule générale III

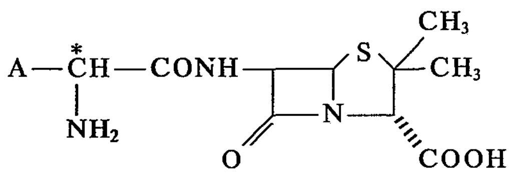

(III)

dans laquelle A est défini comme plus haut, avec un dérivé de pyrimidine de formule générale IV

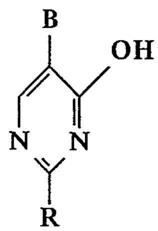

(IV)

dans laquelle R est defini comme plus haut et B représente le groupe —NCO ou un dérivé réactiv du groupe —NHCOOH, dans un solvant à des températures comprises entre —20 et + 50°C ou

b) on amène à réagir un acide uréidocarboxylique de formule générale V

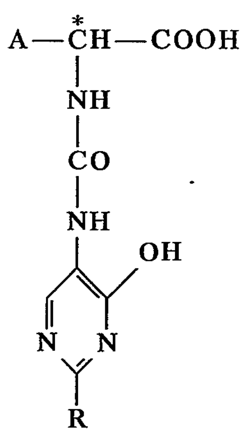

(V)

dans laquelle
A et R possèdent les significations données plus haut, ou respectivement leurs sels ou dérivés réactifs avec l'acide 6-aminopénicillanique de formule VI

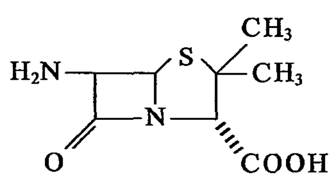

(VI)

ou ses sels minéraux ou organiques ou dérivés qui sont facilement transformables en l'acide

6-aminopénicillanique et, éventuellement ensuite, le produit ainsi obtenu est hydrolysé ou hydrogénolysé catalytiquement en une pénicilline de formule générale I et/ou éventuellement ensuite la pénicilline obtenue de formule générale I est transformée en son sel à l'aide de bases minérales ou organiques.

6. Procédé selon la revendication 5a, caractérisé en ce qu'on utilise un dérivé de pyrimidine de formule générale IV selon la revendication 5a, dans laquelle B représente le groupe —NCO ou dans laquelle B représente un dérivé réactif du groupe —NHCOOH, ou, dans le dernier cas, un mélange d'un tel dérivé de pyrimidine avec un autre dérivé de pyrimidine de formule générale IV dans laquelle B représente le groupe —NCO et en ce qu'on effectue la réaction soit, en présence d'un sel minéral ou organique d'un composé de formule générale III, dans des mélanges d'eau avec des solvants organiques miscibles à l'eau ou dans des solvants organiques ou dans des mélanges d'eau et de solvants organiques non miscibles à l'eau, dans un domaine de pH compris entre 2,0 et 9,0 ou dans l'eau en tant que solvant en présence d'une base ou d'une solution tampon ou, en présence d'un dérivé silylé ou carboxylé, d'un composé de formule générale III, dans des solvants anhydres ou ne contenant pas de groupes hydroxy, éventuellement avec addition de bases.

7. Procédé selon la revendication 5b, caractérisé en ce qu'on utilise, en tant que dérivés réactifs des acides uréidocarboxyliques de formule générale V, leurs anhydrides d'acides, leurs esters ou amides réactifs, leurs halogénures d'acides ou azides d'acides et on amène à réagir l'acide 6-aminopénicillanique de formule VI sous forme de ses ester silylé, ester tritylé, ester p-nitrobenzylé, ester phénacylé et dérivés O,N-bis-triméthylsilylés dans un solvant aproptique, mais sous forme de leurs sels dans un solvant protique, dans le chlorure de méthylène ou dans un milieu aqueux — solvant organique à des températures comprises entre −40 et +40°C, éventuellement en présence d'une base et/ou d'un agent de condensation, et, dans le cas où le produit final se présente encore sous la forme d'un ester, on hydrolyse ou hydrogénolyse ensuite le groupe ester.

8. Médicament contenant un ou plusieurs composés selon les revendication 1 à 4 conjointement aux excipients et/ou adjuvants habituels.

9. Médicament contenant au moins un composé selon les revendications 1—4 conjointement à un antibiotique aminoglycosidique et/ou un acide clavulanique ou leurs sels pharmacologiquement supportables et un excipient et/ou adjuvant habituel.